# EUROPEAN PATENT SPECIFICATION

(11) **EP 0 545 195 B1**
(45) Date of publication and mention of the grant of the patent: **22.11.1995**
(21) Application number: 92119904.8
(22) Date of filing: 23.11.1992
(51) Int. Cl.: C07H 19/044, A61K 31/70

(54) **Indolopyrrolocarbazole derivatives**
Indolopyrrolocarbazolederivate
Dérivés d'indolopyrrolocarbazole

(30) Priority: 29.11.1991 JP 341916/91; 18.02.1992 JP 69269/92; 01.09.1992 JP 257306/92
(43) Date of publication of application: 09.06.1993
(73) Proprietor: BANYU PHARMACEUTICAL CO., LTD., Chuo-ku, Tokyo (JP)
(72) Inventor: Katsuhisa, Kojiri, c/o Banyu Pharmaceutical, Tsukuba-shi, Ibaraki-ken (JP); Hisao, Kondo, c/o Banyu Pharmaceutical, Tsukuba-shi, Ibaraki-ken (JP); Hiroharu, Arakawa, c/o Banyu Pharmaceutical, Tsukuba-shi, Ibaraki-ken (JP); Ohkubo, Mitsuru, c/o Banyu Pharmaceutical, Tsukuba-shi, Ibaraki-ken (JP); Hiroyuki, Suda, c/o Banyu Pharmaceutical, Tsukuba-shi, Ibaraki-ken (JP)
(74) Representative: Kraus, Walter, Dr.

(56) References cited:
- EP-A- 0 269 025
- EP-A- 0 445 736
- EP-A- 0 450 327

## Description

This invention is useful in the field of medicine, and relates to novel indolopyrrolocarbazole derivatives inhibiting proliferation of antitumor cells and exhibiting an antitumor effect, a process for preparation thereof and a use thereof.

In the field of cancer chemotherapy, many compounds are already put to practical use as antitumor agents. However, their effects on various kinds of tumors is not always adequate, and the problem of resistance of tumor cells against these drugs makes clinical use of these antitumor agents complicated [refer to The 47th Japan Society of Cancer General Meeting Article, pages 12 to 15 (1988)].

In such state of things, development of novel carcinostatic substances is always made in the field of cancer therapy. Particularly, substances are necessitated which overcome resistance against existing carcinostatic substances and exhibit effectiveness against such kinds of cancers on which existing carcinostatic substances cannot exhibit sufficient effects.
EP-A-0 269 025 relates to a compound having the formula 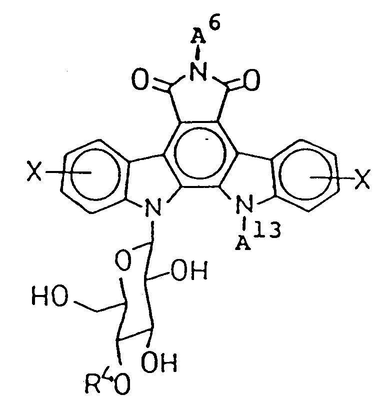
wherein:
n is an integer from 1 to 6; A⁶ and A¹³ are selected from H and -(CH₂)ₙ-NR¹R² and at least one of A⁶ and A¹³ is -(CH₂)ₙ-NR¹R²;
R¹ and R², independently, are selected from hydrogen, unsubstituted and substituted C₁-C₆ alkyl, aralkyl having 1 to 3 carbons in the alkyl moiety and unsubstituted phenyl or phenyl substituted with 1 to 3 alkyl, alkoxy, hydroxy, halo, carboxyl, alkoxycarbonyl and amino and mono- and di-lower-alkylamino groups in the aryl moiety, and aryl selected from unsubstituted phenyl and phenyl substituted with 1 to 3 alkyl, alkoxy, hydroxy, halo, amino, mono- and dialkylamino, nitro, carboxyl, alkoxycarbonyl and cyano groups provided that both R¹ and R² are not each aryl and, when taken together, R¹ and R² may be selected from -(CH₂)₄- and (CH₂)₂-R³-(CH₂)₂- to form a 5- or 6-membered ring together with the N-atom wherein R³ is selected from CH₂, NH, O and S;
X is selected from H, F, Cl, Br, C₁-C₃ alkyl, OH, carboxyl, alkoxycarbonyl and alkoxy wherein the alkyl moiety is C₁-C₃ alkyl, benzyloxy, amino and mono- and dialkylamino; and
R⁴ is selected from H and CH₃; and pharmaceutically acceptable acid addition and base salts thereof.
EP-A-0 445 736 describes a rebeccamycin analog having the formula 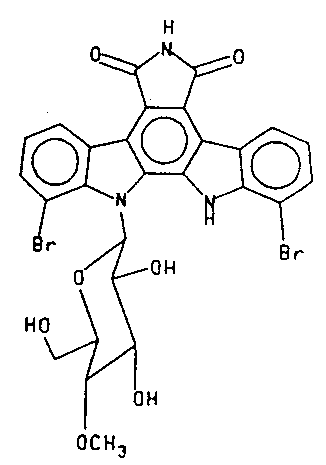
or a pharmaceutically acceptable acid addition salt thereof.
EP-A-0 450 327 describes a rebeccamycin analog having the formula 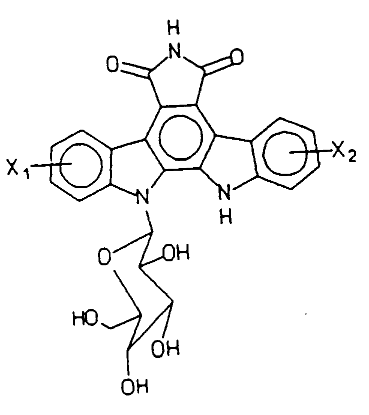
wherein X₁ and X₂ are independently fluorine or hydrogen, provided that X₁ and X₂ are not simultaneously hydrogen, as well as pharmaceutically acceptable acid addition salts thereof.

In the light of such present state of things, the present inventors widely screened microbial metabolic products, as a result, found a novel antitumor activity-possessing compound BE-13793C (12,13-dihydro-1,11-dihydroxy-5H-indolo[2,3-a]pyrrolo[3,4-c]carbazoles-5,7(6H)-dione), and disclosed it [refer to Japanese Laid-Open Patent Publication No. 20277/1991 and J. Antibiotics, 44, 723-728 (1991)].

Thereafter, the present inventors created indolopyrrolocarbazole compounds having an excellent antitumor activity by chemically modifying BE-13793C, and disclosed them (refer to PCT/WO91/18003).

For the purpose of creating compounds having a further excellent antitumor activity by chemically modifying previously disclosed indolopyrrolocarbazole antitumor compounds, the present inventors synthesized many indolopyrrolocarbazole derivatives, investigated their antitumor activity, and as a result, now, found that a series of compounds represented by the following general formula are novel compounds having an extremely excellent antitumor activity.

Thus, this invention provides indolopyrrolocarbazole derivatives represented by the following general formula and pharmaceutically acceptable salts thereof. 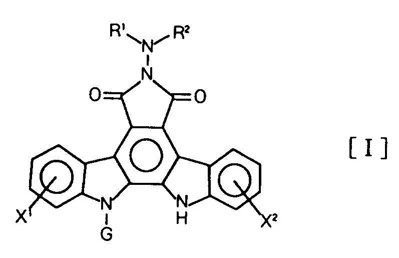
wherein
R¹ and R² each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and heterocyclic group may each have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and therein Y represents a carbonyl group, thiocarbonyl group or sulfonyl group and R³ represents a hydrogen atom, C₁-C₆ alkyl group, cycloalkyl group, cycloalkylalkyl group, aromatic hydrocarbon group having 6 to 12 carbon atoms, arakyl group, C₁-C₆ alkoxy group, hydrazino group, amino group, arylamino group, carbamoyl group or 5-or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by substituent(s) selected from the group consisting of halogen atoms, hydroxyl groups, amino groups, carboxyl groups, carbamoyl groups and C₁-C₆ alkoxycarbonyl groups); or
R¹ and R² combine to represent a C₁-C₆ alkylidene group (the C₁-C₆ alkylidene group may have 1 to 4 substituents selected from the group consisting of amino groups, mono-C₁-C₆ alkylamino groups, di-C₁-C₆ alkylamino groups, hydroxyl groups, carboxyl groups and sulfonyl groups); or
R¹ and R² combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by group(s) selected from the group consisting of amino groups, hydroxyl groups, carboxyl groups and sulfo groups),
G represents a pentose group or hexose group, and
X¹ and X² each independently represent a hydrogen atom, halogen atom, amino group, mono-C₁-C₆ alkylamino group, di-C₁-C₆ alkylamino group, hydroxyl group, C₁-C₆ alkoxy group, aralkoxy group, carboxyl group, C₁-C₆ alkoxycarbonyl group or C₁-C₆ alkyl group.

The "C₁-C₆ alkyl group" is a straight-chain or branched chain alkyl group having 1 to 6 carbon atoms, and examples thereof are a methyl group, an ethyl group, a propyl group, an isopropyl group, a butyl group, an isobutyl group, a sec-butyl group, a tert-butyl group, a pentyl group, an isopentyl group, a neopentyl group, a hexyl group, etc.

The "C₁-C₆ alkenyl group" includes a straight-chain or branched chain alkenyl group having 3 to 6 carbon atoms, and examples thereof are a propenyl group, a 2-butenyl group, a 3-butenyl group, a 3-pentenyl group, a 4-hexenyl group, etc.

The "C₁-C₆ alkynyl group" can be a straight-chain or branched chain alkynyl group having 3 to 6 carbon atoms, and examples thereof are a propynyl group, a 2-butynyl group, a 3-butynyl group, a 3-pentynyl group, a 4-hexynyl group, etc.

The "cycloalkyl group" includes a 3- to 6-membered cycloalkyl group, and examples thereof are a cyclopropyl group, a cyclobutyl group, a cyclopentyl group, a cyclohexyl group, etc.

The "cycloalkyl C₁-C₆ alkyl group" means an alkyl group substituted by a cycloalkyl group wherein the cycloalkyl and C₁-C₆ alkyl parts have the above meanings, respectively, and examples thereof are a cyclopropylmethyl group, a cyclobutylmethyl group, a cyclopentylmethyl group, a cyclohexylmethyl group, a 1-cyclopropylethyl group, a 2-cyclopropylethyl group, a 1-cyclobutylethyl group, a 2-cyclobutylethyl group, a 1-cyclopentylethyl group, a 2-cyclopentylethyl group, a 1-cyclohexylethyl group, a 3-cyclohexylpropyl group, a 3-cyclopentylpropyl group, a 4-cyclohexylbutyl group, a 4-cyclopentylbutyl group, etc., and preferably, the cycloalkylalkyl group has 4 to 10 carbon atoms in total.

The aromatic hydrocarbon group having 6 to 12 carbon atoms can be a phenyl group, a naphthyl group and a tetrahydronaphthyl group.

The "aralkyl" group means a C₁-C₆ alkyl group substituted by an aryl group wherein the aromatic hydrocarbon and C₁-C₆ alkyl parts have the above meanings, respectively, and aralkyl groups having 7 to 15 carbon atoms can be mentioned such as, for example, a benzyl group, a phenethyl group, a phenylpropyl group, a phenylbutyl group, a phenylpentyl group, a naphthylmethyl group and a naphthylethyl group.

As examples for the 5- or 6-membered heterocyclic group containing 1 to 4 hetero atoms selected from the group consisting of nitrogen atoms, oxygen atoms and sulfur atoms, there can be mentioned aromatic heterocyclic groups such as, for example, a pyrrolyl group, a furyl group, a thienyl group, an oxazolyl group, an isoxazolyl group, a thiazolyl group, an isothiazolyl group, an imidazolyl group, a pyrazolyl group, an oxadiazolyl group, a thiadiazolyl group, a triazolyl group, a tetrazolyl group, a furazanyl group, a pyridyl group, a pyridazinyl group, a pyrimidinyl group, a pyrazinyl group and a triazinyl group; and nonaromatic heterocyclic groups such as, for example, a dihydrothienyl group, a tetrahydrothienyl group, a pyrrolinyl group, a pyrrolidinyl group, an imidazolidinyl group, an imidazolinyl group, a piperidinol group, a piperazinyl group, an oxazolinyl group, an oxazolidinyl group, an isoxazolinyl group, an isoxazolidinyl group, a thiazolinyl group, a thiazolidinyl group, an isothiazolinyl group, an isothiazolidinyl group, a 1,2-dithiolanyl group, a 1,3-dithiolanyl group, a 1,2-dithiolyl, a 1,3-dithiolyl group, a dihydrothiopyranyl group, a tetrahydrothiopyranyl group, a 1,4-dithianyl group, a 1,4-dithiinyl group, a 1,4-oxathiinyl group and a thiomorpholinyl group.

As the "mono-C₁-C₆ alkylamino groups", there can, for example, be mentioned a methylamino group, an ethylamio group, a propylamino group, an isopropylamino group, a butylamino group, a pentylamino group, a hexylamino group, etc., and as the "di-C₁-C₆ alkylamino groups" there can, for example, be mentioned a dimethylamino group, an ethylmethylamino group, a diethylamino group, an ethylpropylamino group, a dipropylamino group, a butylmethylamino group, a dibutylamino group, a butylethylamino group, a methylpentylamino group, a hexylmethylamino group, an ethylhexylamino group, etc.

The "arylamino group" means an amino group substituted by an aryl group wherein the aryl part has the above meanings, and the arylamino group can be mentioned such as, for example, a phenylamino group and a naphthylamino group.

The "halogen atoms" include a fluorine atom, a chlorine atom, a bromine atom and an iodine atom.

As the "C₁-C₆ alkylidene groups", there can be mentioned straight-chain or branched alkylidene groups having 1 to 6 carbon atoms such as, for example, a methylene group, an ethylidene group, a propylidene group, an isopropylidene group, a butylidene group, an isobutylidene group, a sec-butylidene group, a pentylidene group, an isopentylidene group, a neopentylidene group and a hexylidene group.

The "C₁-C₆ alkoxy group" means a (C₁-C₆ alkyl)-O-group wherein the C₁-C₆ alkyl part has the above meaning, and examples thereof are a methoxy group, an ethoxy group, a propoxy group, an isopropoxy group, a butoxy group, an isobutoxy group, a sec-butoxy group, a tert-butoxy group, a pentoxy group, an isopentoxy group, a neopentoxy group, a hexoxy group, etc.

The "C₁-C₆ alkyloxycarbonyl group" means a (lower alkoxy)-CO-group wherein the C₁-C₆ alkoxy part has the above meaning, and examples thereof are a methoxycarbonyl group, an ethoxycarbonyl group, a propyloxycarbonyl group, an isopropyloxycarbonyl group, a butyloxycarbonyl group, an isobutyloxycarbonyl group, a pentyloxycarbonyl group, a hexyloxycarbonyl group, etc.

The "aralkoxy group" means a C₁-C₆ alkoxy group substituted by an aryl group wherein the aryl and lower alkoxy parts have the aforesaid meanings, respectively, and examples thereof are a benzyloxy group, a phenethyloxy group, a phenylpropoxy group, an α-naphthylmethoxy group, a β-naphthylmethoxy group, a naphthylethoxy group, a tetrahydronaphthylmethoxy group, etc.

Mentioned as examples of the protective group in the "optionally substituted hydroxyl group" are alkanoyl groups having 2 to 6 carbon atoms such as an acetyl group, a propionyl group and a butyryl group; aroyl groups such as a benzoyl group; substituted or unsubstituted aralkyl groups such as a benzyl group and a 4-methoxybenzyl group; groups forming an acetal such as acetonide; etc.

The "pentose group" and "hexose group" mean a pentose group and a hexose group the hydroxyl groups of which may be substituted by the same or different 1 to 3 groups selected from the group consisting of hydrogen atoms, C₁-C₆ alkyl groups, C₁-C₆ alkylcarbonyloxy groups, lower alkoxy groups and amino groups, or oxidized, and there can be mentioned groups derived from pentoses such as, for example, ribose, arabinose, xylose and 2-deoxyribose, and groups derived from hexoses such as, for example, allose, glucose, mannose, galactose, glucosamine, galactosamine, 2-deoxyglucose, 4-O-methylglucose, rhamnose and glucuronic acid.

Preferred among the compounds of the aforesaid formula [I] provided by this invention are compounds represented by the following formula 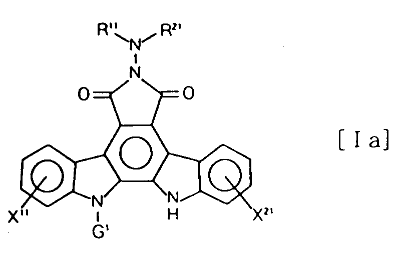
wherein
R¹¹ and R²¹ each independently represent a hydrogen atom, C₁-C₆ alkyl group, C₁-C₆ alkenyl group, aromatic hydrocarbon group having 6 to 12 carbon atoms, aralkyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group, thiazolidinyl group (the C₁-C₆ alkyl group, C₁-C₆ alkenyl group, aromatic hydrocarbon group having 6 to 12 carbon atoms, aralkyl group and hetercyclic group may have 1 to 5 substituents selected from the group consisting of carboxyl groups, carbamoyl groups, cyano groups and hydroxyl groups), or a group of the formula -Y-R³¹, and therein Y represents a carbonyl group, thiocarbonyl group or sulfonyl group, and R³¹ represents a hydrogen atom, C₁-C₆ alkyl group, aromatic hydrocarbon group having 6 to 12 carbon atoms (the C₁-C₆ alkyl group and aromatic hydrocarbon having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group or thiazolidinyl group; or
R¹¹ and R²¹ combine to represent a C₁-C₆ alkylidene group optionally having carboxyl group(s), or
R¹¹ and R²¹ combine together with the nitrogen atom to which they bind to form a pyrrolidinyl group, imidazolidinyl group, imidazolinyl group, piperidino group or piperazinyl group (these heterocyclic groups may have on the ring C₁-C₆ alkyl group(s) optionally substituted by hydroxyl group(s)),
G¹ represents a group of the formula 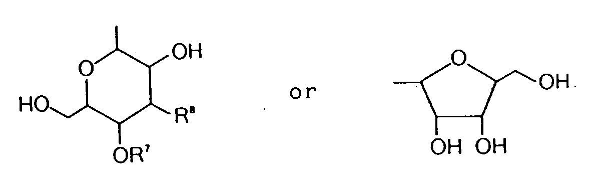
and therein R⁷ represents a hydrogen atom or C₁-C₆ alkyl group and R⁸ represents a hydroxyl group or amino group, and
X¹¹ and X²¹ bind to the indolopyrrolocarbazole ring at the 1- or 2-position and at the 10- or 11-position, respectively, and each independently represent a halogen atom, hydroxyl group, C₁-C₆ alkoxy group or aralkoxy group.

Further preferred compounds are those represented by the following formula 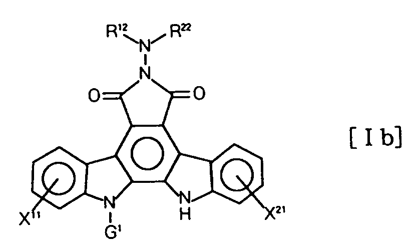
wherein
R¹² represents a hydrogen atom or C₁-C₆ alkyl group,
R²² represents a hydrogen atom, C₁-C₆ alkyl group (the C₁-C₆ alkyl group may have 1 to 5 substituents selected from the group consisting of carboxyl groups, carbamoyl groups, hydroxyl groups and cyano groups), aromatic hydrocarbon group having 6 to 12 carbon atoms, aralkyl group (the aromatic hydrocarbon group having 6 to 12 carbon atoms and aralkyl group may have 1 to 4 substituents selected from the group consisting of hydroxyl groups and carboxyl groups), pyridyl group, imidazolyl group, imidazolinyl group, thiazolyl group, pyrrolidinyl group, piperazinyl group, or a group of the formula -Y-R³², and therein Y represents a carbonyl group, thiocarbonyl group or sulfonyl group, and when Y is a carbonyl group or thiocarbonyl group, R³² represents a hydrogen atom, C₁-C₆ alkyl group, aromatic hydrocarbon group having 6 to 12 carbon atoms (the C₁-C₆ alkyl group and aromatic hydrocarbon group having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyridyl group, pyrimidinyl group, imidazolinyl group or pyrrolidinyl group, and when Y is a sulfonyl group, R³² represents a C₁-C₆ alkyl group or aromatic hydrocarbon group having 6 to 12 carbon atoms; or
R¹² and R²² combine to represent a C₁-C₆ alkylidene group having carboxyl group(s); or
R¹² and R²² combine together with the nitrogen atom to which they bind to form a pyrrolidinyl group, piperidino group or piperazinyl group (these heterocyclic groups may have on the ring C₁-C₆ alkyl groups optionally having hydroxyl group(s)), and
G¹, X¹¹ and X²¹ have the same meanings as defined in the above formula [Ia].

Preferred as G¹ is generally 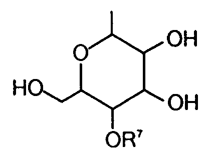
, and preferred as X¹¹ and X²¹ are hydroxyl groups bound to the 1-position and 11-position of the indolopyrrolocarbazole ring, respectively.

The compounds of this invention can exist in the form of pharmaceutical acceptable salts. Such salts include addition salts with inorganic acids such as, for example, hydrochloric acid and sulfuric acid, and with organic acids such as, for example, acetic acid, citric acid, tartaric acid and maleic acid. Further, in case the compounds of this invention contain an acidic group, the acidic group can exist in the form of alkali metal salts such as, for example, a potassium salt and a sodium salt; alkaline earth metal salts such as, for example, a magnesium salt and a calcium salt; and salts with organic bases such as an ethylamine salt and an arginine salt.

A compound of the formula [I] set forth in this invention can be prepared by reacting a compound represented by the following formula or a derivative thereof wherein the functional groups are protected 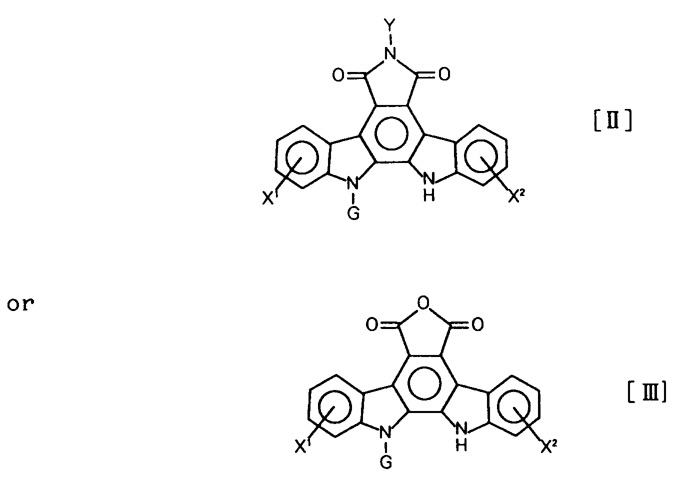
wherein,
Y represents a hydrogen atom or substituted or unsubstituted C₁-C₆ alkyl group, and X¹, X² and G have the same meanings as defined in claim 1 with a compound represented by the following general formula or a derivative thereof wherein in case R¹³ and R²³ contain a functional group, the functional group is each protected 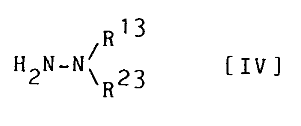
wherein
R¹³ and R²³ each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and herein Y represents a carbonyl group, thiocarbonyl group or sulfonyl group, and R³ represents a hydrogen atom, C₁-C₆ alkyl group, cycloalkyl group, cycloalkylalkyl group, aromatic hydrocarbon group having 6 to 12 carbon atoms, aralkyl group, C₁-C₆ alkoxy group, hydrazino group, amino group, arylamino group, carbamoyl group or 5-or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and heterocyclic group may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by group(s) seleceted from the group consisting of halogen atoms, hydroxyl groups, amino groups, carboxyl groups, carbamoyl groups and C₁-C₆ alkoxycarbonyl groups); or
R¹³ and R²³ combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by groups selected from the group consisting of amino groups, hydroxyl groups, carboxyl groups and sulfo groups); if necessary, removing the protective group(s) existing in the product to prepare a compound represented by the general formula 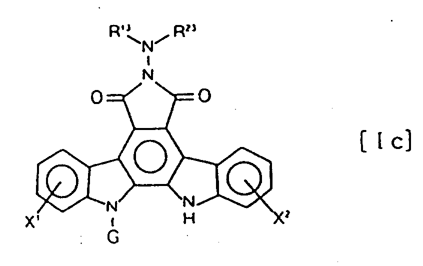
wherein R¹³, R²³, X¹, X² and G have the same meanings as defined above;
or either formylating, alkylating, alkenylating alkynylating, aralkylating, carbamoylating, thiocarbamoylating, alkanoylating or sulfonylating the amino group 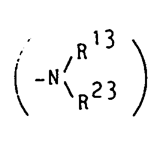
of the compound of the above formula [Ic] or the derivative thereof wherein the functional groups are protected when R¹³ and R²³ represent a hydrogen atom, or condensing the above compound or derivative with a compound represented by the following formula or a derivative thereof wherein a functional group is protected

OHC-R⁶ [V]

wherein R⁶ represents a hydrogen atom or carboxyl group, or a C₁-C₆ alkyl group optionally having 1 to 4 substituents selected from the group consisting of amino groups, mono-C₁-C₆ alkylamino groups, di-C₁-C₆ alkylamino groups, hydroxyl groups, carboxyl groups and sulfo groups,
and if necessary, removing the protective groups existing in the product; or reducing the double bonds of the compound of the above formula [Ic] when R¹³ and/or R²³ contain a double bond, or the compound prepared by condensing the compound of the formula [Ic] with the compound of the formula [V] or the derivative thereof wherein the functional groups are protected, and if necessary removing the protective group(s) existing in the product; and if necessary, converting the resulting compound of the formula [I] into a pharmaceutically acceptable salt.

Herein, the terms of alkylation, alkenylation, alkynylation, aralkylation, alkanoylation and sulfonylation are widely interpreted, and mean all of the reactions to introduce substituents corresponding to R¹ and R² in the structure of the compounds of this invention, and for example, alkylation means introduction of a substituted or unsubstituted alkyl group included in this invention.

Reaction of a compound of the formula [II] or [III] (hereafter, including a derivative thereof wherein a functional group is introduced) with a compound of the formula [IV] (hereafter, including a derivative thereof wherein its functional groups are protected) can be carried out in accordance with reaction known per se of an imide or acid anhydride with a hydrazine or a hydrazine derivative, and can, for example, be carried out in the absence of a solvent or in an inert solvent, for example a solvent such as N,N-dimethylformamide at a temperature between about 0°C and the boiling point of the solvent, preferably in the range of about room temperature to about 80°C.

The ratio of the compound of the formula [IV] used to the compound of the formula [II] or [III] is not particularly limited, and can be varied over a wide range according to the kind of the compound, reaction conditions, etc., but usually, it is suitable to use the compound of the formula [IV] in a quantity in the range of at least 1 mole, preferably 1 to 10 moles, particularly 3 to 5 moles per mole of the compound of the formula [II] or [III]. Further, when the compound of the formula [IV] is liquid in the reaction temperature, it is also possible to use the compound in a largely excessive quantity, for example in a rate of 10 to 40 moles per mole of the compound of the formula [II] or [III] so as to make it serve as a solvent.

Thereby, there can be obtained a compound of the above formula [Ic] wherein the existing functional groups are sometimes protected appropriately.

The thus obtained compound of the formula [Ic] in case R¹³ and R²³ represent a hydrogen atom or a derivative thereof wherein its functional groups are protected (hereafter, generally referred to as a compound of [Ic-1]) can be formylated, alkylated, alkenylated, alkynylated, aralkylated, carbamoylated, thiocarbamoylated, alkanoylated or sulfonylated to give a corresponding compound of the formula [Ic] in case at least one of R¹³ and R²³ represents a group except for a hydrogen atom defined on these groups.

Formylation of a compound of the formula [Ic-1] can be carried out according to a method usually used in formylation of an amino group, and can, for example, be carried out by heating it together with formic acid, formamide, dimethylformamide or the like, or by a method to react it with a mixture of formic acid and an acid anhydride in a solvent having no bad influence or without any solvent, or by another means.

Reaction of the compound of the formula [Ic-1] with formic acid, formamide, dimethylformamide or the like is usually carried out at a temperature in the range of 30°C to the boiling point of the solvent, but if necessary, can also be carried out at a temperature above or under such temperature, and reaction time is usually in the range of 30 minutes to 2 days. Preferably, the reaction is carried out usually in the presence of an acid catalyst such as hydrochloric acid or sulfuric acid.

Formylation using a mixture of formic acid with an acid anhydride is usually carried out at a comparatively low temperature in the range of -5°C to room temperature, but can, if necessary, be carried out in a range above or under this. Further, reaction time is usually 10 minutes to 5 hours, but can, if necessary, be lengthened or shortened.

Alkylation, alkenylation, alkynylation and aralkylation of a compound of the formula [Ic-1] can be carried out in accordance with a method known per se, for example, reaction with an alkylating agent, alkenylating agent, alkynylating agent or aralkylating agent such as an alkyl halide, an alkenyl halide, an alkynyl halide, an aralkyl halide, an alkyl mesylate, an alkenyl mesylate, an aralkyl mesylate, an alkyl tosylate or an aralkyl tosylate; or a method to condense it with an aldehyde compound or a ketone compound and reduce the resultant condensate; or the like. The reduction reaction at that time can be carried out according to a method using formic acid, a metal or a metal hydride or a usual method such as a catalytic reduction method using palladium-carbon or the like.

Carbamoylation and thiocarbamoylation of a compound of the formula [Ic-1] can be carried out by reacting it with a correspond isocyanate compound or thioisocyanate compound in the absence of solvent or in a suitable solvent. Reaction temperature can be in the range of about -20°C to the boiling point of the solvent, preferably about 0 to about 50°C.

Alkanoylation of a compound of the formula [Ic-1] can be carried out by a method to react it with a corresponding acid halide or an acid anhydride in the absence of a solvent or in a suitable solvent. Reaction can usually be carried out at a temperature in the range of about -5°C to the boiling point of the solvent, and if necessary, can also be carried out at a temperature below this.

The acid halide or acid anhydride is, usually, used in a rate of small excess to the compound of the formula [Ic-1], but can, if necessary, be used in a quantity below or above this, and reaction time can, usually, be 30 minutes to 2 days.

Sulfonylation of a compound of the formula [Ic-1] can be carried out by reacting it with a reagent such as a corresponding organic sulfonic acid anhydride or organic sulfonyl halide in the presence or absence of a base. Reaction temperature can, usually, be sufficient in the range of about -10°C to about 50°C, but can, if necessary, be a temperature above or under this, and reaction time can, usually, be 30 minutes to 3 days. A reagent such as an organic sulfonic acid anhydride or an organic sulfonyl halide is, usually, used in a rate of small excess, but can also be used, in a quantity above or under this.

Further, condensation reaction of a compound of the formula [Ic-1] with a compound of the above formula [V] (including a derivative thereof wherein the functional groups are protected) is so-called Schiff base formation reaction, and can, for example, usually be carried out in a solvent inert to the reaction, e.g. in a solvent such as tetrahydrofuran, at a temperature between about 0°C to the boiling point of the solvent, preferably in the range of room temperature to about 50°C. Reaction time is usually in the range of 30 minutes to 2 days, but can, if necessary, be a time above or under this.

Use quantity of the compound of the formula [V] to the compound of the formula [Ic-1] is not strictly limited, but usually, it is suitable to use the compound of the formula [V] in a rate of 1 to 50 moles, particularly 3 to 10 moles per mole of the compound of the formula [Ic-1].

The hydrazone compound obtained by the above reaction can be subjected to usual catalytic hydrogenation reaction using palladium-carbon or the like to give a compound of the formula [I] wherein R¹ or R² represents a hydrogen atom.

In the foregoing processes, protection of functional groups in raw material compounds and removal of protective groups existing in the formed compounds can be carried out using usual and optional methods widely known in the chemical field.

Further, isolation and purification of compounds produced by the above reactions can be carried out according to methods known per se in the field of organic synthetic chemistry, for example precipitation methods, solvent extraction methods, recrystallization, chromatography, etc.

A compound of the above formula [II] used as a starting raw material in the aforesaid processes can be prepared by glycosidating a compound represented by the general formula 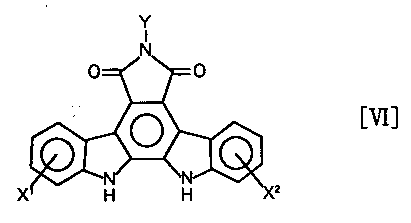
wherein X¹, X² and Y have the same meanings as defined above
prepared by a process known per se [refer to J. Chem. Soc. Perkin Transactions I. pp 2475-2480 (1990)] or a derivative thereof wherein the functional groups are protected.

Glycosidation of the compound of the formula [VI] or the derivative thereof wherein the functional groups are protected can be carried out by a process known per se [refer to J. Am. Chem. Soc. 60, 2559 (1938)], for example by condensing it with a reactive derivative of a pentose or hexose wherein the hydroxyl groups are protected, e.g. 1-bromo-2,3,4,6-O-tetraacetylglucose, using as an activating agent mercury cyanide, silver carbonate, silver oxide or the like, preferably silver oxide, in an aprotic solvent, e.g. a solvent such as benzene, toluene or methylene chloride at a temperature of about 0°C to about 100°C, preferably about 80°C.

Alternatively, a compound of the formula [II] can also be prepared according to the process disclosed in the aforesaid PCT/WO91/18003.

Further, a compound of the formula [III] can be prepared by treating with a base a thus obtained compound of the formula [II] or derivative thereof wherein the functional groups are protected.

Preferred as the base is an aqueous solution of potassium hydroxide, and treatment with this base can usually be carried out at room temperature, but in some usually be carried out at room temperature, but in some occasions, can also be carried out with heating up to a temperature of about 50°C.

Neutralization or acidification of the reaction mixture can, if necessary, be carried out using hydrochloric acid, and thereby it is possible to precipitate the compound of the formula [III] as crystals.

The compounds of the formula [I] provided by this invention have an excellent antitumor action as shown in the following pharmacological test examples.

### (1) Therapeutic effect against mouse tumor (P388)

Therapeutic effect of the compounds of this invention against mouse tumor (P388) is shown in Tables 1 and 2.

**Table 1**

| Effect of the compound of Example 2 against P388 | | | |
|---|---|---|---|
| Tumor⁽¹⁾ | Dose ⁽²⁾, i.p. (mg/kg/injection) | MST⁽³⁾(day) | T/C (%)⁽⁴⁾ |
| P388 | 0 | 12.3 ± 1.06 | 100 |
| | 1 | 15.8 ± 0.84 | 128 |
| | 3 | 17.8 ± 1.92 | 145 |
| | 10 | >26.4 ± 18.82 | >245 |
| | 30 | >42.2 ± 24.38 | >343 |
| | 100 | >47.2 ± 18.90 | >384 |
| (Footnotes of Tables 1 and 2) | | | |

| | | | |
|---|---|---|---|
| (1) Tumor inoculation : 10⁶ cancer cells were intraperitoneally inoculated. | | | |
| (2) Dose: After tumor inoculation, each dose was intraperitoneally administered once a day from the 1st day to 10th day. | | | |
| (3) MST: mean survival number of days | | | |
| (4) T/C (%): (MST of treatment group/MST of control) x 100 | | | |
| (5) Standard: in case of T/C ≧ 125, the test compound was judged to have a remarkable antitumor effect in the dose. | | | |

**Table 2**

| Effect of the compound of Example 5 against P388 | | | |
|---|---|---|---|
| Tumor⁽¹⁾ | Dose ⁽²⁾, i.p. (mg/kg/injection) | MST⁽³⁾(day) | T/C (%)⁽⁴⁾ |
| P388 | 0 | 12.3 ± 0.95 | 100 |
| | 1 | 16.8 ± 0.84 | 137 |
| | 3 | 17.8 ± 1.92 | 145 |
| | 10 | >26.2 ± 10.18 | >213 |
| | 30 | >23.4 ± 9.74 | >190 |
| | 100 | >36.4 ± 8.05 | >296 |
| (Footnotes of Tables 1 and 2) | | | |

| | | | |
|---|---|---|---|
| (1) Tumor inoculation : 10⁶ cancer cells were intraperitoneally inoculated. | | | |
| (2) Dose: After tumor inoculation, each dose was intraperitoneally administered once a day from the 1st day to 10th day. | | | |
| (3) MST: mean survival number of days | | | |
| (4) T/C (%): (MST of treatment group/MST of control) x 100 | | | |
| (5) Standard: in case of T/C ≧ 125, the test compound was judged to have a remarkable antitumor effect in the dose. | | | |

### (2) Proliferation inhibition activity against mouse leukemia cell

### Measurement method:

100 µl portions of a cell culturing medium (10% fetal bovine serum-containing-RPMI-1640 medium) containing 3x10³ mouse leukemia cell (P388) were put in a 96-hole microplate, the cells were cultured under 5% CO₂ at 37°C for 24 hours, 10 µl each of test solutions containing test compounds respectively were added respectively, and the cells were further cultured under 5% CO₂ at 37°C for 24 hours. 10 µl portions of 0.5% Thiazoyl Blue were added to the culture broths, and incubation was carried out under 5% CO₂ at 37°C for 2 hours to carry out enzymatic reaction. 20% sodium dodecyl sulfate (SDS) was added to discontinue the reaction, incubation was further carried out at 37°C for 4 hours to dissolve the formed dye, and absorbance at 550 nm was measured and compared with the control group. The results are shown in Table 3.

**Table 3**

| Proliferation inhibition activity against mouse leukemia cell P388 | |
|---|---|
| Test compound | 50% inhibitory concentration (IC₅₀, µM) |
| Compound of Example 1 | <0.030 |
| Compound of Example 2 | 0.29 |
| Compound of Example 3 | 0.065 |
| Compound of Example 4 | 0.096 |
| Compound of Example 5 | 0.28 |
| Compound of Example 6 | 0.059 |
| Compound of Example 7 | 0.091 |
| Compound of Example 8 | 0.30 |
| Compound of Example 9 | 0.028 |
| Compound of Example 10 | 0.46 |
| Compound of Example 11 | <0.026 |
| Compound of Example 12 | 0.042 |
| Compound of Example 13 | 0.22 |
| Compound of Example 14 | <0.027 |
| Compound of Example 15 | 0.31 |
| Compound of Example 17 | 0.044 |
| Compound of Example 22 | 0.11 |
| Compound of Example 23 | <0.025 |
| Compound of Example 24 | 0.001 |
| Compound of Example 25 | 0.048 |
| Compound of Example 27 | 0.027 |
| Compound of Example 28 | <0.029 |
| Compound of Example 29 | 0.005 |
| Compound of Example 30 | 0.003 |
| Compound of Example 31 | 0.011 |
| Compound of Example 33 | 0.11 |
| Compound of Example 34 | 0.019 |
| Compound of Example 35 | 0.17 |
| Compound of Example 36 | 0.002 |
| Compound of Example 37 | 0.095 |

As apparent from the results of the above pharmacological test, the compounds of this invention exhibit an excellent antitumor action, and are useful as an antitumor agent for control or prevention of diseases, particularly for treatment of cancers. When a compound of this invention is used in such uses, it is, usually, formulated into a pharmaceutical preparation comprising an effective quantity of it and a pharmaceutically acceptable carrier or diluent.

As administration forms at the time of use of a compound of this invention, various forms can be selected, and there can be mentioned oral agents such as, for example, tablet, capsuls, powders, granules or liquids, or sterilized liquid parenteral agents such as, for example solutions or suspensions, or suppositories, ointments, or the like.

Solid preparations can be prepared, as they are, as forms of tablets, capsuls, granules or powders, or can also be prepared using suitable additives. Such additives may be additives usually used, and include saccharides such as, for example, lactose and glucose; starches such as, for example, corn, wheat and rice; fatty acids such as, for example, stearic acid; inorganic salts such as, for example, magnesium metasilicate aluminate and anhydrous calcium phosphate; synthesized macromolecules such as, for example, polyvinylpyrrolidone and polyalkylene glycol; fatty acid salts such as, for example, calcium stearate and magnesium stearate; alcohols such as, for example, stearyl alcohol and benzyl alcohol; synthesized cellulose derivatives such as, for example, methylcellulose, carboxymethylcellulose, ethylcellulose and hydroxypropylmethylcellulose; and further, gelatin, talc, vegetable oils, gum arabic, etc.

Solid preparations such as these tablets, capsuls, granules and powders contain an effective ingredient generally at 0.1-100 weight %, preferably at 5-100 weight %.

Liquid preparations are prepared in forms such as suspensions, syrups, injections or drops using suitable additives usually used in liquid preparations such as water, alcohols or oils originated in vegetables such as, for example, soybean oil, peanut oil and sesame oil.

Particularly, solvents suitable in case of parenteral administration in the form of intramuscular injection, intravenous injection or subcutaneous injection include, for example, distilled water for injection, aqueous lidocaine hydrochloride solutions (for intramuscular injection), physiological saline, aqueous glucose solutions, ethanol, polyethylene glycol, liquids for intravenous injection (e.g. aqueous solutions of citric acid and sodium citrate, etc.), electrolyte solutions (for intravenous drip and intravenous injection), etc., and their mixed solvents.

These injections can take forms that powder itself or to which suitable additives were added is dissolved at the time of use, besides such forms that ingredients are dissolved in advance. Such an injection contains usually 0.1-10 weight %, preferably 1-5 weight % of the effective ingredient.

Further, a liquid agent of a suspension, syrup or the like for oral administration can usually contain 0.5-10 weight % of the effective ingredient.

The preferred dose of the compounds of this invention can be varied according to the kind of a compound to be used, the kind and application frequency of the compounded composition, the specified site to be treated, the degree of diseases, the age of patients, diagnosis of doctors, the kind of tumor, etc., but, as an approximate standard, the dose per day and per one adult can, for example, be in the range of 10 to 500 mg in case of oral administration, and in the range of 10 to 100 mg in case of parenteral administration, preferably intravenous injection. The administration frequency varies depending on administration methods and symptoms, but is 1 to 5 times a day. Further, there can also be adopted administration methods such as intermittent administration, e.g. every second day administration or every third day administration.

This invention is more specifically described below by examples, but not limited only by these examples.

### Example A

The compound represented by the formula 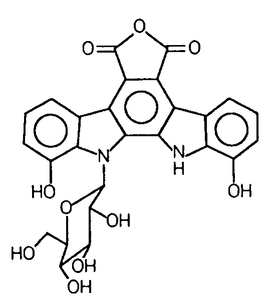
3.4 g of 12,13-dihydro-1,11-dihydroxy-13-(β-D-glucopyranosyl)-5H-indolo[2,3-a]pyrrolo[3,4-c]carbazole-5,7(6H)-dione was dissolved in 120 ml of 10% aqueous potassium hydroxide solution, and the solution was stirred at room temperature for 2 hours. The reaction solution was neutralized with addition of 120 ml of 2N hydrochloric acid, and the precipitated red crystals were filtered, washed with water and dried to give 3.0 g of the captioned compound.
FAB-MS(m/z): 520 (M)⁺, 521 (M+H)⁺
¹H-NMR (400 MHz, DMSO-d₆), δ(ppm): 3.42 (1H,m), 3.56-3.70 (2H,m), 3.76 (1H,m), 3.95-4.10 (2H,m), 4.95 (1H,d,J=4.6Hz), 5.24 (1H,d,J=5.4Hz), 5.32 (1H,dd,J=4.9, 5.1Hz), 7.06 (2H,dd,J=7.6, 7.8Hz), 7.09 (1H,d,J=8.0Hz), 7.20 (1H,d,J=7.8 Hz), 7.40 (1H,d,J=7.8 Hz), 8.36 (1H,d,J=7.6Hz), 8.51 (1H,d,J=7.6Hz), 10.13 (1H,s), 10.52 (1H,s), 11.11 (1H,s)

### Example B

The compound represented by the formula 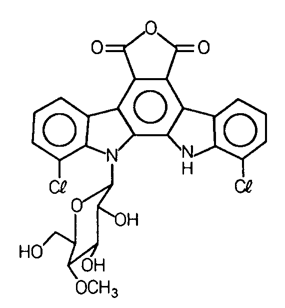
50 mg of rebeccamycin was dissolved in 5 ml of N,N-dimethylformamide, 5 ml of 2N aqueous sodium hydroxide solution was added, and the mixture was stirred at 80°C for 3 hours. 60 ml of water was added to the reaction solution, the mixture was cooled with ice, and the precipitated yellow precipitate was recovered by filtration. This was subjected to column chromatography on silica gel (inner diameter 1.5 cm, length 45 cm), the column was washed with chloroform, elution was carried out with chloroform-tetrahydrofuran (10:1), and the fraction containing the desired product was concentrated to dryness. The resultant yellow powder was washed with chloroform to give 6.4 mg of the captioned compound.

Rf value: 0.51 (produced by Merck Co., Kiesel gel 60F₂₅₄, developing solvent; chloroform : methanol : tetrahydrofuran : acetic acid = 10:1:1:0.2)
FAB-MS(m/z): 571 [M+H]⁺
¹H-NMR (300 MHz, DMSO-d₆), δ(ppm): 10.9 (1H,s), 9.07 (1H,d,J=7.8Hz), 8.92 (1H,d,J=7.8Hz), 7.78 (2H,t,J= 7.8Hz), 7.53 (1H,d,J=7.8Hz), 7.50 (1H,d,J=7.8,Hz), 7.03 (1H,d,J=8.9Hz), 3.96 (2H,m), 3.87 (1H,m), 3.61 (3H,s), 3.54-3.73 (3H,m),

### Example 1

The compound represented by the formula 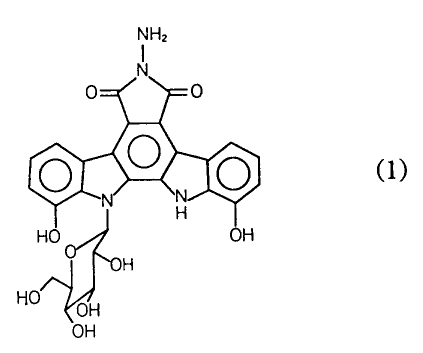
3.51 g of 12,13-dihydro-1,11-dihydroxy-13-(β-D-glucopyranosyl)-5H-indolo[2,3-a]pyrrolo[3,4-c]carbazole-5,7(6H)-dione was dissolved in 8 ml of hydrazine hydrate (Wako Pure Chemical Industries, Ltd.), and reaction was carried out at room temperature for 2 hours. After the reaction, 180 ml of purified water was added, the pH of the solution was adjusted to 5.0 with concentrated hydrochloric acid, the mixture was sufficiently cooled with ice, and the resulting precipitate was collected by filtration, washed with purified water and dried under reduced pressure to give 3.51 g of the captioned compound represented by the formula (1). (yield : 97%)
FAB-MS (m/z): 535 (M+H)⁺
¹H-NMR (400 MHz, DMSO-d₆), δ(ppm): 10.9 (1H, brs), 10.4 (1H, s), 10.0 (1H, s), 8.72 (1H, d, J=7.8Hz), 8.54 (1H, d, J=7.8Hz), 7.19 (2H, t, J=7.8Hz), 7.19 (2H, t, J=7.8Hz), 7.05 (1H, d, J=9.3Hz), 7.02 (1H, d, J=7.8Hz), 7.00 (1H, d, J=7.8Hz), 5.42 (1H, brd, J=5.8Hz), 5.35 (1H, brs), 5.22 (1H, brd, J= 4.4Hz), 4.96 (2H, brs), 4.91 (1H, brd, J=5.3Hz), 4.01 (2H, m), 3.73 (1H, m), 3.63 (2H, m), 3.39 (1H, m)

### Example 2

The compound represented by the formula 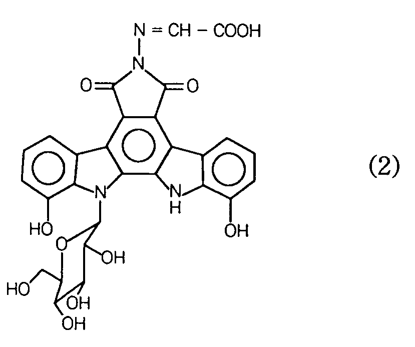
3.47 g of the compound obtained in Example 1 was dissolved in 20 ml of N,N-dimethylformamide (DMF), while the solution was stirred at room temperature, 20 ml of a 100 mg/ml solution of glyoxylic acid (Sigma Co.) was added portionwise, and thereby a precipitate was formed and solidified into a gel-like state. Further, 200 ml of purified water was added, the reaction solution was cooled with ice, and the resultant precipitate was collected by filtration, washed with purified water and dried under reduced pressure to give 3.85 g of the captioned compound represented by the formula (2). (yield : 100%)
FAB-MS(m/z) : 591 (M+H)⁺
¹H-NMR (400 MHz, DMSO-d₆), δ(ppm): 11.1 (1H, brs), 10.5 (1H, brs), 10.1 (1H, brs), 9.01 (1H, s), 8.69 (1H, d, J=7.8Hz), 8.53 (1H, d, J=7.8Hz), 7.23 (2H, t, J=7.8Hz), 7.10 (1H, d, J=9.3Hz), 7.06 (1H, d, J=7.8Hz), 7.04 (1H, d, J=7.8Hz), 5.44 (1H, brs), 5.34 (1H, brs), 5.24 (1H, brs), 4.95 (1H, brd, J= 5.9Hz), 4.02 (2H, m), 3.76 (1H, m), 3.64 (2H, m), 3.40 (1H, m)

### Example 3

The Compound represented by the formula 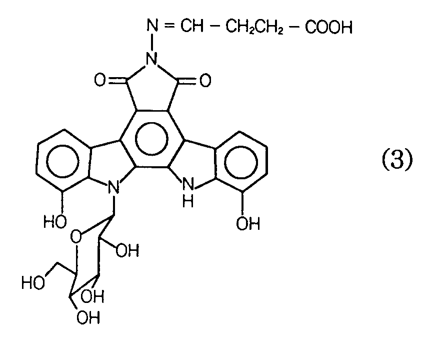
24 mg of the compound obtained in Example 1 was dissolved in 0.5 ml of N,N-dimethylformamide (DMF), while the solution was stirred at room temperature, 0.2 ml of 15% succinic semialdehyde (Aldrich Chemical Co.) was added, and one hour later, 5 ml of purified water was added. After the reaction solution was cooled with ice, and the resultant precipitate was collected by filtration, washed with purified water and dried under reduced pressure to give 25.3 mg of the captioned compound represented by the formula (3). (yield : 91%)
FAB-MS(m/z): 619 (M+H)⁺
¹H-NMR (400 MHz, DMSO-d₆), δ(ppm): 12.1 (1H, brs), 11.0 (1H, brs), 10.4 (1H, brs), 10.0 (1H, brs), 8.69 (1H, brs), 8.68 (1H, d, J=7.8Hz), 8.51 (1H, d. J=8.3Hz), 7.19 (2H, t, J=7.8Hz), 7.07 (1H, d, J=9.3Hz), 7.04 (1H, d, J=7.8Hz), 7.01 (1H, d, J=7.8Hz), 5. 43 (1H, brd. J=5.4Hz), 5.33 (1H, brs), 5.22 (1H, brs), 4.93 (1H, brd, J=4.9Hz), 4.01 (2H, m), 3.74 (1H, m), 3.63 (2H, m), 3.40 (1H, m)

### Example 4

The compound represented by the formula 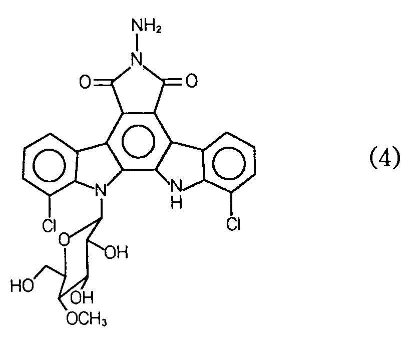
511 mg of rebeccamycin [the compound described in J. Antibiotics 40, 668-678 (1987)] was dissolved in 3 ml of hydrazine hydrate (Wako Pure Chemical Industries, Ltd.), and the solution was allowed to stand at room temperature for one hour. 200 ml of purified water was added, and the resultant precipitate was collected by filtration, washed with 100 ml of purified water and dried under reduced pressure to give 497 mg of the captioned 6-N-aminorebeccamycin represented by the formula (4). (yield : 95%)
FAB-MS(m/z): 585 (M+H)⁺
¹H-NMR (400 MHz, DMSO-d₆), δ(ppm): 10.64 (1H, brs), 9.24 (1H, d, J=7.8Hz), 9.07 (1H, d, J=7.8Hz), 7.70 (2H, t, J=7.8Hz), 7.45 (1H, d, J=7.8Hz), 7.42 (1H, d, J=7.8Hz), 6.93 (1H, d, J=8.8Hz), 5.42 (1H, d, J=5.8Hz), 5.33 (1H, t, J=5.4Hz), 5.03 (3H, brs), 3.97 (2H, m), 3.84 (1H, m), 3.59 (3H, s), 3.50∼3.70 (3H, m)

### Example 5

The compound represented by the formula

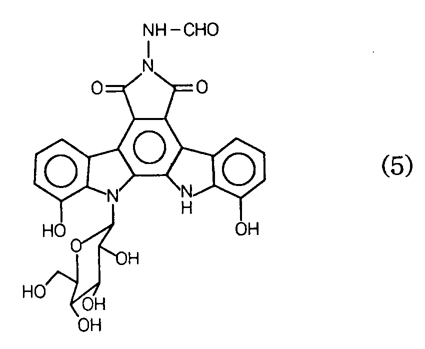

### [Process A]

5 g of the compound obtained in Example 1 was dissolved in 60 ml of N,N-dimethylformamide, 1.8 ml of concentrated hydrochloric acid was added, the mixture was heated at 60°C for 4 hours, 0.8 ml of concentrated hydrochloric acid was further added, and the mixture was warmed at 37°C for 16 hours. This was mixed with 1 l of ethyl acetate, the mixture was washed successively with 2% sodium bicarbonate aqueous solution and water, and then the ethyl acetate layer was dehydrated with anhydrous sodium sulfate and concentrated to dryness to give 3.3 g of orange powder. This was dissolved in methanol and subjected to column chromatography on Sephadex LH 20 (inner diameter 3 cm, length 54 cm, eluted with methanol), and the fractions containing the desired product were concentrated to dryness to give 2413.6 mg of the captioned compound represented by the formula (5) as orange powder.

### [Process B]

25.9 mg of the compound obtained in Example A was dissolved in 0.5 ml of N,N-dimethylformamide, 15.0 mg of formohydrazide was added, and the mixture was stirred at 70°C for 2 hours. This was mixed with 70 ml of ethyl acetate, and the mixture was washed with water (20 ml). The ethyl acetate layer was dehydrated with anhydrous sodium sulfate and concentrated to dryness to give 26.9 mg of orange powder. This was dissolved in methanol and subjected to column chromatography on Sephadex LH 20 (inner diameter 1.5 cm, length 48 cm, eluted with methanol), and the fractions containing the desired product were concentrated to dryness to give 16.3 mg of the captioned compound represented by the formula (5) as orange powder.

Rf value: 0.35 (produced by Merck Co., Kiesel gel 60F₂₅₄, developing solvent; chloroform-methanol-tetrahydrofuran = 2:1:1)
FAB-MS(m/z): 562 (M)⁺
¹H-NMR (400 MHz, DMSO-d₆), δ(ppm): 11.0 (1H, brs), 10.8 (1H, s), 10.4 (1H, s), 10.0 (1H, s), 8.64 (1H, d, J=8.3Hz), 8.47 (1H, d, J=8.3Hz), 8.44 (1H, s), 7.21 (2H, t, J=7.8Hz), 7.06 (1H, d, J=9.7Hz), 7.05 (1H, d, J= 7.8Hz), 7.02 (1H, d, J=7.8Hz), 5.43 (1H, d, J=5.8Hz), 5.36 (1H, brs), 5.22 (1H, d. J=5.4Hz), 4.92 (1H, d. J=5.4Hz), 4.02 (2H, m), 3.75 (1H, m), 3.62 (2H, m), 3.39 (1H, m)

### Example 6

The compound represented by the formula 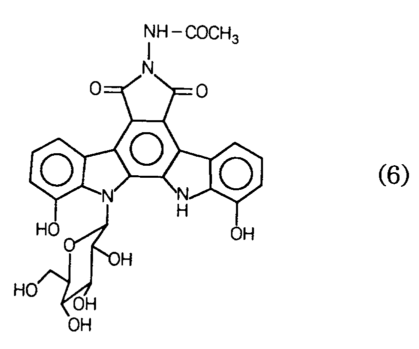
30 ml of acetic acid and 2 ml of acetic anhydride were added to 510 mg of the compound obtained in Example 1, and the compound was dissolved therein with heating at 90°C. Water was added thereto to make the mixture 300 ml, and the reaction product was adsorbed on a column of Diaion HP 20 (inner diameter 3 cm, length 13.5 cm) and after washing the column with 600 ml of water, eluted with 300 ml of methanol. The methanol eluate was concentrated to dryness, the residue was dissolved in 50 ml of methanol, and the solution was concentrated to about 5 ml. 100 ml of ethyl acetate was added thereto, the mixture was allowed to stand overnight at 4°C, and the resultant orange precipitate was collected by filtration to give 426 mg of the captioned compound represented by the formula (6).

Rf value: 0.43 (produced by Merck Co., Kiesel gel 60F₂₅₄, developing solvent; chloroform-methanol-tetrahydrofuran = 2:1:1)
FAB-MS(m/z): 576 (M)⁺
¹H-NMR (400 MHz, DMSO-d₆), δ(ppm): 11.0 (1H, s), 10.7 (1H, s), 10.4 (1H, brs), 10.05 (1H, s), 8.64 (1H, d, J=7.8Hz), 8.47 (1H, d, J=7.8Hz), 7.20 (2H, t, J=7.8Hz), 7.01-7.06 (3H, m), 5.35-5.45 (2H, m), 5.23 (1H, brs), 4.92 (1H, brs), 4.02 (2H, m), 3.74 (1H, m), 3.58-3.70 (2H, m), 3.40 (1H, m), 2.10 (3H, s)

### Example 7

The compound represented by the formula 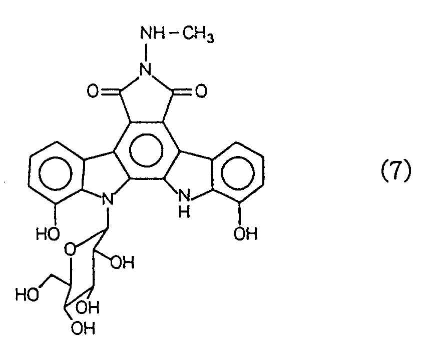
72.5 mg of the compound obtained in Example 1 was dissolved in a mixture of 8 ml of tetrahydrofuran and 5 ml of methanol, 140 µl of 2N hydrochloric acid and 13.2 µl of 37% formaldehyde aqueous solution were added, and the mixture was stirred at room temperature for 2 hours and concentrated to dryness. This was dissolved in 5 ml of N,N-dimethylformamide, 80 mg of 10 % palladium carbon were added, and the mixture was subjected to reduction under hydrogen gas at room temperature for 2 hours and then filtered on Celite. 80 ml of ethyl acetate was added to the resultant filtrate, the mixture was washed successively with 2% sodium bicarbonate aqueous solution and water, and then the resultant ethyl acetate layer was dehydrated and concentrated to dryness to give 28.8 mg of orange powder. This was dissolved in a small quantity of methanol, and the solution was subjected to column chromatography on Sephadex LH-20 (inner diameter 1.5 cm, length 90 cm, eluted with methanol) to give 17.1 mg of the captioned compound represented by the formula (7) as orange powder.

Rf value: 0.49 (produced by Merck Co., Kiesel gel 60F₂₅₄, developing solvent; chloroform-methanol-tetrahydrofuran-acetic acid = 20:10:10:1)
FAB-MS(m/z): 549 (M+H)⁺
¹H-NMR (400 MHz, DMSO-d₆), δ(ppm): 10.9 (1H, s), 10.4 (1H, s), 9.98 (1H, s), 8.72 (1H, d, J=7.8Hz), 8.54 (1H, d, J=7.8Hz), 7.19 (2H, t, J=7.8Hz), 7.00-7.06 (3H, m), 5.73 (1H, q, J=5.4Hz), 5.43 (1H, d, J=5.7Hz), 5.35 (1H, brs), 5.22 (1H, d, J=5.4Hz), 4.90 (1H, d, J=5.4Hz), 3.96-4.03 (2H, m), 3.74 (1H, m), 3.58-3.70 (2H, m), 3.40 (1H, m), 2.74 (3H, d. J=5.4Hz)

### Example 8

The compound represented by the formula 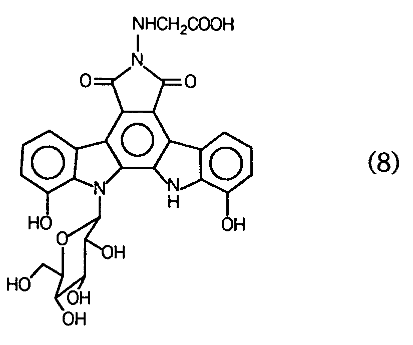
500 mg of the compound obtained in Example 2 was dissolved in 6 ml of N,N-dimethylformamide(DMF), 75 mg of 10 % palladium-carbon (Pd-C) was added, and hydrogenation was carried out at room temperature for 3.5 hours under the stirring. The reaction mixture was filtered using filter paper on which diatom earth was spread to remove Pd-C, and 150 ml of water was added to the filtrate. The mixture was adjusted to pH 5 with 1N NaOH, and then extracted with ethyl acetate (200 ml x 5). The ethyl acetate layer was concentrated, and the precipitated crystals were collected by filtration to give 182.3 mg of the captioned compound represented by the formula (8).
FAB-MS(m/z): 593 (M+H)⁺
¹H-NMR (400 MHz, DMSO-d₆), δ(ppm): 12.6 (1H, brs), 10.9 (1H, s), 10.4 (1H, s), 10.0 (1H, s), 8.69 (1H, d, J=8.3Hz), 8.52 (1H, d, J=7.8Hz), 7.18 (2H, t, J=7.8Hz), 6.99∼7.05 (3H, m), 5.90 (1H, brs), 5.42 (1H, d, J=5.4Hz), 5.35 (1H, t, J=5.4Hz), 5.21 (1H, d, J= 4.9Hz), 4.89 (1H, d, J=5.4Hz), 4.03 (2H, m), 3.83 (2H, s), 3.74 (1H, m), 3.63 (2H, m), 3.39 (1H, m)

### Example 9

The compound represented by the formula 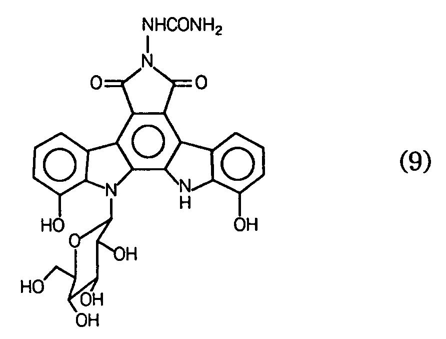
30 ml of methanol was added to 501.7 mg of the compound obtained in Example A and 501.7 mg of semicarbazide hydrochloride, 0.325 ml of triethylamine was then added, and the mixture was refluxed with heating for 8 hours. After the reaction, the reaction solution was concentrated to dryness, 300 ml of methyl ethyl ketone (MEK) and 200 ml of water were added, extraction operation was carried out, and another 300 ml of MEK was added to the water layer to carry out reextration. The MEK layers were combined and concentrated to dryness, 300 ml of methanol was added to the residue to dissolve it, the solution was subjected to a chromatograph tower of Sephadex LH-20 (3 x 28 cm), and elution was carried out with methanol. The fractions containing the desired product were concentrated to dryness to give 461 mg of the captioned compound represented by the formula (9) as red crystal-like powder.

Rf value: 0.15 (produced by Merck Co., Kiesel gel 60F₂₅₄, developing solvent; chloroform : methanol : tetrahydrofuran = 2:1:1)
FAB-MS (m/z): 577 (M)⁺
¹H-NMR (400 MHz, DMSO-d₆), δ(ppm): 11.0 (1H, s), 10.4 (1H, s), 10.0 (1H, s), 8.68 (1H, d, J=7.8Hz), 8.66 (1H, brs), 8.51 (1H, d, J=7.8Hz), 7.20 (2H, t, J=7.8Hz), 7.01∼ 7.07 (3H, m), 6.41 (2H, brs), 5.44 (1H, d, J= 5.4Hz), 5.38 (1H, brs), 5.23 (1H, d, J=4.9Hz), 4,91 (1H, brs), 4.00∼4.09 (2H, m), 3.75 (1H, m), 3.60∼3.68 (2H, m), 3.39 (1H, m)

### Example 10

The compound represented by the formula 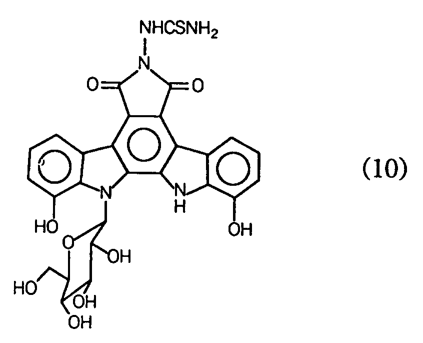
4 ml of methanol was added to 22 mg of the compound obtained in Example A and 20 mg of thiosemicarbazide, and the mixture was refluxed with heating for 22 hours. The reaction solution was concentrated to dryness, the residue was dissolved in 4 ml of methanol, the solution was subjected to a chromatograph tower of Sephadex LH-20 (1.8 x 35 cm), and elution was carried out with methanol. The fractions containing the desired product were concentrated to dryness to give 10.7 mg of the captioned compound represented by the formula (10).

Rf value: 0.29 (produced by Merck Co., Kiesel gel 60F₂₅₄, developing solvent; chloroform : methanol : tetrahydrofuran = 2:1:1)
FAB-MS(m/z): 594 (M+H)⁺
¹H-NMR (400 MHz, DMSO-d₆), δ(ppm): 11.0 (1H, s), 10.4 (1H, brs), 10.1 (1H, brs), 9.73 (1H, brs), 8.65 (1H, d, J=7.8Hz), 8.49 (1H, d, J=7.8Hz), 8.27 (2H, s), 7.21 (2H, t, J=7.8Hz), 7.01∼7.12 (3H, m), 5.45 (1H, brs), 5.37 (1H, brs), 5.24 (1H, brs), 4.91 (1H, brs), 3.97∼4.10 (2H, m), 3.74 (1H, m), 3.62 (2H, m), 3.40 (1H, m)

### Example 11

The compound represented by the formula 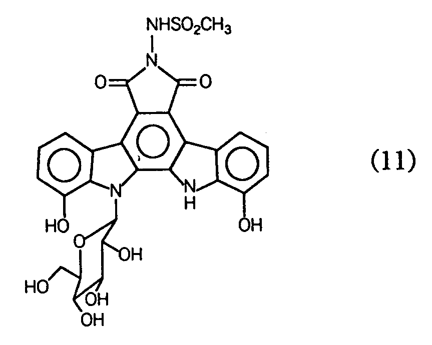
9.5 mg of the compound obtained in Example 1 was dissolved in 2 ml of tetrahydrofuran (THF), to the solution was added 30 mg of methanesulfonic anhydride (Aldrich Chemical Co.), and the mixture was allowed to stand at room temperature for 48 hours. The reaction solution was concentrated to dryness, and the residue was dissolved in 2 ml of methanol, subjected to a chromatograph tower of Sephadex LH-20 (1.8 x 34 cm), and eluted with methanol. The fractions containing the desired product were concentrated to dryness to give 8.3 mg of the captioned compound represented by the formula (11).

Rf value: 0.48 (produced by Merck Co., Kiesel gel 60F₂₅₄, developing solvent; chloroform : methanol : tetrahydrofuran = 2:1:1)
FAB-MS(m/z): 612 (M)⁺
¹H-NMR (400 MHz, DMSO-d₆), δ(ppm): 11.0 (1H, s), 10.5 (1H, brs), 10.4 (1H, s), 10.1 (1H, s), 8.67 (1H, d, J=7.9Hz), 8.50 (1H, d, J=7.7Hz), 7.22 (2H, t, J=7.6Hz), 7.02∼7.07 (3H, m), 5.43 (1H, d, J=5.8Hz), 5.36 (1H, brs), 5.22 (1H, d, J=5.2Hz), 4.89 (1H, d, J= 4.8Hz), 4,03 (2H, m), 3.75 (1H, m), 3.63 (2H, m), 3.40 (1H, m)

### Example 12

The compound represented by the formula 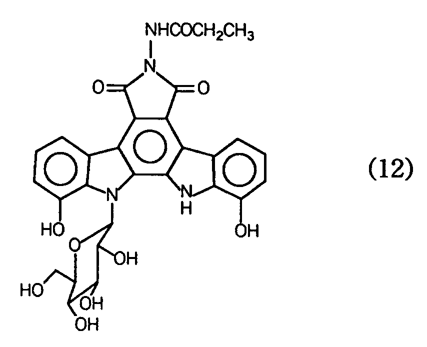
1 ml of the methanol and 2 ml of tetrahydrofuran were added to 11.7 mg of the compound obtained in Example 1 to make a solution, 0.1 ml of propionic anhydride (Aldrich Chemical Co.) was added, and the mixture was stirred at room temperature for 4 hours. 2 ml of water and 3 ml of methanol were added to the reaction solution, the mixture was allowed to stand for 30 minutes and then concentrated to dryness, and 3 ml of methanol was added to make a solution. The solution was subjected to a chromatograph tower of Sephadex LH-20 (1.8 x 30 cm) and eluted with methanol, and the fractions containing the desired product were concentrated to dryness to give 6.2 mg of the captioned compound represented by the formula (12).

Rf value: 0.55 (produced by Merck Co., Kiesel gel 60F₂₅₄, developing solvent; chloroform : methanol : tetrahydrofuran = 2:1:1)
¹H-NMR (400 MHz, DMSO-d₆), δ(ppm): 11.0 (1H, s), 10.6 (1H, brs), 10.4 (1H, brs), 10.0 (1H, s), 8.64 (1H, d, J=7.8Hz), 8.47 (1H, d, J=7.8Hz), 7.20 (2H, t, J=7.8Hz), 7.00∼ 7.08 (3H, m), 5.30∼5.45 (2H, m), 5.21 (1H, m), 4.92 (1H, m), 4.02 (2H, m), 3.75 (1H, m), 3.62 (2H, m), 3.38 (1H, m), 2.39 (2H, q, J=9.3Hz), 1.16 (3H, t, J=7.3Hz)

### Example 13

The compound represented by the formula 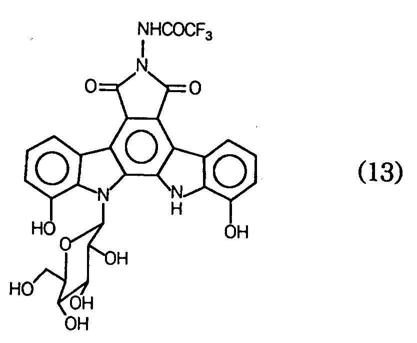
9.9 mg of the compound obtained in Example 1 was dissolved in 2 ml of tetrahydrofuran, 0.06 ml of trifluoroacetic anhydride (Aldrich Chemical Co.) was added, and the mixture was allowed to stand at room temperature for 15 minutes. 2 ml of water was added to the reaction solution, the mixture was concentrated to dryness, 2 ml of water and 10 ml of ethyl acetate were added, extraction operation was carried out, and the resultant ethyl acetate layer was concentrated to dryness. The resultant crude substance was dissolved in 3 ml of methanol, subjected to a chromatograph tower of Sephadex LH-20 (1.8 x 30 cm) and eluted with methanol, and the fractions containing the desired product were concentrated to dryness to give 9.5 mg of the captioned compound represented by the formula (13).

Rf value: 0.53 (produced by Merck Co., Kiesel gel 60F₂₅₄, developing solvent; chloroform : methanol : tetrahydrofuran = 2:1:1)
FAB-MS(m/z): 630 (M)⁺
¹H-NMR (500 MHz, DMSO-d₆), δ(ppm): 12.7 (1H, brs), 11.0 (1H, brs), 10.5 (1H, brs), 10.1 (1H, brs), 8.61 (1H, d, J=7.6Hz), 8.45 (1H, d, J=7.9Hz), 7.21 (2H, t, J=7.6Hz), 7.02∼7.07 (3H, m), 5.42 (1H, d, J=5.8Hz), 5.35 (1H, brs), 5.21 (1H, brs), 4.91 (1H, d, J= 5.5Hz), 4.02 (2H, m), 3.76 (1H, m), 3.61 (2H, m), 3.39 (1H, m)

### Example 14

The compound represented by the formula 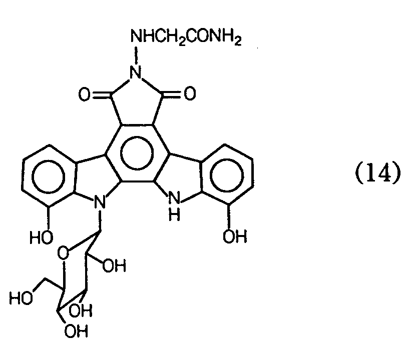
4 ml of methanol and 4 ml of benzene were added to 31.6 mg of the compound obtained in Example 8 to make a solution, 0.15 ml of trimethylsilyldiazomethane (10% hexane solution, Tokyo Kasei Co.) was added, and the mixture was allowed to stand at room for 10 minutes and concentrated to dryness to give 29.3 mg of the methyl ester of the compound obtained in Example 8. This was dissolved in 5 ml of methanol, 0.6 ml of concentrated ammonia water was added, and the mixture was stirred at room temperature for 16 hours. The reaction solution was concentrated to dryness, 3 ml of methanol was added to the residue to make a solution, and the solution was subjected to a chromatograph tower of Sephadex LH-20 (1.8 x 36 cm) and eluted with methanol. The fractions containing the desired product were concentrated to dryness to give 16.9 mg of the captioned compound represented by the formula (14).

Rf value: 0.22 (produced by Merck Co., Kiesel gel 60F₂₅₄, developing solvent; chloroform : methanol : tetrahydrofuran = 2:1:1)
FAB-MS(m/z): 592 (M+H)⁺
¹H-NMR (400 MHz, DMSO-d₆), δ(ppm): 10.9 (1H, s), 10.4 (1H, brs), 10.0 (1H, brs), 8.69 (1H, d, J=7.3Hz), 8.52 (1H, d, J=8.3Hz), 7.77 (1H, brs), 7.39 (1H, brs), 7.19 (2H, t, J=7.8Hz), 6.98∼7.05 (3H, m), 6.25 (1H, t, J =3.9Hz), 5.41 (1H, d, J=5.4Hz), 5.35 (1H, brs), 5.20 (1H, d, J=5.4Hz), 4.87 (1H, d, J= 5.4Hz), 4.02 (2H, m), 3.74 (1H, m), 3.68∼3.70 (4H, m), 3.39 (1H, m)

### Example 15

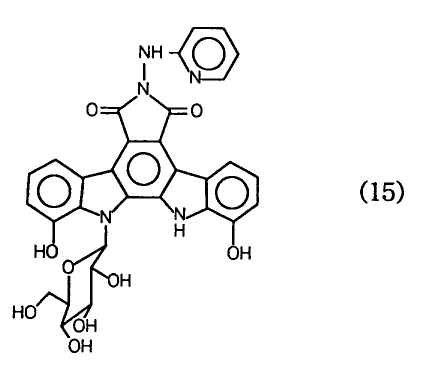

2 ml of methanol was added to 11 mg of the compound obtained in Example A and 10 mg of 2-hydrazinopyridine (Aldrich Chemical Co.) to make a solution, and the solution was refluxed with heating for 1.5 hours. The reaction solution was concentrated to dryness, 30 ml of water and 50 ml of ethyl acetate were added, the water layer was adjusted to pH 5 with 1N hydrochloric acid, extraction operation was carried out, and the resultant ethyl acetate layer was concentrated to dryness. The resultant crude substance was dissolved in 2 ml of methanol, subjected to a chromatograph tower of Sephadex LH-20 (1.8 x 36 cm) and eluted with methanol. The fractions containing the desired product were concentrated to dryness to give 10 mg of the captioned compound represented by the formula (15).

Rf value: 0.46 (produced by Merck Co., Kiesel gel 60F₂₅₄, developing solvent; chloroform : methanol : tetrahydrofuran = 2:1:1)
FAB-MS(m/z): 612 (M+H)⁺
¹H-NMR (400 MHz, DMSO-d₆), δ(ppm): 11.0 (1H, s), 10.4 (1H, s), 10.0 (1H, s), 9.34 (1H, s), 8.65 (1H, d, J=8.3Hz), 8.48 (1H, d, J=7.8Hz), 7.95 (1H, d, J=4.9Hz), 7.62 (1H, t, J=7.8Hz), 7.18 (2H, t, J=7.8Hz), 7.00∼ 7.08 (3H, m), 6.86 (1H, d, J=7.8Hz), 6.78 (1H, dd, J=4.9, 7.8Hz), 5.44 (1H, d, J=5.8Hz), 5.37 (1H, brs), 5.23 (1H, d, J=5.8Hz), 4.92 (1H, brs), 4.02 (2H, m), 3.76 (1H, m), 3.64 (2H, m), 3.41 (1H, m)

### Example 16

The compound represented by the formula 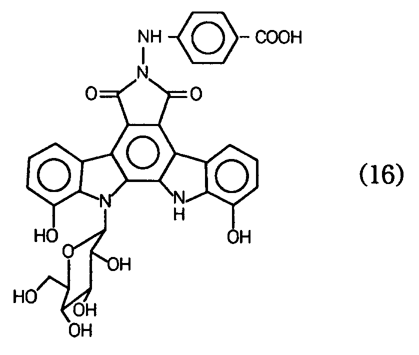
4 ml of methanol was added to 24 mg of the compound obtained in Example A and 4-hydrazinobenzoic acid (Aldrich Chemical Co.), and the mixture was refluxed with heating for 2 hours. The reaction solution was subjected to a chromatograph tower of Sephadex LH-20 (1.8 x 44 cm) and eluted with methanol. The fractions containing the desired product were concentrated to dryness to give 20.9 mg of the captioned compound represented by the formula (16) as red crystal-like powder.

Rf value: 0.31 (produced by Merck Co., Kiesel gel 60F₂₅₄, developing solvent; chloroform : methanol : tetrahydrofuran = 2:1:1)
FAB-MS(m/z): 655 (M+H)⁺
¹H-NMR (500 MHz, DMSO-d₆), δ(ppm): 11.0 (1H, s), 10.5 (1H, brs), 10.1 (1H, brs), 9.11 (1H, s), 8.65 (1H, d, J=7.9Hz), 8.48 (1H, d. J=7.9Hz), 7.80 (2H, d, J=8.3Hz), 7.18 (2H, t, J=7.6Hz), 7.01∼7.08 (3H, m), 6.84 (2H, d, J=8.3Hz), 5.20∼5.60 (3H, brs), 4.96 (1H, brs), 4.03 (2H, m), 3.76 (1H, m), 3.65 (2H, m), 3.41 (1H, m)

### Example 17

The compound represented by the formula 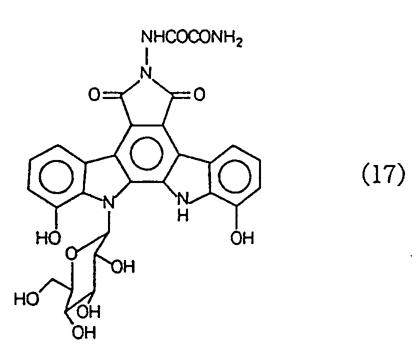
6 ml of 50% methanol was added to 26 mg of the compound obtained in Example A and 38 mg of oxamic hydrazide (Aldrich Chemical Co.), and the mixture was stirred with heating at 80°C for 20 hours. The reaction solution was concentrated to dryness, 15 ml of water and 50 ml of ethyl acetate were added, the mixture was adjusted to pH 2 with 1N hydrochloric acid, and extraction operation was carried out. The ethyl acetate layer was concentrated, and the precipitated crystals were collected by filtration to give 10 mg of the captioned compound represented by the formula (17).

Rf value: 0.38 (produced by Merck Co., Kiesel gel 60F₂₅₄, developing solvent; chloroform : methanol : tetrahydrofuran = 2:1:1)
FAB-MS(m/z): 606 (M+H)⁺
¹H-NMR (500 MHz, DMSO-d₆), δ(ppm): 11.4 (1H, s), 11.0 (1H, s), 10.4 (1H, s), 10.0 (1H, s), 8.63 (1H, d, J=7.9Hz), 8.46 (1H, d, J=7.9Hz), 8.38 (1H, s), 8.11 (1H, s), 7.21 (2H, t, J=7.9Hz), 7.02∼7.07 (3H, m), 5.41 (1H, d, J=5.8Hz), 5.35 (1H, t, J=5.8Hz), 5.19 (1H, d, J=5.2Hz), 4.89 (1H, d, J=5.5Hz), 4.03 (2H, m), 3.76 (1H, m), 3.63 (2H, m), 3.40 (1H, m)

### Example 18

The compound represented by the formula 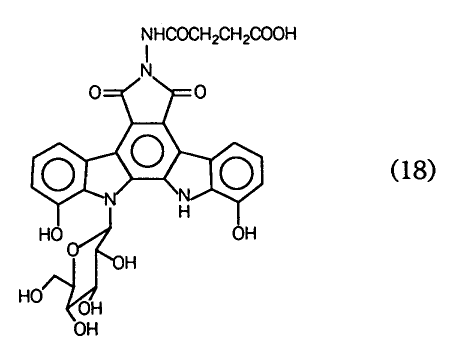
26.7 mg of the compound obtained in Example 1 and 5.5 mg of succinic anhydride were dissolved in 0.5 ml of pyridine, and the solution was stirred at room temperature for 18 hours. This was concentrated to dryness under reduced pressure and the residue was dissolved in a small quantity of N,N-dimethylformamide and subjected to high performance liquid chromatography (HPLC) [Chromatolex ODS, 20 x 250 mm, moving phase : 20% acetonitrile]. The fractions containing the desired product were concentrated to remove acetonitrile, adjusted to pH 2 and extracted with 100 ml of ethyl acetate. The ethyl acetate layer was dehydrated with anhydrous sodium sulfate and concentrated to dryness. The residue was dissolved in methanol and subjected to column chromatography on Sephadex LH-20 (inner diameter 1.5 cm, length 90 cm, eluted with methanol), and the fractions containing the desired product were concentrated to dryness to give 9.7 mg of the captioned compound represented by the formula (18) as orange powder.

HPLC; Rt, 5.3 minutes (column : Chromatolex ODS, inner diameter 4.6 mm, length 250 mm, detection; UV 305 nm, flow rate; 1 ml/minute, moving phase; 27.5% acetonitrile : trifluoroacetic acid = 1000:1)
FAB-MS(m/z): 657 (M+Na)⁺
¹H-NMR (400 MHz, DMSO-d₆), δ(ppm): 11.0 (1H, s), 10.7 (1H, brs), 10.4 (1H, brs), 10.1 (1H, brs), 8.64 (1H, d, J=7.9Hz), 8.47 (1H, d, J=7.9Hz), 7.19 (2H, t, J=7.8Hz), 7.01∼7.07 (3H, m), 5.42 (2H, brs), 5.22 (1H, brs), 4.92 (1H, brs), 4.02 (2H, m), 3.75 (1H, m), 3.63 (2H, m), 3.40 (1H, m), 2.65 (2H, t, J=7.3Hz), 2.52 (2H, t, J=7.3Hz)

### Example 19

The compound represented by the formula 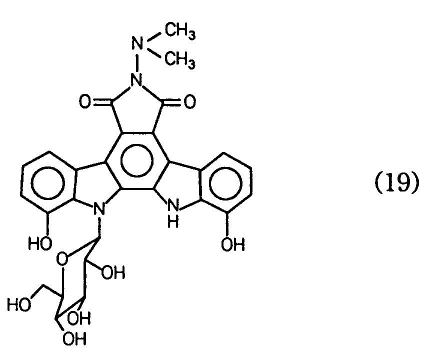
30 mg of the compound obtained in Example 1 was dissolved in 0.5 ml of N,N-dimethylformamide, 0.1 ml of methyl iodide was added, and the mixture was stirred at room temperature for 18 hours. This was mixed with 50 ml of ethyl acetate, the mixture was washed successively with 1% sodium bicarbonate aqueous solution and then water, and the ethyl acetate layer was dehydrated with anhydrous sodium sulfate and concentrated to dryness. The residue was dissolved in methanol and subjected to column chromatography on Sephadex LH-20 (1.5 x 90 cm, eluted with methanol), and the fractions containing the desired product were concentrated to dryness to give 18.0 mg of the captioned compound represented by the formula (19) as orange powder.

Rf value: 0.51 (produced by Merck Co. , Kiesel gel 60F₂₅₄, developing solvent; chloroform : methanol : tetrahydrofuran : acetic acid = 20:10:10:1)
FAB-MS(m/z): 563 (M+H)⁺
¹H-NMR (400 MHz, DMSO-d₆), δ(ppm): 10.9 (1H, s), 10.3 (1H, s), 9.95 (1H, s), 8.70 (1H, d, J=8.3Hz), 8.53 (1H, d, J=8.3Hz), 7.18 (2H, t, J=7.8Hz), 7.00∼7.06 (3H, m), 5.41 (1H, d. J=5.4Hz), 5.34 (1H, t, J=5.4Hz), 5.19 (1H, d, J=5.4Hz), 4.86 (1H, d, J=5.4Hz), 4.02 (2H, m), 3.75 (1H, m), 3.62 (2H, m), 3.39 (1H, m), 3.02 (6H, s)

### Example 20

The compound represented by the formula 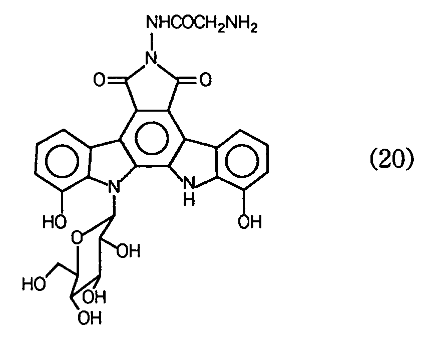
82.1 mg of t-butyloxycarbonyl (Boc)-glycine was dissolved in 1 ml of methylene chloride, the solution was stirred under ice cooling for 15 minutes, 96.7 mg of dicyclohexylcarbodiimide dissolved in 1 ml of methylene chloride was added, and the mixture was stirred under ice cooling for 15 minutes. To this was added 227.6 mg of the compound obtained in Example 1 dissolved in 6 ml of pyridine, and the mixture was stirred at room temperature for 17 hours. The reaction solution was concentrated to dryness, the residue was dissolved in ethyl acetate, the solution was washed successively with saturated saline, acidic water (pH 2) and then water, and the ethyl acetate layer was dehydrated with anhydrous sodium sulfate and concentrated to dryness. The residue was subjected to silica gel column chromatography (1.5 x 55 cm, eluted with toluene : methanol = 6:1), and the fractions containing the desired product were concentrated to dryness to give 105.2 mg of the Boc derivative of the captioned compound represented by the formula (20) as orange powder. This was dissolved in 1.2 ml of trifluoroacetic acid, and the solution was stirred at room temperature for 30 minutes to remove the Boc group. The reaction solution was concentrated to dryness, the residue was dissolved in 15 ml of water, and the solution was adjusted to pH 7.5-8 and extracted with n-butanol. 40 ml of water was added to the n-butanol layer (50 ml), and the mixture was adjusted to pH 2 with dilute hydrochloric acid and concentrated to dryness. The resultant orange powder was dissolved in methanol and subjected to column chromatography on Sephadex LH-20 (1.5 x 38 cm, eluted with methanol), and the fractions containing the desired product were concentrated to dryness to give 63.7 mg of the hydrochloride of the captioned compound represented by the formula (20) as orange powder.

HPLC; Rt, 8.7 minutes (column : Chromatolex ODS, inner diameter 4.6 mm, length 250 mm, detection; UV 305 nm, flow rate; 1 ml/minute, moving phase; 20% acetonitrile : trifluoroacetic acid = 1000:1 → 70% acetonitrile : trifluoroacetic acid = 1000:1, 30 minutes linear gradient)
FAB-MS(m/z): 592 (M+H)⁺
¹H-NMR (hydrochloride, 400 MHz, DMSO-d₆), δ(ppm): 11.3 (1H, brs), 11.0 (1H, brs), 10.5 (1H, s), 10.1 (1H, s), 8.62 (1H, d, J=8.3Hz), 8.46 (1H, d. J=8.3Hz), 8.31 (2H, s), 7.19 (2H, t, J=7.8Hz), 7.03∼7.08 (3H, m), 5.46 (1H, brs), 5.34 (1H, brs), 5.27 (1H, brs), 4.91 (1H, brd. J=4.9Hz), 4.03 (2H, m), 3.98 (2H, s), 3.76 (1H, m), 3.64 (2H, m), 3.40 (1H, m)

### Example 21

The compound represented by the formula 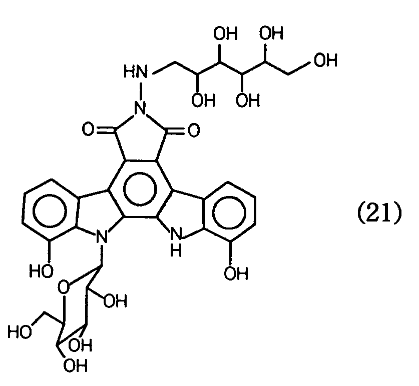
40.0 mg of the compound obtained in Example A was dissolved in 3 ml of N,N-dimethylformamide, 42.2 mg of 1-deoxy-1-hydrazino-D-sorbitol and 0.1 ml of triethylamine was added, and the mixture was refluxed with heating for 16 hours. This was brought back to room temperature, subjected to a chromatograph tower of Sephadex LH-20 (1.8 x 20 cm) and eluted with methanol. The fractions containing the desired product were concentrated to dryness to give 20.0 mg of the captioned compound represented by the formula (21).
FAB-MS(m/z): 699 (M+H)⁺
¹H-NMR (400 MHz, DMSO-d₆), δ(ppm): 10.91 (1H, s), 10.35 (1H, brs), 9.96 (1H, brs), 8.73 (1H, d, J=8.9Hz), 8.54 (1H, d, J=8.9Hz), 7.20 (2H, t, J=8.4Hz), 7.00-7.10 (3H, m), 5.76 (1H, t, J=3.8Hz), 5.42 (1H, d, J=5.5Hz), 5.37 (1H, brs), 5.22 (1H, d, J=5.5Hz), 4.89 (1H, brs), 4.67 (1H, d, J=3.4Hz), 4.45 (1H, d, J=5.1Hz), 4.37 (1H, d, J= 7.0Hz), 4.25-4.43 (2H, m), 4.00 (2H, m), 3.55-3.80 (7H, m), 3.44-3.52 (2H, m), 3.35-3.44 (2H, m), 3.05-3.20 (2H, m)

### Example 22

The compound represented by the formula 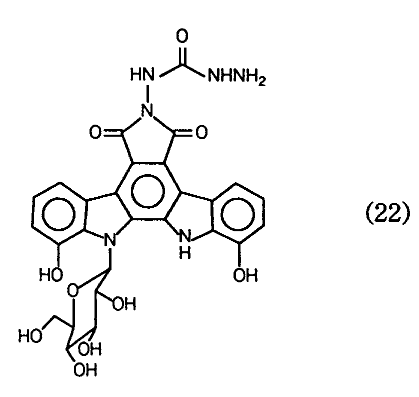
100 mg of the compound obtained in Example A was dissolved in 5 ml of N,N-dimethylformamide, 100 mg of carbohydrazide was added, and the mixture was stirred at 80°C for 3 hours and concentrated to dryness. The residue was dissolved in methanol and the insoluble matters were removed by Celite filtration. The resultant filtrate was concentrated, and the residue was dissolved in a small quantity of methanol, subjected to a chromatograph tower of Sephadex LH-20 (1.5 x 20 cm) and eluted with methanol. The fractions containing the desired product were concentrated to dryness to give 91.2 mg of the captioned compound represented by the formula (22).

Rf value: 0.1 (produced by Merck Co., Kiesel gel 60F₂₅₄, developing solvent; chloroform-methanol-tetrahydrofuran = 2:1:1:)
FAB-MS(m/z): 593 (M+H)⁺
¹H-NMR (400 MHz, DMSO-d₆), δ(ppm): 10.96 (1H, s), 10.40 (1H, s), 10.01 (1H, s), 8.95 (1H, s), 8.65 (1H, d, J=8.2Hz), 8.50 (1H, d, J=8.2Hz), 7.90 (1H, s), 7.17 (2H, t, J=6.9Hz), 7.00-7.10 (3H, m), 5.43 (1H, d, J=4.1Hz), 5.38 (1H, brs), 5.20 (1H, s), 4.90 (1H, s), 4.39 (2H, brs), 4.04 (2H, m), 3.75 (1H, m), 3.55-3.70 (2H, m), 3.38 (1H, m)

### Example 23

The compound represented by the formula 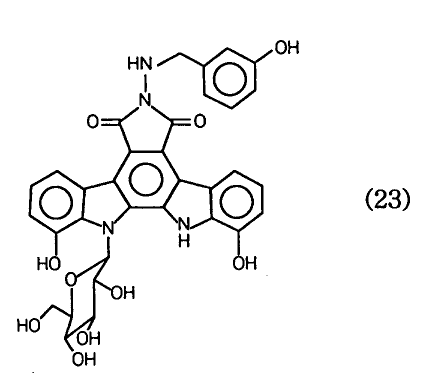
15.0 mg of the compound obtained in Example A was dissolved in 1 ml of N,N-dimethylformamide, 32 mg of 3-hydoxybenzylhydrazine dihydrochloride and 0.1 ml of 10% sodium bicarbonate aqueous solution were added, and the mixture was stirred at 80°C for 4 hours. This was mixed with 50 ml of ethyl acetate, the mixture was washed successively with 0.2N hydrochloric acid and then saturated saline, and the ethyl acetate layer was dehydrated with anhydrous sodium sulfate and concentrated to dryness. The residue was dissolved in a small quantity of methanol, subjected to a chromatograph tower of Sephadex LH-20 (1.8 x 15 cm) and eluted with methanol. The fractions containing the desired product were concentrated to dryness to give 15.3 mg of the captioned compound represented by the formula (23).

Rf value: 0.22 (produced by Merck Co., Kiesel gel 60F₂₅₄, developing solvent; chloroform-methanol-tetrahydrofuran = 5:1:1)
FAB-MS(m/z): 641 (M+H)⁺
¹H-NMR (200 MHz, DMSO-d₆), δ(ppm): 10.90 (1H, s), 10.38 (1H, s), 9.99 (1H, s), 9.30 (1H, s), 8.70 (1H, d, J=8.1Hz), 8.53 (1H, d, J=8.5Hz), 6.86-7.22 (8H, m), 6.61 (1H, dd, J=2.2, 8.4Hz), 6.03 (1H, t, J=5.1Hz), 5.43 (1H, d, J=5.4Hz), 5.35 (1H, t, J=5.0Hz), 5.22 (1H, d, J=5.4Hz), 4.89 (1H, d, J=5.4Hz), 4.19 (2H, d, J=5.1Hz), 4.00 (2H, m), 3.72 (1H, m), 3.53-3.70 (2H, m), 3.38 (1H, m)

### Example 24

The compound represented by the formula 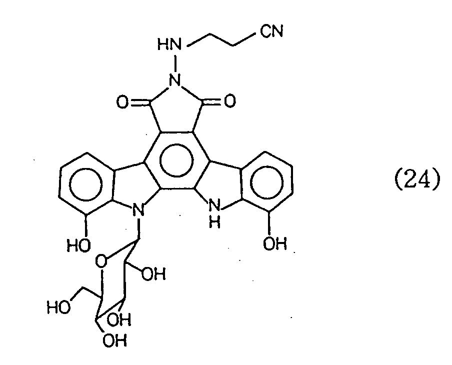
64.6 mg of the compound obtained in Example A was dissolved in 2 ml of N,N-dimethylformamide, 30 mg of 2-cyanoethylhydrazine were added, and the mixture was stirred at 90°C for 1.5 hours. 50 ml of 0.2N hydrochloric acid was added to the reaction solution, and the mixture was extracted with ethyl acetate (50 ml x 2). The ethyl acetate layer was concentrated to dryness, and the residue was dissolved in a small quantity of methanol, subjected to a chromatograph tower of Sephadex LH-20 (1.8 x 30 cm) and eluted with methanol. The fractions containing the desired product were concentrated to dryness to give 45.0 mg of the captioned compound represented by the formula (24).

Rf value: 0.39 (produced by Merck Co., Kiesel gel 60F₂₅₄, developing solvent; chloroform : methanol = 3:1)
FAB-MS(m/z): 588 (M+H)⁺
¹H-NMR (200 MHz, DMSO-d₆), δ(ppm): 10.91 (1H, s), 10.36 (1H, s), 9.98 (1H, s), 8.70 (1H, d, J=8.4Hz), 8.53 (1H, d, J=8.4Hz), 7.18 (2H, t, J=8.4Hz), 6.95-7.10 (3H, m), 6.15 (1H, t, J=4.2Hz), 5.42 (1H, d, J=5.7Hz), 5.34 (1H, brs), 5.23 (1H, d, J=4.4Hz), 4.91 (1H, d, J=5.3Hz), 4.00 (2H, m), 3.72 (1H, m), 3.55-3.70 (2H, m), 3.39 (1H, m), 3.30 (2H, td, J=4.2, 6.2Hz), 2.69 (2H, t, J=6.2Hz)

### Example 25

The compound represented by the formula 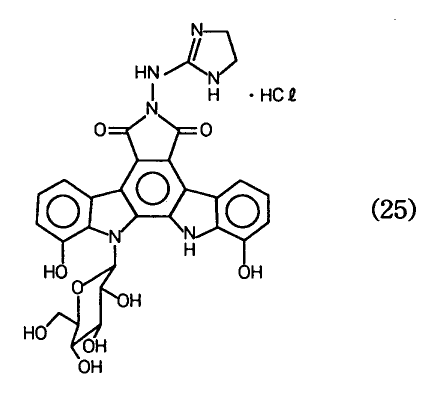
1.09 g of the compound obtained in Example A was dissolved in 35 ml of N,N-dimethylformamide ― 2 ml of water, 455 mg of 2-hydrazino-2-imidazoline hydrobromide and 211 mg of sodium bicarbonate were added, and the mixture was stirred at 80°C for 2 hours and concentrated to dryness. The residue was dissolved in 300 ml of 0.2N hydrochloric acid and extracted with n-butanol (1L x 2). The butanol layer was concentrated to dryness, and the residue was dissolved in a small quantity of methanol, subjected to a chromatograph tower of Sephadex LH-20 (3.0 x 80 cm) and eluted with methanol. The fractions containing the desired product were concentrated to dryness to give 650 mg of the captioned compound represented by the formula (25).

Rf value: 0.55 (produced by Merck Co., Kiesel gel 60F₂₅₄, developing solvent; n-butanol : acetic acid : water = 4:1:1)
FAB-MS(m/z): 603 (M+H)⁺
¹H-NMR (400 MHz, DMSO-d₆), δ(ppm): 11.2 (1H, s), 10.90 (1H, brs), 10.50 (1H, s), 10.14 (1H, s), 9.42 (1H, brs), 8.92 (1H, brs), 8.62 (1H, d, J=10.6Hz), 8.45 (1H, d, J=9.5Hz), 7.22 (2H, t, J=6.5Hz), 7.02-7.10 (3H, m), 5.48 (1H, d, J=4.7Hz), 5.32 (2H, brm), 4.94 (1H, d, J=3.5Hz), 4.04 (2H, m), 3.70-3.90 (5H, m), 3.54-3.70 (2H, m), 3.41 (1H, m)

### Example 26

The compound represented by the formula 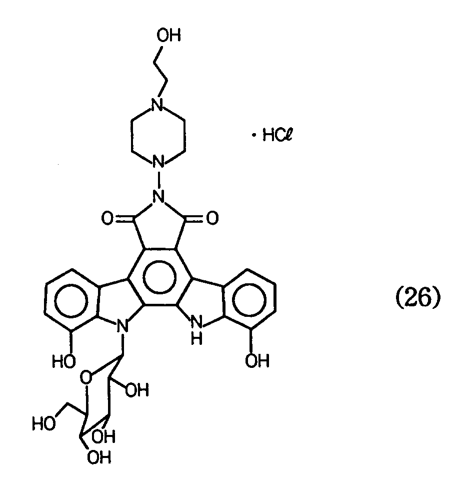
48.3 mg of the compound obtained in Example A was dissolved in 1 ml of N,N-dimethylformamide, 14.3 mg of 1-amino-4-(2-hydroxyethyl) piperazine and 0.1 ml of saturated sodium bicarbonate aqueous solution were added, and the mixture was stirred at 80°C for 2 hours. This was distributed between 50 ml of ethyl acetate and 50 ml of water, 5 ml of 0.2 N hydrochloric acid was added to the water layer, and the mixture was extracted with n-butanol (100 ml x 2). The butanol layer was concentrated to dryness, and the residue was dissolved in a small quantity of methanol, subjected to a chromatograph tower of Sephadex LH-20 (1.8 x 30 cm) and eluted with methanol. The fractions containing the desired product were concentrated to dryness to give 22 mg of the captioned compound represented by the formula (26).

Rf value: 0.53 (produced by Merck Co., Kiesel gel 60F₂₅₄, developing solvent; n-butanol : acetic acid : water = 4:1:1)
FAB-MS(m/z): 648 (M+H)⁺
¹H-NMR (400 MHz, DMSO-d₆), δ(ppm): 10.92 (1H, s), 10.50 (2H, brs), 10.10 (1H, s), 8.66 (1H, d, J=7.2Hz), 8.50 (1H, d, J=8.9Hz), 7.18 (2H, t, J=8.9Hz), 7.02-7.12 (3H, m), 5.46 (1H, d, J=5.6Hz), 5.25-5.40 (3H, brm), 4.86 (1H, d, J=5.6Hz), 3.95-4.20 (4H, m), 3.70-3.90 (4H, m), 3.55-3.70 (4H, m), 3.20-3.50 (6H, m)

### Example 27

The compound represented by the formula 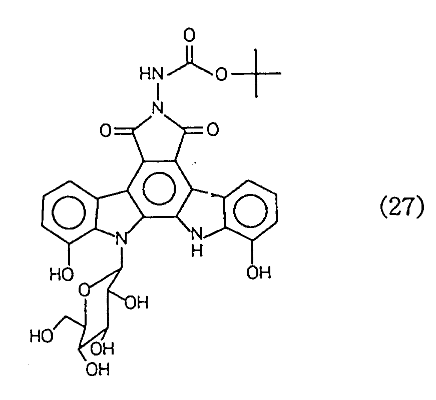
24 mg of the compound obtained in Example A was dissolved in 0.6 ml of N,N-dimethylformamide, 10 mg of t-butyl carbazinate was added, and the mixture was stirred at 80°C for 6 hours. This was mixed with 50 ml of ethyl acetate, the mixture was washed successively with water (30 ml x 2) and saturated saline, and the ethyl acetate layer was dehydrated with anhydrous sodium sulfate and concentrated to dryness. The residue was dissolved in 1 ml of methanol, subjected to a chromatograph tower of Sephadex LH-20 (1.6 x 20 cm) and eluted with methanol. The fractions containing the desired product were concentrated to dryness to give 27.2 mg of the captioned compound represented by the formula (27).

Rf value: 0.42 (produced by Merck Co., Kiesel gel 60F₂₅₄, developing solvent; chloroform : methanol = 4:1)
FAB-MS(m/z): 634 (M+H)⁺
¹H-NMR (400 MHz, DMSO-d₆), δ(ppm): 10.99 (1H, s), 10.42 (1H, s), 10.02 (1H, s), 9.82 (1H, brs), 8.65 (1H, d, J=7.7Hz), 8.49 (1H, d, J=7.7Hz), 7.18 (2H, t, J=7.7Hz), 7.00-7.10 (3H, m), 5.42 (1H, brs), 5.35 (1H, brs), 5.21 (1H, brs), 4.90 (1H, brs), 4.02 (2H, m), 3.72 (1H, m), 3.56-3.70 (2H, m), 3.40 (1H, m), 1.50 (9H, s)

### Example 28

The compound represented by the formula 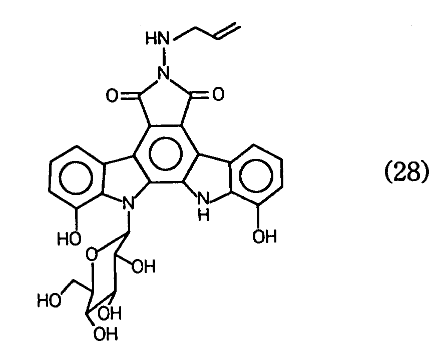
and the compound represented by the formula 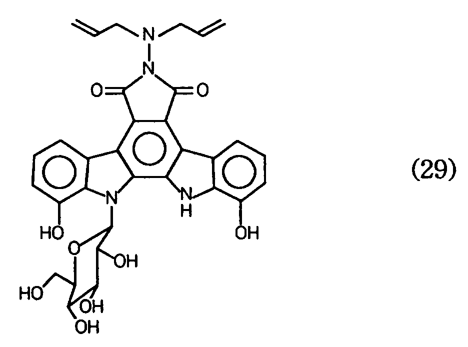
177 mg of the compound obtained in Example 1 was dissolved in 6 ml of N,N-dimethylformamide, 0.68 ml of allyl bromide was added, and the mixture was stirred at room temperature for 1 day. 200 ml of water was added to the mixture, the mixture was extracted with ethyl acetate (200 ml x 3), and the ethyl acetate layer was dehydrated with saturated saline and concentrated to dryness. The residue was dissolved in 3 ml of methanol, subjected to a chromatograph tower of Sephadex LH-20 (2.5 x 40 cm) and eluted with methanol. The fractions containing the desired products respectively were concentrated to dryness to give 42.1 mg of the captioned compound represented by the formula (28) and 67.5 mg of the compound represented by the formula (29).

### The compound represented by the formula (28)

Rf value: 0.68 (produced by Merck Co., Kiesel gel 60F₂₅₄, developing solvent; chloroform : methanol = 2:1)
FAB-MS(m/z): 575 (M+H)⁺
¹H-NMR (400 MHz, DMSO-d₆), δ(ppm): 10.90 (1H, s), 10.38 (1H, s), 9.98 (1H, s), 8.70 (1H, d, J=9.0Hz), 8.52 (1H, d, J=10.2Hz), 7.20 (2H, t, J=7.7Hz), 6.95-7.08 (3H, m), 5.92 (2H, m), 5.40 (1H, d, J=6.4Hz), 5.32 (1H, m), 5.20 (2H, m), 5.05 (1H, d, J=11.5Hz), 4.88 (1H, d, J=5.8Hz), 4.00 (2H, m), 3.67-3.78 (3H, m), 3.58-3.65 (2H, m), 3.35 (1H, m)

### The compound represented by the formula (29)

Rf value: 0.75 (produced by Merck Co., Kiesel gel 60F₂₅₄, developing solvent; chloroform : methanol = 2:1)
¹H-NMR (400 MHz, DMSO-d₆), δ(ppm): 10.91 (1H, s), 10.40 (1H, brs), 10.00 (1H, brs), 8.66 (1H, d, J=9.4Hz), 8.50 (1H, d, J=9.4Hz), 7.18 (2H, t. J=8.0Hz), 7.00-7.10 (3H, m), 5.90 (2H, ddt, J=6.3, 10.2, 17.0Hz), 5.42 (1H, d, J=5.3Hz), 5.33 (1H, brs), 5.23 (2H, d, J=17.0Hz), 5.22 (1H, brs), 5.04 (2H, d, J=10.2Hz), 4.91 (1H, brs), 4.02 (2H, m), 3.97 (4H, d, J=6.3Hz), 3.70 (1H, m), 3.51-3.66 (2H, m), 3.35 (1H, m)

### Example 29

The compound represented by the formula 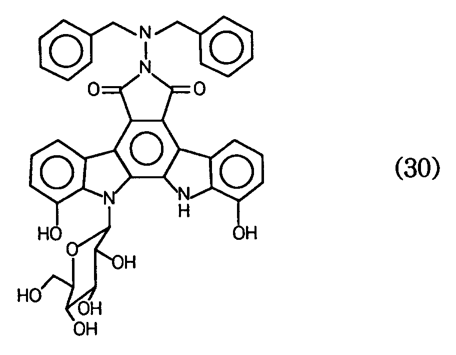
and the compound represented by the formula 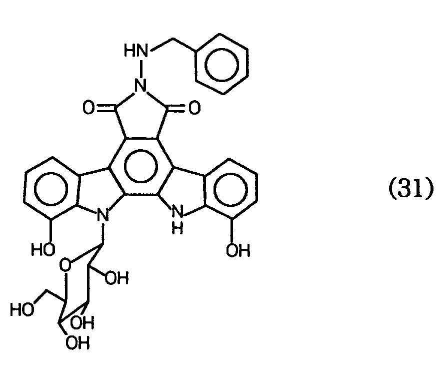
20 mg of the compound obtained in Example 1 was dissolved in 1 ml of N,N-dimethylformamide, 0.3 ml of benzyl bromide was added, and the mixture was stirred overnight. This was mixed with 40 ml of ethyl acetate, the mixture was washed successively with water (30 ml x 2) and and then saturated saline, and the ethyl acetate layer was dehydrated with anhydrous sodium sulfate and concentrated to dryness. The residue was dissolved in 1 ml of methanol, subjected to a chromatograph tower of Sephadex LH-20 (1.6 x 30 cm) and eluted with methanol. The fractions containing the desired products respectively were concentrated to dryness to give 13.2 mg of the captioned compound represented by the formula (30) and 7.2 mg of the compound represented by the formula (31).

### The compound represented by the formula (30)

Rf value: 0.44 (produced by Merck Co., Kiesel gel 60F₂₅₄, developing solvent; chloroform : methanol = 3:1)
FAB-MS(m/z): 715 (M+H)⁺
¹H-NMR (200 MHz, DMSO-d₆), δ(ppm): 10.85 (1H, s), 10.35 (1H, s), 9.96 (1H, s), 8.65 (1H, d, J=8.5Hz), 8.45 (1H, d, J=9.0Hz), 7.50-7.65 (4H, m), 7.10-7.40 (8H, m), 6.95-7.10 (3H, m), 5.40 (1H, d, J=5.4Hz), 5.30 (1H, brs), 5.18 (1H, d, J=4.9Hz), 4.83 (1H, d, J=4.9Hz), 4.58 (2H, s), 4.55 (2H, s), 4.00 (2H, m), 3.46-3.80 (3H, m), 3.36 (1H, m)

### The compound represented by the formula (31)

Rf value: 0.38 (produced by Merck Co., Kiesel gel 60F₂₅₄, developing solvent; chloroform : methanol = 3:1)
FAB-MS(m/z): 625 (M+H)⁺
¹H-NMR (300 MHz, DMSO-d₆), δ(ppm): 10.88 (1H, s), 10.40 (1H, brs), 10.00 (1H, brs), 8.67 (1H, d, J=7.9Hz), 8.51 (1H, d, J=7.3Hz), 7.50 (2H, d, J=6.9Hz), 7.30 (2H, t, J= 6.9Hz), 7.21 (1H, t, J=6.9Hz), 7.16 (2H, t, J=7.3Hz), 6.96-7.07 (3H, m), 6.13 (1H, t, J =5.3Hz), 5.42 (1H, d, J=5.9Hz), 5.21 (1H, d, J=5.3Hz), 4.91 (1H, brs), 4.55 (1H, brs), 4.28 (2H, d, J=5.3Hz), 4.02 (2H, m), 3.72 (1H, m), 3.55-3.70 (2H, m), 3.40 (1H, m)

### Example 30

The compound represented by the formula 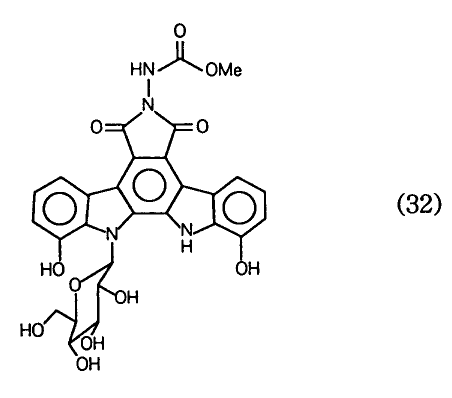
1.4 g of the compound obtained in Example A was dissolved in 30 ml of N,N-dimethylformamide, 1 g of methyl carbazinate was added, and the mixture was stirred at 80°C for 2 hours. 400 ml of water was added to the mixture, and the mixture was extracted with ethyl acetate (500 ml x 3). The resultant ethyl acetate layer was concentrated to dryness. The residue was dissolved in 5 ml of methanol, subjected to a chromatograph tower of Sephadex LH-20 (3.0 x 80 cm) and eluted with methanol. The fractions containing the desired product were concentrated to dryness to give 1.3 g of the captioned compound represented by the formula (32).

Rf value: 0.18 (produced by Merck Co., Kiesel gel 60F₂₅₄, developing solvent; chloroform : methanol = 4:1)
FAB-MS(m/z): 592 (M+H)⁺
¹H-NMR (300 MHz, DMSO-d₆), δ(ppm): 11.00 (1H, s), 10.42 (1H, brs), 10.18 (1H, s), 10.04 (1H, brs), 8.64 (1H, d, J=7.6Hz), 8.47 (1H, d, J=8.3Hz), 7.20 (2H, t. J=8.3Hz), 7.00-7.10 (3H, m), 5.42 (1H, brs), 5.35 (1H, brs), 5.21 (1H, brs), 4.91 (1H, brs), 4.02 (2H, m), 3.75 (3H, s), 3.50-3.70 (3H, m), 3.40 (1H, m)

### Example 31

The compound represented by the formula 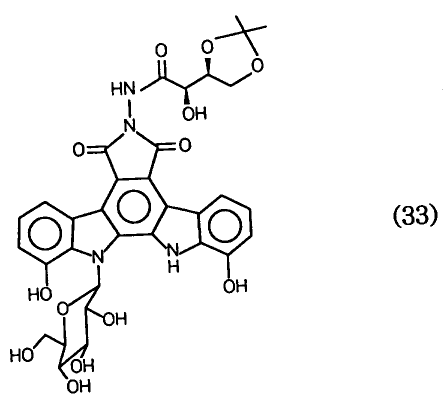
90 mg of the compound obtained in Example A was dissolved in 1 ml of N,N-dimethylformamide, 67 mg of (2R,3S)-3,4-0-isopropylidene-2,3,4-trihydroxybutane carbohydrazide, and the mixture was stirred at 80°C for 7 hours and then at room temperature for 3 days. 50 ml of water was added to the mixture, and the mixture was extracted with ethyl acetate (50 ml x 2). The resultant ethyl acetate layer was concentrated to dryness. The residue was dissolved in 3 ml of methanol, subjected to a chromatograph tower of Sephadex LH-20 (1.8 x 25 cm) and eluted with methanol. The fractions containing the desired product were concentrated to dryness to give 112 mg of the captioned compound represented by the formula (33).

Rf value: 0.14 (produced by Merck Co., Kiesel gel 60F₂₅₄, developing solvent; chloroform : methanol = 4:1)
FAB-MS(m/z): 692 (M+H)⁺
¹H-NMR (300 MHz, DMSO-d₆), δ(ppm): 11.01 (1H, s), 10.70 (1H, s), 10.45 (1H, s), 10.05 (1H, s), 8.75 (1H, d, J=7.4Hz), 8.47 (1H, d, J=7.4Hz), 7.21 (2H, t, J=7.4Hz), 7.00-7.10 (3H, m), 6.26 (1H, d, J=6.7Hz), 5.44 (1H, d, J=5.9Hz), 5.39 (1H, brs), 5.24 (1H, d, J= 5.9Hz), 4.93 (1H, d, J=5.9Hz), 4.31 (1H, dd, J=6.7, 11.9Hz), 4.22 (1H, t, J=6.7Hz), 4.10 (1H, ddd, J=6.7, 6.7, 11.9Hz), 4.05 (2H, m), 3.91 (1H, t, J=6.7Hz), 3.76 (1H, m), 3.57-3.71 (2H, m), 3.40 (1H, m), 1.45 (3H, s), 1.36 (3H, s)

### Example 32

The compound represented by the formula 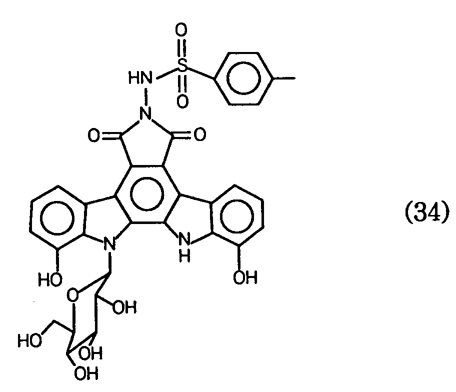
25 mg of the compound obtained in Example 1 was dissolved in 5 ml of anhydrous tetrahydrofuran, 10 mg of p-toluenesulfonic anhydride was added, and the mixture was stirred at room temperature for 1 day. The reaction solution was concentrated to dryness, and the residue was dissolved in 1 ml of methanol, subjected to a chromatograph tower of Sephadex LH-20 (1.8 x 20 cm) and eluted with methanol. The fractions containing the desired product were concentrated to dryness to give 12.3 mg of the captioned compound represented by the formula (34).

Rf value: 0.49 (produced by Merck Co., Kiesel gel 60F₂₅₄, developing solvent; chloroform : methanol : tetrahydrofuran = 3:1:1)
FAB-MS(m/z): 688 (M+H)⁺
¹H-NMR (300 MHz, DMSO-d₆), δ(ppm): 10.98 (1H, s), 10.87 (1H, s), 10.42 (1H, s), 10.05 (1H, s), 8.54 (1H, d, J=7.9Hz), 8.38 (1H, d, J=7.9Hz), 7.84 (2H, d, J=8.7Hz), 7.44 (2H, d, J=8.7Hz), 7.19 (2H, t, J=7.9Hz), 7.00-7.08 (3H, m), 5.43 (1H, d, J=4.7Hz), 5.35 (1H, brs), 5.23 (1H, d, J=4.9Hz), 4.90 (1H, d, J=4.4Hz), 4.04 (2H, m), 3.75 (1H, m), 3.55-3.70 (2H, m), 3.40 (1H, m), 2.42 (3H, s)

### Example 33

The compound represented by the formula 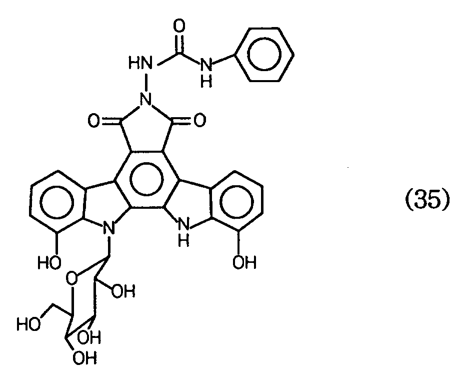
20 mg of the compound obtained in Example 1 was dissolved in 2 ml of tetrahydrofuran, 0.1 ml of phenyl isocyanate was added, and the mixture was stirred at room temperature for 2 hours. The reaction solution was concentrated to dryness, and the residue was dissolved in 1 ml of methanol, subjected to a chromatograph tower of Sephadex LH-20 (1.6 x 30 cm) and eluted with methanol. The fractions containing the desired product were concentrated to dryness to give 12 mg of the captioned compound represented by the formula (35).

Rf value: 0.38 (produced by Merck Co., Kiesel gel 60F₂₅₄, developing solvent; chloroform : methanol : tetrahydrofuran = 2:1:1)
FAB-MS(m/z): 653 (M+H)⁺
¹H-NMR (400 MHz, DMSO-d₆), δ(ppm): 11.00 (1H, s), 10.40 (1H, brs), 10.10 (1H, brs), 9.48 (1H, s), 9.50 (1H, s), 8.67 (1H, d, J=8.3Hz), 8.50 (1H, d, J=8.3Hz), 7.48 (2H, d, J= 7.8Hz), 7.27 (2H, t, J=7.8Hz), 7.20 (2H, t, J=7.8Hz), 6.95-7.10 (4H, m), 5.43 (1H, d, J =4.2Hz), 5.30 (1H, brs), 5.23 (1H, brs), 4.95 (1H, brs), 4.03 (2H, m), 3.75 (1H, m), 3.58-3.70 (2H, m), 3.38 (1H, m)

### Example 34

The compound represented by the formula 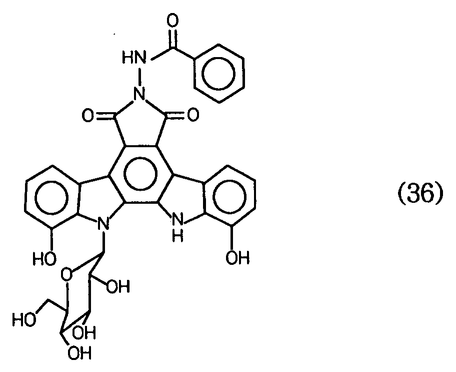
15 mg of the compound obtained in Example 1 was dissolved in 2 ml of tetrahydrofuran, 16 µl of benzoyl chloride was added, and the mixture was stirred at room temperature for 2 hours. The solvent was distilled away, and the residue was dissolved in 1 ml of methanol, subjected to a chromatograph tower of Sephadex LH-20 (1.6 x 20 cm) and eluted with methanol. The fractions containing the desired product were concentrated to dryness to give 12 mg of the captioned compound represented by the formula (36).

Rf value: 0.57 (produced by Merck Co., Kiesel gel 60F₂₅₄, developing solvent; chloroform : methanol : tetrahydrofuran = 2:1:1)
FAB-MS(m/z): 639 (M+H)⁺
¹H-NMR (200 MHz, DMSO-d₆), δ(ppm): 11.35 (1H, brs), 11.04 (1H, s), 10.45 (1H, brs), 10.08 (1H, brs), 8.66 (1H, d, J=8Hz), 8.49 (1H, d, J=8.5Hz), 8.04 (2H, d, J=7.1Hz), 7.55-7.78 (3H, m), 7.20 (2H, t, J=8.5Hz), 7.00-7.15 (3H, m), 5.45 (2H, brs), 5.25 (1H, brs), 4.97 (1H, brs), 4.02 (2H, m), 3.55-3.82 (3H, m), 3.41 (1H, m)

### Example 35

The compound represented by the formula 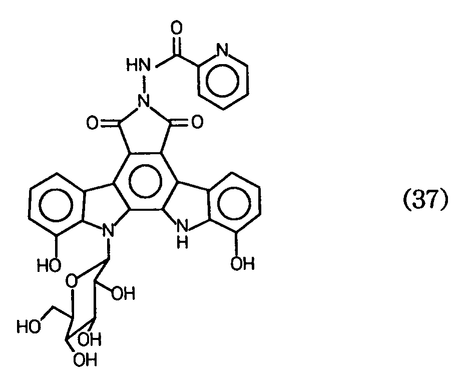
25 mg of the compound obtained in Example A was dissolved in 1.5 ml of N,N-dimethylformamide, 30 mg of α-picolinohydrazide was added, and the mixture was stirred at 80°C for 2 hours. This was mixed with 50 ml of ethyl acetate, and the mixture was washed successively with water and then saturated saline, dehydrated with anhydrous sodium sulfate, and concentrated to dryness. The residue was dissolved in 1 ml of methanol, subjected to a chromatograph tower of Sephadex LH-20 (1.8 x 15 cm) and eluted with methanol. The fractions containing the desired product were concentrated to dryness to give 30 mg of the captioned compound represented by the formula (37).

Rf value: 0.58 (produced by Merck Co., Kiesel gel 60F₂₅₄, developing solvent; chloroform : methanol : tetrahydrofuran = 2:1:1)
FAB-MS(m/z): 640 (M+H)⁺
¹H-NMR (300 MHz, DMSO-d₆), δ(ppm): 11.43 (1H, s), 11.02 (1H, s), 10.45 (1H, s), 10.07 (1H, s), 8.82 (1H, d, J=4.2Hz), 8.75 (1H, d, J=7.3Hz), 8.48 (1H, d, J=7.8Hz), 8.12 (2H, m), 7.75 (1H, m), 7.20 (2H, t, J=7.0Hz), 7.00-7.15 (3H, m), 5.45 (1H, d, J=6.3Hz), 5.40 (1H, brs), 5.25 (1H, d, J=6.3Hz), 4.96 (1H, brs), 4.04 (2H, m), 3.76 (1H, m), 3.55-3.72 (2H, m), 3.42 (1H, m)

### Example 36

The compound represented by the formula 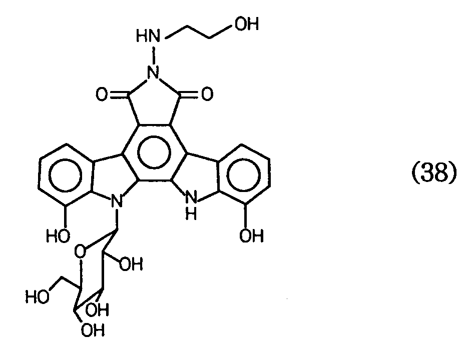
30 mg of the compound obtained in Example A was dissolved in 1 ml of N,N-dimethylformamide, 30 mg of 2-hydrazinoethanol was added, and the mixture was stirred at 80°C for 2 hours. This was concentrated to dryness. The residue was dissolved in 1 ml of methanol, subjected to a chromatograph tower of Sephadex LH-20 (1.8 x 20 cm) and eluted with methanol. The fractions containing the desired product were concentrated to dryness to give 32 mg of the captioned compound represented by the formula (38).

Rf value: 0.32 (produced by Merck Co., Kiesel gel 60F₂₅₄, developing solvent; chloroform : methanol = 2:1)
¹H-NMR (300 MHz, DMSO-d₆), δ(ppm): 10.91 (1H, s), 10.35 (1H, brs), 9.98 (1H, brs), 8.70 (1H, d, J=6.7Hz), 8.53 (1H, d, J=6.9Hz), 7.18 (2H, t, J=7.6Hz), 6.99-7.06 (3H, m), 5.76 (1H, t, J=5.2Hz), 5.41 (1H, d, J=5.6Hz), 5.32 (1H, brs), 5.20 (1H, d, J=5.2Hz), 4.90 (1H, brs), 4.51 (1H, t, J=4.9Hz), 3.96-4.06 (2H, m), 3.73 (1H, m), 3.55 -3.70 (4H, m), 3.39 (1H, m), 3.12 (2H, m)

### Example 37

The compound represented by the formula 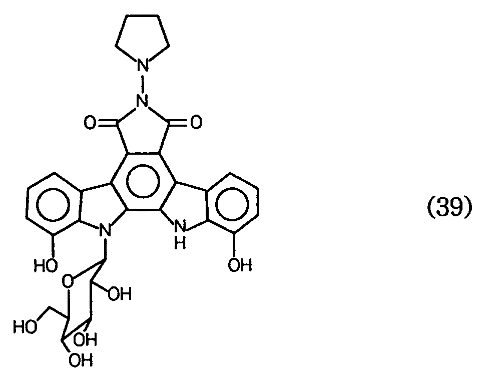
40 mg of the compound obtained in Example A was dissolved in 2 ml of N,N-dimethylformamide, 10 mg of 1-aminopyrrolidine hydrochloride and 0.1 ml of sodium bicarbonate aqueous solution were added, and the mixture was stirred at 80°C for 2 hours. 40 ml of water was added thereto and the mixture was extracted with ethyl acetate (40 ml x 2). The resultant ethyl acetate layer was dehydrated with anhydrous sodium sulfate, and concentrated to dryness. The residue was dissolved in 1 ml of methanol, subjected to a chromatograph tower of Sephadex LH-20 (1.8 x 20 cm) and eluted with methanol. The fractions containing the desired product were concentrated to dryness to give 10.0 mg of the captioned compound represented by the formula (39).

Rf value: 0.33 (produced by Merck Co., Kiesel gel 60F₂₅₄, developing solvent; chloroform : methanol = 4:1)
FAB-MS(m/z): 589 (M+H)⁺
¹H-NMR (300 MHz, DMSO-d₆), δ(ppm): 10.91 (1H, s), 10.35 (1H, s), 9.95 (1H, s), 8.78 (1H, d, J=8.3Hz), 8.52 (1H, d, J=8.3Hz), 7.16 (2H, t, J=7.6Hz), 6.98-7.06 (3H, m), 5.40 (1H, d, J=5.5Hz), 5.33 (1H, t, J=5.7Hz), 5.18 (1H, d, J=5.5Hz), 4.85 (1H, d, J=4.8Hz), 4.02 (2H, m), 3.74 (1H, m), 3.53-3.68 (2H, m), 3.30-3.42 (5H, m), 1.97 (4H, m)

### Example 38

The compound represented by the formula 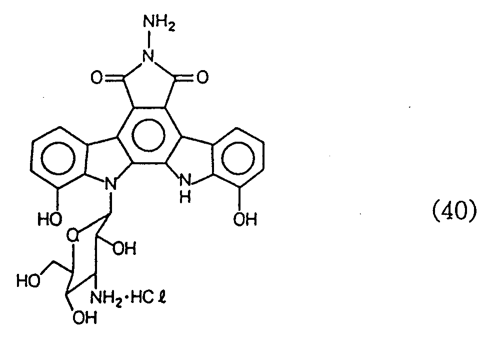
90 mg of 6-benzyloxymethyl-1,11-dibenzyloxy-12,13-dihydro-5H-indolo[2,3-a]pyrrolo[3,4-c]carbazole-4,7(6H)-dione, a compound disclosed in PCT/WO91/18003, 1.3 g of silver oxide and 550 mg of 4Å molecular sieve were suspended in 30 ml of anhydrous benzene. After reflux with heating for 20 minutes, a solution of 416.4 mg of α-bromo-3-deoxy-3-azido-2,4,6-triacetyl-D-glucose in 5 ml of anhydrous benzene was added dropwise over a period of 10 minutes. After further reflux with heating for 2 days, the insoluble matters were filtered using Celite. The filtrate was concentrated to dryness, and the residue was dissolved in 150 ml of ethyl acetate, washed successively with 0.2 N hydrochloric acid, water and then saturated saline, dehydrated with anhydrous sodium sulfate, and concentrated to dryness. The residue was dissolved in 5 ml of chloroform, subjected to a chromatograph tower of Sephadex LH-20 (3.0 x 80 cm) and eluted with chloroform. The fractions containing the desired product were concentrated to dryness, and the residue was purified by preparative thin layer chromatography [n-hexane : acetone : tetrahydrofuran = 3:1:0.1 (Rf: 0.5), then toluene : acetone = 10:1 (Rf: 0.5)] to give 9.2 mg of 6-benzyloxymethyl-1,11-dibenzyloxy-12,13-dihydro-13-(β-D-glucopyranosyl)-5H-indolo[2,3-a]pyrrolo-[3,4-c]carbazole-5,7(6H)-dione.

9.2 mg of the resultant compound was dissolved in 1 ml of hydrazine monohydrate, and the solution was stirred at room temperature for 4 hours. This was mixed with 30 ml of ethyl acetate, and the mixture was washed successively with 0.2 N hydrochloric acid, water and then saturated saline, dehydrated with anhydrous sodium sulfate, and concentrated to dryness. The residue was dissolved in 0.5 ml of tetrahydrofuran - 1 ml of methanol, palladium black was added, and the mixture was stirred, under a hydrogen stream, at room temperature for 3 hours. The insoluble matters were filtered using Celite, 1.5 ml of 10% hydrogen chloride-methanol was added to the filtrate, and the mixture was concentrated to dryness. The residue was dissolved in 0.5 ml of methanol, subjected to a chromatograph tower of Sephadex LH-20 (1.0 x 15 cm) and eluted with methanol. The fractions containing the desired product were concentrated to dryness to give 2.0 mg of the captioned compound represented by the formula (40).

Rf value: 0.5 (produced by Merck Co., Kiesel gel 60F₂₅₄, developing solvent; n-butanol : acetic acid : water = 4:1:1)
FAB-MS(m/z): 534 (M+H)⁺
¹H-NMR (400 MHz, DMSO-d₆), δ(ppm): 10.80 (1H, s), 10.48 (1H, s), 10.20 (1H, s), 8.79 (1H, d, J=7.9Hz), 8.52 (3H, br), 8.50 (1H, d, J=9.2Hz), 7.61 (1H, d, J=6.6Hz), 7.16 (1H, dd, J=9.2, 9.2Hz), 7.10 (1H, dd, J=9.2, 9.2Hz), 7.05 (1H, dd, J=9.2, 9.2Hz), 7.00 (1H, dd, J=9.2, 9.2Hz), 6.42 (1H, d, J=5.2Hz), 6.16 (1H, d, J=3.9Hz), 5.18 (1H, br), 4.93 (1H, br), 4.40 (1H, m), 4.16 (1H, m), 4.03 (1H, m), 3.78 (1H, m), 3.68 (1H, m), 3.42 (1H, m)

### Example 39

The compound represented by the formula 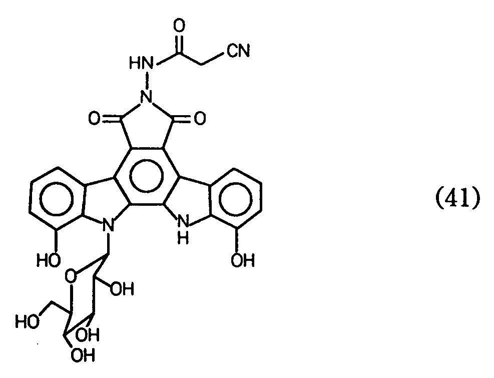
30 mg of the compound obtained in Example A was dissolved in 1.5 ml of N,N-dimethylformamide, 60 mg of cyanoacetohydrazide was added, and the mixture was stirred at 80°C for 9 hours. This was mixed with 30 ml of ethyl acetate, the mixture was washed successively with water and then saturated saline, and the ethyl acetate layer was dehydrated with anhydrous sodium sulfate and concentrated to dryness. The residue was dissolved in a small quantity of methanol, subjected to a chromatograph tower of Sephadex LH-20 (1.5 x 15 cm) and eluted with methanol. The fractions containing the desired product were concentrated to dryness to give 27.8 mg of the captioned compound represented by the formula (41).

Rf value: 0.53 (produced by Merck Co., Kiesel gel 60F₂₅₄, developing solvent; chloroform : methanol: tetrahydrofuran = 3:1:0.1)
FAB-MS(m/z): 601 (M+H)⁺
¹H-NMR (300 MHz, DMSO-d₆), δ(ppm): 11.14 (1H, s), 11.01 (1H, s), 10.42 (1H, s), 10.04 (1H, s), 8.65 (1H, d, J=7.6Hz), 8.47 (1H, d, J=7.6Hz), 7.21 (2H, t, J=7.6Hz), 7.05 (3H, t, J=7.6Hz), 5.41 (2H, d, J=4.5Hz), 5.19 (1H, d, J=6.8Hz), 4.90 (1H, d, J=6.8Hz), 4.13 (2H, s), 4.04 (2H, br), 3.75 (1H, m), 3.64 (2H, m), 3.43 (1H, m)

### Example 40

The compound represented by the formula 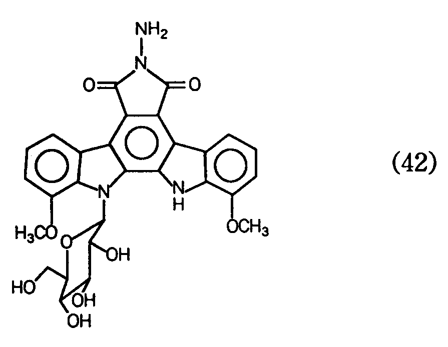
1 g of 12,13-dihydro-1,11-dihydroxy-13-(β-D-glucopyranosyl)-5H-indolo[2,3-a]pyrrolo[3,4-c]carbazole-5,7(6H)-dione was dissolved in 25 ml of tetrahydrofuran, an ether solution of an excessive quantity of diazomethane was added, the mixture was stirred at 4°C overnight, and the formed yellow precipitate was collected by filtration. This was dissolved in 3 ml of hydrazine monohydrate, and the solution was subjected to reaction at room temperature for 1.5 hours. After the reaction, 200 ml of purified water was added, and the resultant precipitate was collected by filtration, washed successively with purified water and then methanol, and dried under reduced pressure to give 683.4 mg of the captioned compound represented by the formula (42).

HPLC; Rt, 10.5 minutes (column : Chromatolex ODS, inner diameter 4.6 mm, length 250 mm, detection; UV 305 nm, flow rate; 1 ml/minute, moving phase; methanol : water = 6:4)
FAB-MS(m/z): 563 (M+H)⁺
¹H-NMR (300 MHz, DMSO-d₆), δ(ppm): 10.9 (1H, s), 8.87 (1H, d, J=7.8Hz), 8.65 (1H, d, J=7.8Hz), 7.35 (1H, t, J=7.8Hz), 7.23 (1H, t, J=7.8Hz), 7.25 (1H, d, J=7.8Hz), 7.18 (1H, d, J=7.8Hz), 6.90 (1H, d, J=9.3Hz), 5.40 (1H, brs), 5.18 (1H, brs), 5.00 (2H, brs), 4.90 (2H, brs), 4.06 (6H, s), 4.00 (2H, m), 3.78 (1H, m), 3.63 (2H, m), 3.42 (1H, m)

### Example 41

The compound represented by the formula 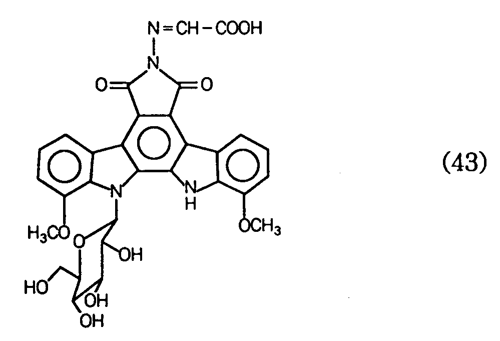
708.8 mg of the captioned compound represented by the formula (43) was obtained from 679 mg of the compound obtained in Example 40, according to the process of Example 2,

HPLC; Rt, 10.9 minutes (column : Chromatolex ODS, inner diameter 4.6 mm, length 250 mm, detection; UV 310 nm, flow rate; 1 ml/minute, moving phase; acetonitrile : water = 2.8 → acetonitrile : water = 6:4, 30 minutes linear gradient)
FAB-MS(m/z): 618 [M]⁺
¹H-NMR (400 MHz, DMSO-d₆), δ(ppm): 13.5 (1H, brs), 11.1 (1H, s), 9.01 (1H, s), 8.83 (1H, d, J=7.8Hz), 8.63 (1H, d, J=7.8Hz), 7.39 (1H, t, J=7.8Hz), 7.37 (1H, t, J=7.8Hz), 7.29 (1H, d, J=7.8Hz), 7.22 (1H, d, J=7.8Hz), 6.94 (1H, d, J=9.3Hz), 5.43 (1H, d, J= 5.4Hz), 5.22 (1H, d, J=5.4Hz), 5.01 (1H, brs), 4.93 (1H, d, J=5.4Hz), 4.07 (6H, s), 4.05 (1H, m), 3.96 (1H, m), 3.79 (1H, m), 3.60 (2H, m), 3.44 (1H, m)

### Example 42

The compound represented by the formula 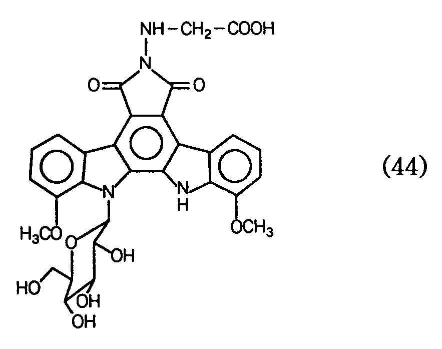
704 mg of the compound obtained in Example 41 was dissolved in 10 ml of N,N-dimethylformamide, 60 mg of 10% palladium-carbon (Pd-C) was added, and the mixture was subjected to hydrogenation at room temperature for 6 hours under stirring. The reaction mixture was filtered using a sheet of filter paper on which Celite was spread to remove Pd-C, 200 ml of ethyl acetate was added to the filtrate, and the mixture was extracted with 50 ml of sodium bicarbonate aqueous solution (pH 8). The water layer was adjusted to pH 2 and extracted with ethyl acetate (500 ml). The ethyl acetate layer was extracted with 2% sodium bicarbonate aqueous solution (70 ml). The 2% sodium bicarbonate aqueous solution layer was concentrated under reduced pressure, adsorbed on a column of Diaion HP 20 (inner diameter 3 cm, length 30 cm), washed with water, and then eluted with 300 ml of methanol. The methanol eluate was concentrated to dryness, the residue was dissolved in a small quantity of N,N-dimethylformamide, and the solution was subjected to preparative HPLC (column : Chromatolex ODS, inner diameter 20 mm, length 250 mm, detection; UV 310 nm, flow rate; 9 ml/minute, moving phase; acetonitrile : water = 25:75). The fractions containing the desired product were concentrated to dryness, and the residue was dissolved in a small quantity of water, subjected to column chromatography of Sephadex G-15 (inner diameter 3 cm, length 63 cm) and eluted with water : methanol = 9:1. The fractions containing the desired product were concentrated and then freeze dried to give 84.2 mg of the sodium salt of the captioned compound represented by the formula (44).

HPLC; Rt, 8.9 minutes (column : Chromatolex ODS, inner diameter 4.6 mm, length 250 mm, detection; UV 310 nm, flow rate; 1 ml/minute, moving phase; acetonitrile : water : trifluoroacetic acid = 300:700:1)
FAB-MS(m/z): 643 (M+Na)⁺
¹H-NMR (400 MHz, DMSO-d₆), δ(ppm): 10.9 (1H, brs), 8.85 (1H, d, J=7.8Hz), 8.63 (1H, d, J=7.8Hz), 7.33 (1H, t, J=7.8Hz), 7.31 (1H, t, J=7.8Hz), 7.24 (1H, d, J=7.8Hz), 7.16 (1H, d, J=7.8Hz), 6.89 (1H, d, J=9.3Hz), 5.63 (1H, brs), 5.42 (1H, brs), 5.10 (1H, brs), 4.99 (1H, brs), 4.06 (6H, s), 4.02 (2H, m), 3.80 (1H, m), 3.67 (1H, t, J=8.8Hz), 3.58 (1H, m), 3.42 (1H, t, J=8.3Hz), 3.34 (2H, s)

### Example 43

The compound represented by the formula 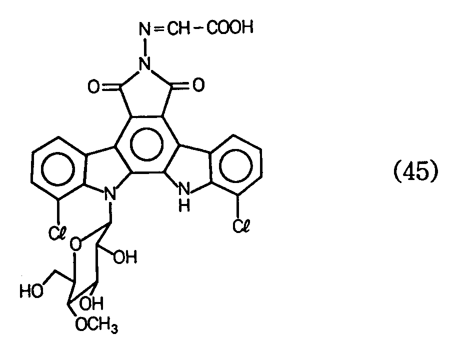
23.8 mg of the captioned compound represented by the formula (45) was obtained from 70 mg of the compound obtained in Example 4, according to the same process as in Example 2.
FAB-MS(m/z): 641 (M+H)⁺
¹H-NMR (400 MHz, DMSO-d₆), δ(ppm): 10.8 (1H, s), 9.26 (1H, d, J=7.8Hz), 9.09 (1H, d, J=7.8Hz), 8.94 (1H, s), 7.78 (1H, d, J=7.8Hz), 7.74 (1H, d, J=7.8Hz), 7.50 (2H, t, J= 7.8Hz), 6.98 (1H, d, J=9.3Hz), 5.44 (1H, d, J=5.9Hz), 5.33 (1H, brs), 5.09 (1H, d, J=5.4Hz), 3.96 (2H, m), 3.85 (1H, m), 3.67 (2H, m), 3.59 (3H, s), 3.56 (1H, m)

### Example 44

The compound represented by the formula 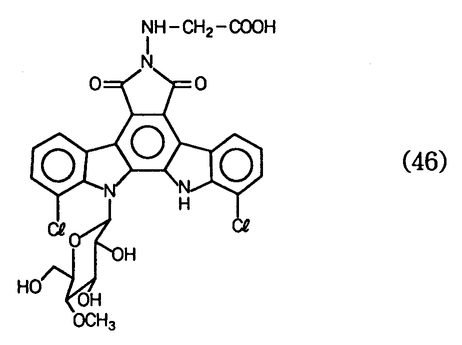
210 mg of the captioned compound represented by the formula (46) was obtained from 1 g of the compound obtained in Example 43, according to the same process as in Example 42.
FAB-MS(m/z): 643 (M+H)⁺
¹H-NMR (500 MHz, DMSO-d₆), δ(ppm): 10.7 (1H, s), 9.26 (1H, d, J=7.8Hz), 9.09 (1H, d, J=7.8Hz), 7.74 (1H, d, J=7.8Hz), 7.71 (1H, d, J=7.8Hz), 7.46 (2H, t, J=7.8Hz), 6.93 (1H, d, J=9.2Hz), 6.00 (1H, brs), 5.42 (1H, brs), 5.31 (1H, brs), 5.03 (1H, brs), 3.96 (2H, brs), 3.85 (2H, s), 3.83 (1H, m), 3.59 (3H, s), 3.50-3.70 (3H, m)

### Example 45

The compound represented by the formula 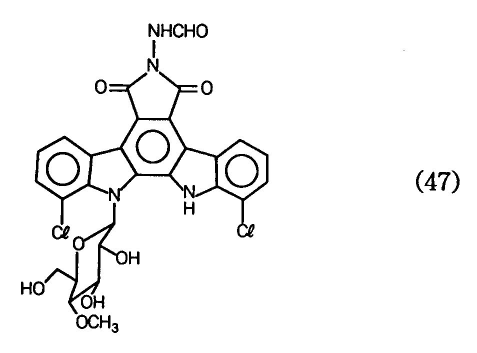
48.2 mg of the captioned compound represented by the formula (47) was obtained from 51.4 mg of the compound obtained in Example 4, according to the same process as in Example 5.
FAB-MS(m/z): 613 (M+H)⁺
¹H-NMR (400 MHz, DMSO-d₆), δ(ppm): 10.9 (1H, brs), 10.8 (1H, brs), 9.20 (1H, m), 9.03 (1H, m), 8.48 (1H, s), 7.75 (1H, d, J=7.8Hz), 7.70 (1H, d, J=7.8Hz), 7.45 (2H, t, J= 7.8Hz), 6.93 (1H, brt, J=9.3Hz), 5.41 (2H, m), 5.04 (1H, d, J=5.9Hz), 3.99 (2H, brs), 3.86 (1H, m), 3.60 (3H, s), 3.52-3.67 (3H, m)

### Example 46

The compound represented by the formula 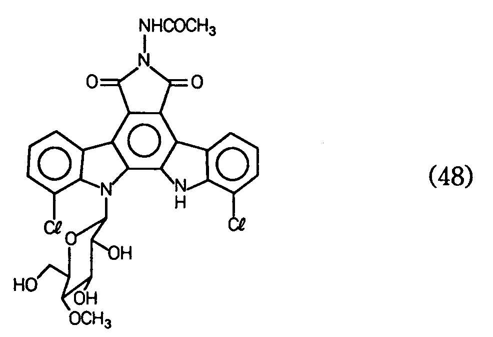
13 mg of the captioned compound represented by the formula (48) was obtained from 14.1 mg of the compound obtained in Example 4, according to the same process as in Example 6.
FAB-MS(m/z): 627 (M+H)⁺
¹H-NMR (500 MHz, DMSO-d₆), δ(ppm): 10.8 (2H, s), 9.20 (1H, m), 9.04 (1H, m), 7.74 (2H, m), 7.47 (2H, m), 6.93 (1H, m), 5.41 (1H, m), 5.32 (1H, brs), 5.04 (1H, m), 3.96 (2H, brs), 3.85 (1H, m), 3.58 (3H, s), 3.50-3.70 (3H, m), 2.12 (3H, s)

### Example 47

The compound represented by the formula 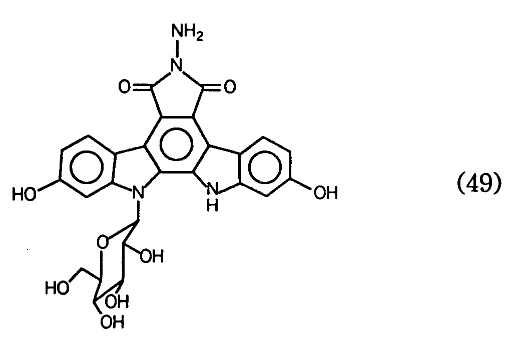
1 ml of hydrazine monohydrate was added to 3.2 mg of 12,13-dihydro-2,10-dihydroxy-13-(β-D-glucopyranosyl)-5H-indolo[2,3-a]pyrrolo[3,4-c]carbazole-5,7(6H)-dione, and the mixture was stirred at room temperature for 2 hours. This was distributed with ethyl acetate-0.2N hydrochloric acid, and the ethyl acetate layer was washed successively with water then saturated saline, and concentrated to dryness. The residue was dissolved in a small quantity of methanol, subjected to a chromatograph tower of Sephadex LH-20 (1.0 x 5 cm) and eluted with methanol. The fractions containing the desired product were concentrated to dryness to give 3.0 mg of the captioned compound represented by the formula (49).

Rf value: 0.22 (produced by Merck Co., Kiesel gel 60F₂₅₄, developing solvent; chloroform : methanol: tetrahydrofuran = 3:1:1)
FAB-MS(m/z): 534 [M]⁺
¹H-NMR (300MHz, DMSO-d₆), δ(ppm): 11.16 (1H, s), 9.76 (1H, s), 9.73 (1H, s), 8.90 (1H, d, J=7.3Hz), 8.82 (1H, d, J=7.3Hz), 7.18 (1H, d, J=2.0Hz), 6.98 (1H, d, J=2.0Hz), 6.83 (2H, dt, J=2.0, 7.3Hz), 5.97 (1H, d, J= 7.2Hz), 5.84 (1H, t, J=3.3Hz), 5.32 (1H, d, J=5.3Hz), 5.10 (1H, d, J=5.3Hz), 4.93 (1H, d, J=5.2Hz), 4.90 (2H, s), 4.04-3.86 (2H, m), 3.78 (1H, m), 3.60-3.35 (3H, m)

### Example 48

The compound represented by the formula 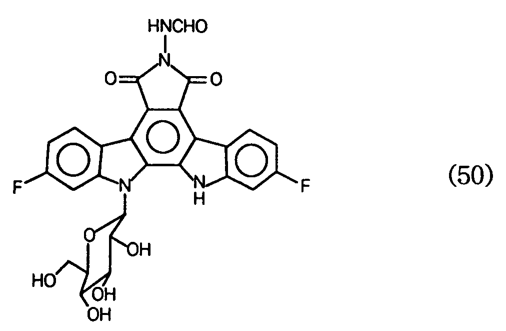
0.4 ml of hydrazine hydrate was added to 7.1 mg of 2,10-difluoro-12,13-dihydro-13-(β-D-glucopyranosyl)-5H-indolo[2,3-a]pyrrolo[3,4-c]carbazole-5,7(6H)-dione, and the mixture was stirred at room temperature for 40 minutes. 1.34 ml of concentrated hydrochloric acid was added thereto, and the mixture was extracted with ethyl acetate. The ethyl acetate layer was washed with water and concentrated. The residue was dissolved in 3.7 ml of N,N-dimethylformamide and 0.37 ml of concentrated hydrochloric acid, and the solution was stirred at room temperature overnight. This was distributed between ethyl acetate and water, and the ethyl acetate layer was concentrated to dryness. The residue was dissolved in a small quantity of ethanol, subjected to a chromatograph tower of Sephadex LH-20 and eluted with ethanol. The fractions containing the desired product were concentrated to dryness to give 4.6 mg of the captioned compound represented by the formula (50).
FAB-MS(m/z): 566 [M]⁺
¹H-NMR (400 MHz, DMSO-d₆), δ(ppm): 11.9 (1H, s), 10.8 (1H, brs), 9.07 (1H, dd, J=5.8, 8.8Hz), 9.01 (1H, dd, J=5.9, 8.8Hz), 8.45 (1H, s), 7.93 (1H, brd, J=8.8Hz), 7.44 (1H, brd, J=8.8Hz), 7.27 (2H, m), 6.28 (1H, d, J=8.8Hz), 6.20 (1H, brs), 5.42 (1H, brs), 5.13 (1H, brd, J=5.4Hz), 4.96 (1H, d, J=5.4Hz), 4.09 (1H, brd, J=7.3Hz), 3.94 (2H, m), 3.83 (1H, brd, J=7.3Hz), 3.58 (1H, m), 3.45 (1H, m)

### Example 49

The compound represented by the formula 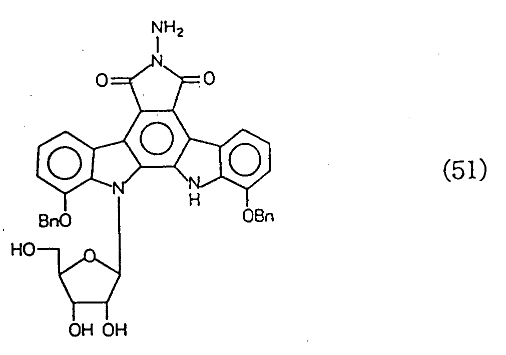
wherein Bn represents a benzyl group.

100 mg of 6-benzyloxymethyl-1,11-dibenzyloxy-12,13-dihydro-5H-indolo[2,3-a]pyrrolo[3,4-c]carbazole-5,7(6H)-dione, 1.4 g of silver oxide and 0.7 g of 4Å molecular sieve were suspended in 40 ml of anhydrous benzene, the suspension was refluxed with heating for 20 minutes, and then a solution of 1-bromo-2,3,5-tri-O-acetyl-D-ribose in 10 ml of anhydrous benzene was added dropwise over a period of 10 minutes. The mixture was further refluxed with heating for 3 hours, and the insoluble matters were filtered using Celite.

The filtrate was concentrated to dryness, and the residue was dissolved in 100 ml of ethyl acetate and the solution was washed successively with 0.2N hydrochloric acid, water and then saturated saline, dried over anhydrous sodium sulfate, and concentrated to dryness. The residue was dissolved in chloroform, subjected to a chromatograph tower of Sephadex LH-20 (2.5 x 20 cm) and eluted with chloroform. The fractions containing the desired product were concentrated to dryness, the residue was subjected to a chromatograph tower of silica gel (2.5 x 25 cm) and eluted with toluene-ethyl acetate (3:1), and the fractions containing the desired product were concentrated to dryness. The residue was further purified by preparative thin layer chromatography (toluene-ethyl acetate = 5:1 (Rf = 0.6)) to give 20.8 mg of 6-benzyloxymethyl-1,11-dibenzyloxy-12,13-dihydro-13-(β-D-ribofuranosyl)-5H-indolo[2,3-a]pyrrolo[3,4-c]carbazole-5,7(6H)-dione.

20.8 mg of this compound was dissolved in 2 ml of hydrazine monohydrate, and the solution was stirred at room temperature for 2 hours. This was mixed with 30 ml of ethyl acetate, the mixture was washed successively with 0.2N hydrochloric acid, water and then saturated saline, and concentrated to dryness. The residue was dissolved in methanol, subjected to a chromatograph tower of Sephadex LH-20 (1.0 x 15 cm) and eluted with methanol. The fractions containing the desired product were concentrated to dryness, and the residue was purified by preparative thin layer chromatography (chloroform-methanol = 10:1 (Rf = 0.5)) to give 2.9 mg of the captioned compound represented by the formula (51).

Rf value: 0.5 (produced by Merck Co., Kiesel gel 60F₂₅₄, developing solvent; chloroform : methanol = 10:1)
FAB-MS(m/z): 684 [M]⁺
¹H-NMR (300 MHz, DMSO-d₆), δ(ppm): 10.45 (1H, s), 8.90 (1H, d, J=0.75Hz), 8.68 (1H, d, J=0.75Hz), 7.18 (2H, d, J=0.75Hz), 7.11 (2H, d, J=0.75Hz), 7.20-7.50 (11H, m), 5.35-5.45 (5H, m), 5.17 (1H, d, J=0.38Hz), 5.10 (1H, d, J=0.45Hz), 4.98 (2H, s), 3.90-4.00 (2H, m), 3.60-3.70 (2H, m)

### Example 50

The compound represented by the formula 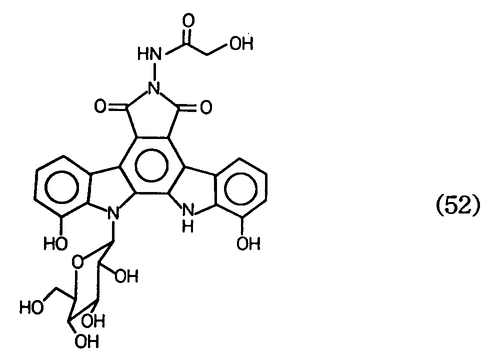
33.0 mg of the compound obtained in Example A was dissolved in 3 ml of N,N-dimethylformamide, 8.4 mg of hydroxyacetohydrazide was added, and the mixture was stirred at 80°C for 2 days. This was concentrated to dryness, and the residue was dissolved in a small quantity of methanol, subjected to a chromatograph tower of Sephadex LH-20 (1.5 x 25 cm), and eluted with methanol. The fractions containing the desired product were concentrated to dryness, and the residue was dissolved in 30 ml of ethyl acetate. The solution was washed with water, and the ethyl acetate layer was dried over anhydrous sodium sulfate and concentrated to dryness. The residue was dissolved in a small quantity of methanol, subjected to a chromatograph tower of Sephadex LH-20 (1.5 x 15 cm) and eluted with methanol. The fractions containing the desired product were concentrated to dryness to give 29.0 mg of the captioned compound represented by the formula (52).
FAB-MS(m/z): 593 [M+H]⁺
¹H-NMR (300 MHz, DMSO-d₆), δ(ppm): 11.00 (1H, s), 10.55 (1H, s), 10.41 (1H, s), 10.02 (1H, s), 8.63 (1H, d, J=7.8Hz), 8.47 (1H, d, J=7.8Hz), 7.20 (2H, t, J=7.8Hz), 7.04 (3H, m), 5.88 (1H, t, J=7.0Hz), 5.41 (1H, d, J=6.2Hz), 5.35 (1H, br), 5.20 (1H, d, J=6.2Hz), 4.90 (1H, d, J=6.2Hz), 4.16 (2H, d, J=5.7Hz), 4.03 (2H, m), 3.74 (1H, m), 3.59-3.68 (2H, m), 3.39 (1H, m)

### Example 51

The compound represented by the formula 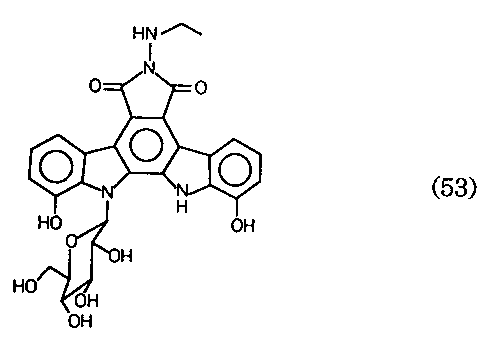
35.0 mg of the compound obtained in Example A was dissolved in 1.0 ml of N,N-dimethylformamide, 35.0 mg of ethylhydrazine oxalate and 0.5 ml of saturated sodium bicarbonate aqueous solution were added, and the mixture was stirred at 80°C for 1 day. This was concentrated to dryness, and the residue was dissolved in a small quantity of methanol, subjected to a chromatograph tower of Sephadex LH-20 (1.5 x 15 cm) and eluted with methanol. The fractions containing the desired product were concentrated to dryness to give 20.8 mg of the captioned compound represented by the formula (53).

Rf value: 0.5 (produced by Merck Co., Kiesel gel 60F₂₅₄, developing solvent; chloroform : methanol : tetrahydrofuran = 2:1:1)
FAB-MS(m/z): 563 [M+H]⁺
¹H-NMR (300 MHz, DMSO-d₆), δ(ppm): 10.90 (1H, s), 10.35 (1H, s), 9.96 (1H, s), 8.72 (1H, d, J=7.9Hz), 8.54 (1H, d, J=7.9Hz), 7.17 (2H, t, J=7.9Hz), 7.03 (3H, m), 5.72 (1H, t, J=4.8Hz), 5.41 (1H, d, J=6.3Hz), 5.35 (1H, t, J=4.0Hz), 5.21 (1H, d, J=4.0Hz), 4.87 (1H, d, J=6.3Hz), 3.96-4.09 (2H, m), 3.73-3.77 (1H, m), 3.58-3.67 (2H, m), 3.37-3.45 (1H, m), 3.07 (2H, m), 1.09 (3H, t, J=7.1Hz)

### Example 52

50 g of the compound of Example 5 was dissolved in a solution wherein 600 g of macrogol 400 of the Japanese Pharmacopoeia was dissolved in 400 g of distilled water for injection, and the solution was filtered for removal of bacteria using a filter of 0.2 µm. 5 ml portions of the filtrate were filled into washed and sterilized vials according to a conventional method, and the vials were stopped and capped to give an injection containing 250 mg of the compound of Example 5 per vial. Administration is made using an agent for intravenous drip wherein 5 to 10 ml of this injection (250 to 500 mg of the compound of Example 5) was added to and diluted with 500 ml of an infusion such as 5% glucose.

## Claims (Claims for the following Contracting State(s): AT, BE, CH, LI, DE, DK, FR, GB, GR, IE, IT, LU, MC, NL, PT, SE)

1. A compound represented by the following general formula and a pharmaceutically acceptable salt thereof 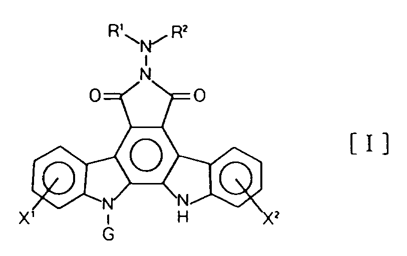 wherein
R¹ and R² each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and heterocyclic group may each have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and therein Y represents a carbonyl group, thiocarbonyl group or sulfonyl group and R³ represents a hydrogen atom, C₁-C₆ alkyl group, cycloalkyl group, cycloalkylalkyl group, aromatic hydrocarbon group having 6 to 12 carbon atoms, arakyl group, C₁-C₆ alkoxy group, hydrazino group, amino group, arylamino group, carbamoyl group or 5-or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by substituent(s) selected from the group consisting of halogen atoms, hydroxyl groups, amino groups, carboxyl groups, carbamoyl groups and C₁-C₆ alkoxycarbonyl groups); or
R¹ and R² combine to represent a C₁-C₆ alkylidene group (the C₁-C₆ alkylidene group may have 1 to 4 substituents selected from the group consisting of amino groups, mono-C₁-C₆ alkylamino groups, di-C₁-C₆ alkylamino groups, hydroxyl groups, carboxyl groups and sulfonyl groups); or
R¹ and R² combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by group(s) selected from the group consisting of amino groups, hydroxyl groups, carboxyl groups and sulfo groups),
G represents a pentose group or hexose group, and
X¹ and X² each independently represent a hydrogen atom, halogen atom, amino group, mono-C₁-C₆ alkylamino group, di-C₁-C₆ alkylamino group, hydroxyl group, C₁-C₆ alkoxy group, aralkoxy group, carboxyl group, C₁-C₆ alkoxycarbonyl group or C₁-C₆ alkyl group.

2. A compound set forth in claim 1 which is a compound represented by the formula 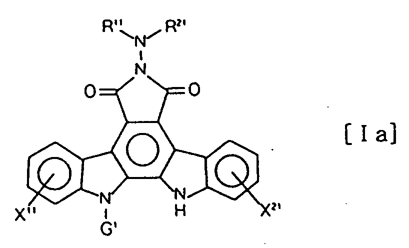 wherein
R¹¹ and R²¹ each independently represent a hydrogen atom, C₁-C₆ alkyl group, C₁-C₆ alkenyl group, aromatic hydrocarbon group having 6 to 12 carbon atoms, aralkyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group, thiazolidinyl group (the C₁-C₆ alkyl group, C₁-C₆ alkenyl group, aromatic hydrocarbon group having 6 to 12 carbon atoms, aralkyl group and hetercyclic group may have 1 to 5 substituents selected from the group consisting of carboxyl groups, carbamoyl groups, cyano groups and hydroxyl groups), or a group of the formula -Y-R³¹, and therein Y represents a carbonyl group, thiocarbonyl group or sulfonyl group, and R³¹ represents a hydrogen atom, C₁-C₆ alkyl group, aromatic hydrocarbon group having 6 to 12 carbon atoms (the C₁-C₆ alkyl group and aromatic hydrocarbon group having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group or thiazolidinyl group; or
R¹¹ and R²¹ combine to represent a C₁-C₆ alkylidene group optionally having carboxyl group(s), or
R¹¹ and R²¹ combine together with the nitrogen atom to which they bind to form a pyrrolidinyl group, imidazolidinyl group, imidazolinyl group, piperidino group or piperazinyl group (these heterocyclic groups may have on the ring C₁-C₆ alkyl group(s) optionally substituted by hydroxyl group(s)),
G¹ represents a group of the formula 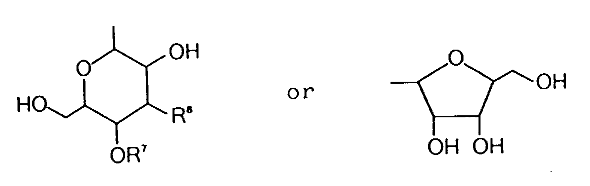 and therein R⁷ represents a hydrogen atom or C₁-C₆ alkyl group and R⁸ represents a hydroxyl group or amino group, and X¹¹ and X²¹ bind to the indolopyrrolocarbazole ring at the 1- or 2-position and at the 10- or 11-position, respectively, and each independently represent a halogen atom, hydroxyl group, C₁-C₆ alkoxy group or aralkoxy group.

3. A compound set forth in claim 1 which is a compound represented by the formula 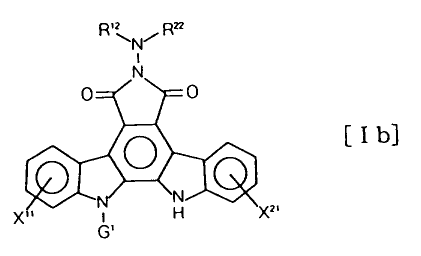 wherein
R¹² represents a hydrogen atom or C₁-C₆ alkyl group,
R²² represents a hydrogen atom, C₁-C₆ alkyl group (the C₁-C₆ alkyl group may have 1 to 5 substituents selected from the group consisting of carboxyl groups, carbamoyl groups, hydroxyl groups and cyano groups), aromatic hydrocarbon group having 6 to 12 carbon atoms, aralkyl group (the aromatic hydrocarbon group having 6 to 12 carbon atoms and aralkyl group may have 1 to 4 substituents selected from the group consisting of hydroxyl groups and carboxyl groups), pyridyl group, imidazolyl group, imidazolinyl group, thiazolyl group, pyrrolidinyl group, piperazinyl group, or a group of the formula -Y-R³², and therein Y represents a carbonyl group, thiocarbonyl group or sulfonyl group, and when Y is a carbonyl group or thiocarbonyl group, R³² represents a hydrogen atom, C₁-C₆ alkyl group, aromatic hydrocarbon group having 6 to 12 carbon atoms (the C₁-C₆ alkyl group and aromatic hydrocarbon group having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyridyl group, pyrimidinyl group, imidazolinyl group or pyrrolidinyl group, and when Y is a sulfonyl group, R³² represents a C₁-C₆ alkyl group or aromatic hydrocarbon group having 6 to 12 carbon atoms; or
R¹² and R²² combine to represent a C₁-C₆ alkylidene group having carboxyl group(s); or
R¹² and R²² combine together with the nitrogen atom to which they bind to form a pyrrolidinyl group, piperidino group or piperazinyl group (these heterocyclic groups may have on the ring C₁-C₆ alkyl groups optionally having hydroxyl group(s)), and
G¹, X¹¹ and X²¹ have the same meanings as defined in claim 2.

4. A compound of the general formula [I] set forth in claim 1 or a pharmaceutically acceptable salt thereof as a pharmaceutically acceptable substance.

5. A process for preparation of a compound of the general formula [I] set forth in claim 1 or a pharmaceutically acceptable salt thereof which comprises reacting a compound represented by the following formula or a derivative thereof wherein the functional groups are protected 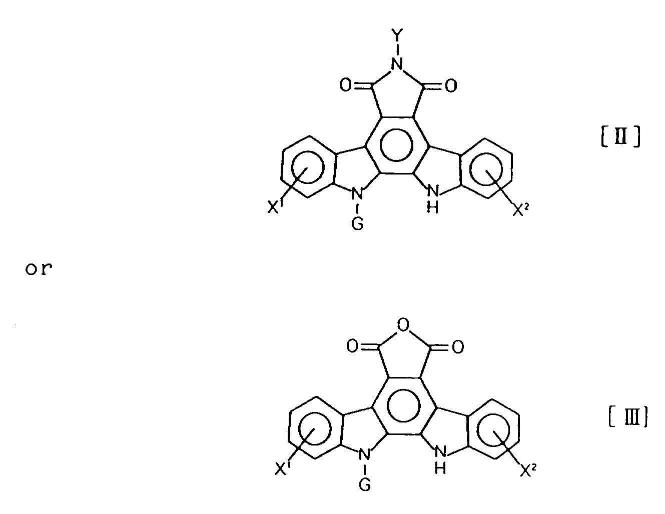 wherein
Y represents a hydrogen atom or substituted or unsubstituted C₁-C₆ alkyl group, and X¹, X² and G have the same meanings as defined in claim 1 with a compound represented by the following general formula or a derivative thereof wherein in case R¹³ and R²³ contain a functional group, the functional group is each protected 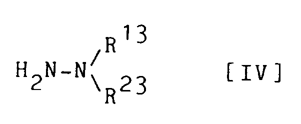 wherein
R¹³ and R²³ each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and herein Y represents a carbonyl group, thiocarbonyl group or sulfonyl group, and R³ represents a hydrogen atom, C₁-C₆ alkyl group, cycloalkyl group, cycloalkylalkyl group, aromatic hydrocarbon group having 6 to 12 carbon atoms, aralkyl group, C₁-C₆ alkoxy group, hydrazino group, amino group, arylamino group, carbamoyl group or 5-or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and heterocyclic group may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by group(s) seleceted from the group consisting of halogen atoms, hydroxyl groups, amino groups, carboxyl groups, carbamoyl groups and C₁-C₆ alkoxycarbonyl groups); or
R¹³ and R²³ combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by groups selected from the group consisting of amino groups, hydroxyl groups, carboxyl groups and sulfo groups); if necessary, removing the protective group(s) existing in the product to prepare a compound represented by the general formula 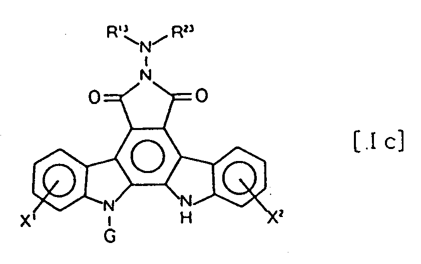 wherein R¹³, R²³, X¹, X² and G have the same meanings as defined above;
or either formylating, alkylating, alkenylating, alkynylating, aralkylating, carbamoylating, thiocarbamoylating, alkanoylating or sulfonylating the amino group 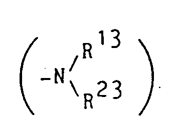 of the compound of the above formula [Ic] or the derivative thereof wherein the functional groups are protected when R¹³ and R²³ represent a hydrogen atom, or condensing the above compound or derivative with a compound represented by the following formula or a derivative thereof wherein a functional group is protected
OHC-R⁶ [IV]
wherein R⁶ represents a hydrogen atom or carboxyl group, or a C₁-C₆ alkyl group optionally having 1 to 4 substituents selected from the group consisting of amino groups, mono-C₁-C₆ alkylamino groups, di-C₁-C₆ alkylamino groups, hydroxyl groups, carboxyl groups and sulfo groups,
and if necessary, removing the protective groups existing in the product; or reducing the double bonds of the compound of the above formula [Ic] when R¹³ and/or R²³ contain a double bond, or the compound prepared by condensing the compound of the formula [Ic] with the compound of the formula [V] or the derivative thereof wherein the functional groups are protected, and if necessary removing the protective group(s) existing in the product; and if necessary, converting the resulting compound of the formula [I] into a pharmaceutically acceptable salt.

6. A compound represented by the following general formula and a derivative thereof wherein the functional groups are protected 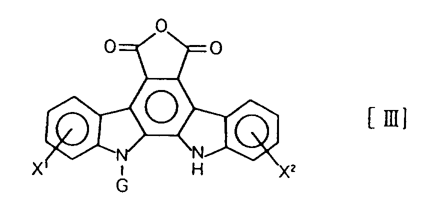 wherein X¹, X² and G have the same meanings as in claim 1.

7. A process for preparation of a compound of the general formula [III] set forth in claim 6 which comprises treating with a base a compound represented by the following general formula or a derivative thereof wherein the functional groups are protected 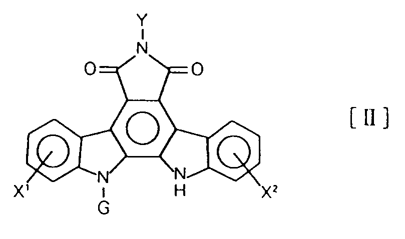 wherein Y represents a hydrogen atom or substituted or unsubstituted C₁-C₆ alkyl group, and X¹, X² and G have the same meanings as defined in claim 1.

8. A medicament containing a compound of the general formula [I] set forth in claim 1 or a pharmaceutically acceptable salt thereof.

9. An antitumor agent containing a compound of the general formula [I] set forth in claim 1 or a pharmaceutically acceptable salt thereof.

10. A pharmaceutical preparation comprising an effective quantity of a compound of the general formula [I] set forth in claim 1 or a pharmaceutically acceptable salt thereof, and a pharmaceutically acceptable carrier or diluent.

11. A compound of the general formula [I] set forth in claim 1 or a pharmaceutically acceptable salt thereof for use in a method for treatment of cancer.

12. The use of a compound of the general formula [I] set forth in claim 1 or a pharmaceutically acceptable salt thereof in the control or prevention of illness.

13. The use of a compound of the general formula [I] set forth in claim 1 or a pharmaceutically acceptable salt thereof for the manufacture of antitumor active medicament.

14. A compound represented by the following formula:

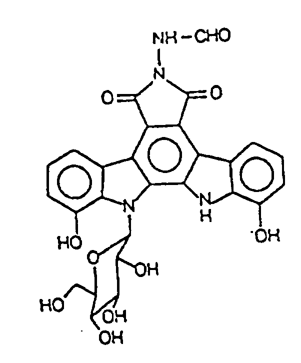

15. A compound represented by the following formula:

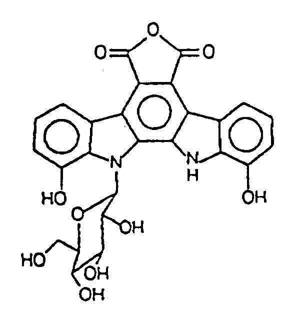

16. A process according to Claim 5 wherein a compound represented by the formula: 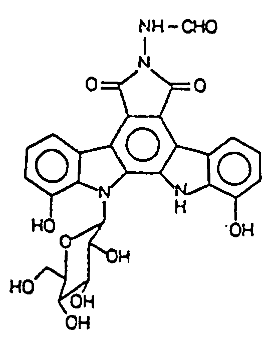 is prepared.

17. A process according to Claim 7 wherein a compound represented by the formula: 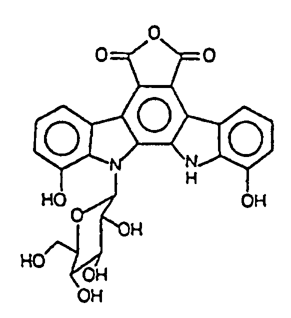 is prepared.

## Claims (Claims for the following Contracting State(s): ES)

1. A process for preparation of a compound represented by the following general formula and a pharmaceutically acceptable salt thereof 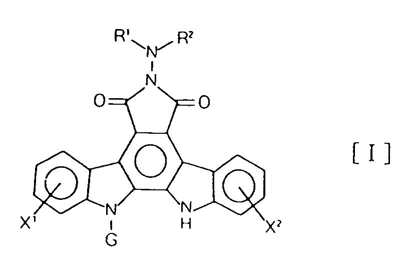 wherein
R¹ and R² each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and heterocyclic group may each have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and therein Y represents a carbonyl group, thiocarbonyl group or sulfonyl group and R³ represents a hydrogen atom, C₁-C₆ alkyl group, cycloalkyl group, cycloalkylalkyl group, aromatic hydrocarbon group having 6 to 12 carbon atoms, arakyl group, C₁-C₆ alkoxy group, hydrazino group, amino group, arylamino group, carbamoyl group or 5-or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by substituent(s) selected from the group consisting of halogen atoms, hydroxyl groups, amino groups, carboxyl groups, carbamoyl groups and C₁-C₆ alkoxycarbonyl groups); or
R¹ and R² combine to represent a C₁-C₆ alkylidene group (the C₁-C₆ alkylidene group may have 1 to 4 substituents selected from the group consisting of amino groups, mono-C₁-C₆ alkylamino groups, di-C₁-C₆ alkylamino groups, hydroxyl groups, carboxyl groups and sulfonyl groups); or
R¹ and R² combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by group(s) selected from the group consisting of amino groups, hydroxyl groups, carboxyl groups and sulfo groups),
G represents a pentose group or hexose group, and
X¹ and X² each independently represent a hydrogen atom, halogen atom, amino group, mono-C₁-C₆ alkylamino group, di-C₁-C₆ alkylamino group, hydroxyl group, C₁-C₆ alkoxy group, aralkoxy group, carboxyl group, C₁-C₆ alkoxycarbonyl group or C₁-C₆ alkyl group which comprises reacting a compound represented by the following formula or a derivative thereof wherein the functional groups are protected 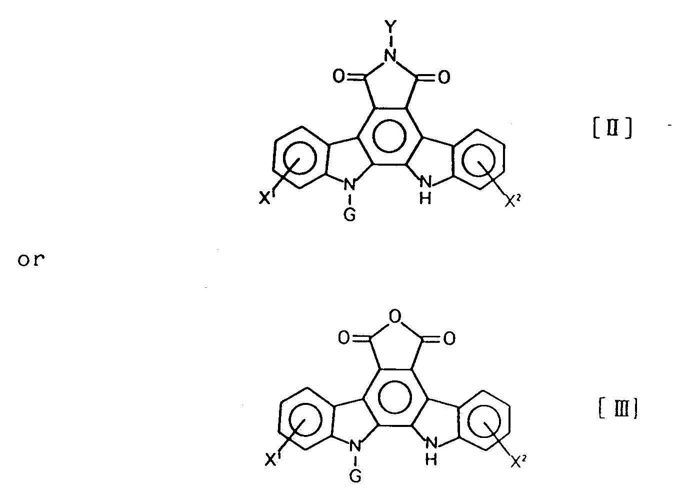 wherein
Y represents a hydrogen atom or substituted or unsubstituted C₁-C₆ alkyl group, and X¹, X² and G have the same meanings as defined in claim 1 with a compound represented by the following general formula or a derivative thereof wherein in case R¹³ and R²³ contain a functional group, the functional group is each protected 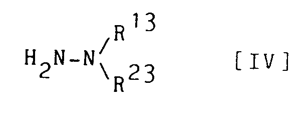 wherein
R¹³ and R²³ each independently represent a hydrogen atom, C₁-C₆ alkyl group, C₁-C₆ alkenyl group, C₁-C₆ alkynyl group, aromatic hydrocarbon group having 6 to 12 carbon atoms, aralkyl group or 5- or 6-membered heterocyclic group containing 1 to 4 heteroatoms selected from the group consisting of nitrogen atoms, oxygen atoms and sulfur atoms (the C₁-C₆ alkyl group, C₁-C₆ alkenyl group, C₁-C₆ alkynyl group, aromatic hydrocarbon group having 6 to 12 carbon atoms, aralkyl group and 5- or 6-membered heterocyclic group containing 1 to 4 heteroatoms selected from the group consisting of nitrogen atoms, oxygen atoms and sulfur atoms may have 1 to 5 substituents selected from the group consisting of carboxyl groups, carbamoyl groups, sulfo groups, amino groups, cyano groups, mono-C₁-C₆ alkylamino groups, di-C₁-C₆ alkylamino groups, hydroxyl groups and halogen atoms), or a group of the formula -Y-R³, and herein Y represents a carbonyl group, thiocarbonyl group or sulfonyl group, and R³ represents a hydrogen atom, C₁-C₆ alkyl group, cycloalkyl group, cycloalkylalkyl group, aromatic hydrocarbon group having 6 to 12 carbon atoms, aralkyl group, C₁-C₆ alkoxy group, hydrazino group, amino group, arylamino group, carbamoyl group or 5- or 6-membered heterocyclic group containing 1 to 4 heteroatoms selected from the group consisting of nitrogen atoms, oxygen atoms and sulfur atoms (the C₁-C₆ alkyl group, cycloalkyl group, cycloalkylalkyl group, aromatic hydrocarbon group having 6 to 12 carbon atoms, aralkyl group and heterocyclic group may each have 1 to 4 substituents selected from the group consisting of halogen atoms, optionally protected hydroxyl groups, amino groups, carboxyl groups, carbamoyl groups, cyano groups and C₁-C₆ alkoxycarbonyl groups, and the amino group and carbamoyl group may each be mono- or di-substituted by C₁-C₆ alkyl group(s) optionally substituted by group(s) seleceted from the group consisting of halogen atoms, hydroxyl groups, amino groups, carboxyl groups, carbamoyl groups and C₁-C₆ alkoxycarbonyl groups); or
R¹³ and R²³ combine together with the nitrogen atom to which they bind to form a 5- or 6-membered nitrogen containing heterocyclic group which may further contain 1 to 3 heteroatoms selected from the group consisting of nitrogen atoms, oxygen atoms and sulfur atoms (the heterocyclic group may have on the ring C₁-C₆ alkyl group(s) optionally substituted by groups selected from the group consisting of amino groups, hydroxyl groups, carboxyl groups and sulfo groups); if necessary, removing the protective group(s) existing in the product to prepare a compound represented by the general formula 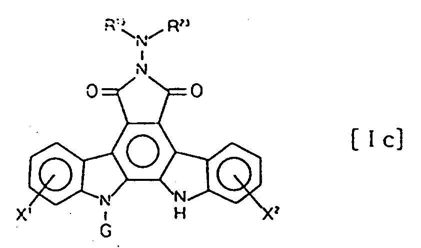 wherein R¹³, R²³, X¹, X² and G have the same meanings as defined above;
or either formylating, alkylating, alkenylating, alkynylating, aralkylating, carbamoylating, thiocarbamoylating, alkanoylating or sulfonylating the amino group 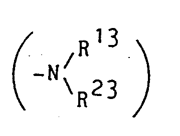 of the compound of the above formula [Ic] or the derivative thereof wherein the functional groups are protected when R¹³ and R²³ represent a hydrogen atom, or condensing the above compound or derivative with a compound represented by the following formula or a derivative thereof wherein a functional group is protected
OHC-R⁶ [V]
wherein R⁶ represents a hydrogen atom or carboxyl group, or a C₁-C₆ alkyl group optionally having 1 to 4 substituents selected from the group consisting of amino groups, mono-C₁-C₆ alkylamino groups, di-C₁-C₆ alkylamino groups, hydroxyl groups, carboxyl groups and sulfo groups,
and if necessary, removing the protective groups existing in the product; or reducing the double bonds of the compound of the above formula [Ic] when R¹³ and/or R²³ contain a double bond, or the compound prepared by condensing the compound of the formula [Ic] with the compound of the formula [V] or the derivative thereof wherein the functional groups are protected, and if necessary removing the protective group(s) existing in the product; and if necessary, converting the resulting compound of the formula [I] into a pharmaceutically acceptable salt.

2. A process according to claim 1 wherein a compound represented by the formula 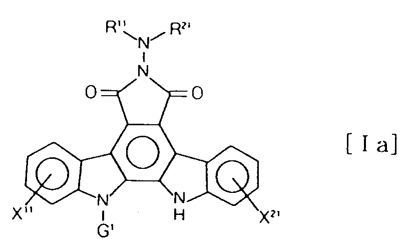 wherein
R¹¹ and R²¹ each independently represent a hydrogen atom, C₁-C₆ alkyl group, C₁-C₆ alkenyl group, aromatic hydrocarbon group having 6 to 12 carbon atoms, aralkyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group, thiazolidinyl group (the C₁-C₆ alkyl group, C₁-C₆ alkenyl group, aromatic hydrocarbon group having 6 to 12 carbon atoms, aralkyl group and hetercyclic group may have 1 to 5 substituents selected from the group consisting of carboxyl groups, carbamoyl groups, cyano groups and hydroxyl groups), or a group of the formula -Y-R³¹, and therein Y represents a carbonyl group, thiocarbonyl group or sulfonyl group, and R³¹ represents a hydrogen atom, C₁-C₆ alkyl group, aromatic hydrocarbon group having 6 to 12 carbon atoms (the C₁-C₆ alkyl group and aromatic hydrocarbon having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyrrolyl group, oxazolyl group, isoxazolyl group, thiazolyl group, imidazolyl group, pyridyl group, pyrimidinyl group, oxazolinyl group, oxazolidinyl group, imidazolinyl group, imidazolidinyl group, pyrrolidinyl group, piperazinyl group, thiazinyl group or thiazolidinyl group; or
R¹¹ and R²¹ combine to represent a C₁-C₆ alkylidene group optionally having carboxyl group(s), or
R¹¹ and R²¹ combine together with the nitrogen atom to which they bind to form a pyrrolidinyl group, imidazolidinyl group, imidazolinyl group, piperidino group or piperazinyl group (these heterocyclic groups may have on the ring C₁-C₆ alkyl group(s) optionally substituted by hydroxyl group(s)),
G¹ represents a group of the formula 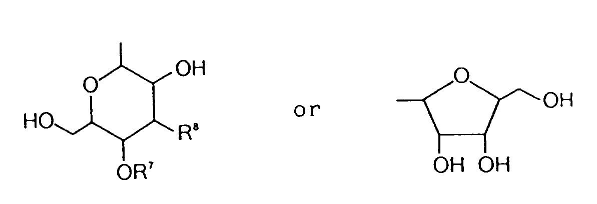 and therein R⁷ represents a hydrogen atom or C₁-C₆ alkyl group and R⁸ represents a hydroxyl group or amino group, and
X¹¹ and X²¹ bind to the indolopyrrolocarbazole ring at the 1- or 2-position and at the 10- or 11-position, respectively, and each independently represent a halogen atom, hydroxyl group, C₁-C₆ alkoxy group or aralkoxy group is prepared.

3. A process according to claim 1 wherein a compound represented by the formula 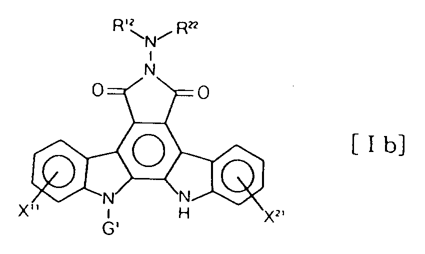 wherein
R¹² represents a hydrogen atom or C₁-C₆ alkyl group,
R²² represents a hydrogen atom, C₁-C₆ alkyl group (the C₁-C₆ alkyl group may have 1 to 5 substituents selected from the group consisting of carboxyl groups, carbamoyl groups, hydroxyl groups and cyano groups), aromatic hydrocarbon group having 6 to 12 carbon atoms, aralkyl group (the aromatic hydrocarbon group having 6 to 12 carbon atoms and aralkyl group may have 1 to 4 substituents selected from the group consisting of hydroxyl groups and carboxyl groups), pyridyl group, imidazolyl group, imidazolinyl group, thiazolyl group, pyrrolidinyl group, piperazinyl group, or a group of the formula -Y-R³², and therein Y represents a carbonyl group, thiocarbonyl group or sulfonyl group, and when Y is a carbonyl group or thicarbonyl group, R³² represents a hydrogen atom, C₁-C₆ alkyl group, aromatic hydrocarbon group having 6 to 12 carbon atoms (the C₁-C₆ alkyl group and aromatic hydrocarbon group having 6 to 12 carbon atoms may have 1 to 4 substituents selected from the group consisting of halogen atoms, optionally protected hydroxyl groups, amino groups and carboxyl groups), amino group, hydrazino group, arylamino group, C₁-C₆ alkoxy group, carbamoyl group, pyridyl group, pyrimidinyl group, imidazolinyl group or pyrrolidinyl group, and when Y is a sulfonyl group, R³² represents a C₁-C₆ alkylg group or aromatic hydrocarbon group having 6 to 12 carbon atoms; or
R¹² and R²² combine to represent a C₁-C₆ alkylidene group having carboxyl group(s); or
R¹² and R²² combine together with the nitrogen atom to which they bind to form a pyrrolidinyl group, piperidino group or piperazinyl group (these heterocyclic groups may have on the ring C₁-C₆ alkyl groups optionally having hydroxyl group(s)), and
G¹, X¹¹ and X²¹ have the same meanings as defined in claim 2 is prepared.

4. A process for the preparation of a compound represented by the following general formula and a derivative thereof wherein the functional groups are protected 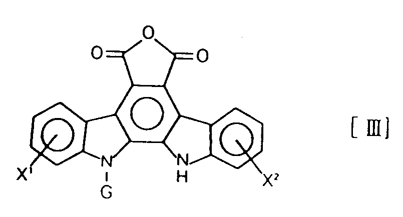 wherein X¹, X² and G have the same meanings as in claim 1 which comprises treating with a base a compound represented by the following general formula or a derivative thereof wherein the functional groups are protected 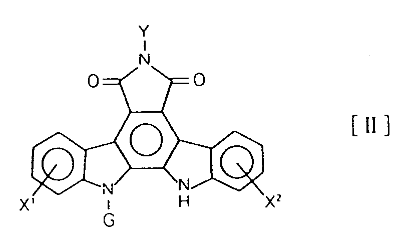 wherein Y represents a hydrogen atom or substituted or unsubstituted C₁-C₆ alkyl group, and X¹, X² and G have the same meanings as defined in claim 1.

## Patentansprüche (Patentansprüche für folgende(n) Vertragsstaat(en): AT, BE, CH, LI, DE, DK, FR, GB GR, IE, IT, LU, MC, NL, PT, SE)

1. Verbindung der folgenden allgemeinen Formel und das pharmazeutisch annehmbare Salz davon: 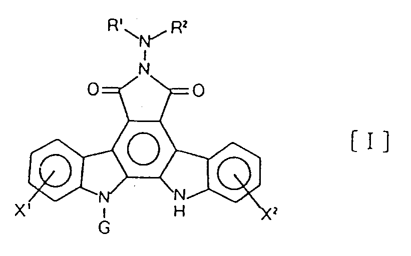 worin
R¹ und R² je unabhängig ein Wasserstoffatom, eine C₁-C₆-Alkylgruppe, eine C₁-C₆-Alkenylgruppe, eine C₁-C₆-Alkinylgruppe, eine aromatische Kohlenwasserstoffgruppe mit 6 bis 12 Kohlenstoffatomen, eine Aralkylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält (wobei die C₁-C₆-Alkylgruppe, die C₁-C₆-Alkenylgruppe, die C₁-C₆-Alkinylgruppe, die aromatische Kohlenwasserstoffgruppe mit 6 bis 12 Kohlenstoffatomen, die Aralkylgruppe und die heterocyclische Gruppe je 1 bis 5 Substituenten, ausgewählt aus der Gruppe bestehend aus Carboxylgruppen, Carbamoylgruppen, Sulfogruppen, Aminogruppen, Cyanogruppen, Mono-C₁-C₆-alkylaminogruppen, Di-C₁-C₆-alkylaminogruppen, Hydroxylgruppen und Halogenatomen, enthalten kann), oder eine Gruppe der Formel -Y-R³ bedeuten, worin Y eine Carbonylgruppe, eine Thiocarbonylgruppe oder eine Sulfonylgruppe bedeutet und R³ ein Wasserstoffatom, eine C₁-C₆-Alkylgruppe, eine Cycloalkylgruppe, eine Cycloalkylalkylgruppe, eine aromatische Kohlenwasserstoffgruppe mit 6 bis 12 Kohlenstoffatomen, eine Aralkylgruppe, eine C₁-C₆-Alkoxygruppe, eine Hydrazinogruppe, eine Aminogruppe, eine Arylaminogruppe, eine Carbamoylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, bedeutet (wobei die C₁-C₆-Alkylgruppe, die Cycloalkylgruppe, die Cycloalkylalkylgruppe, die aromatische Kohlenwasserstoffgruppe mit 6 bis 12 Kohlenstoffatomen, die Aralkylgruppe und die 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, je 1 bis 4 Substituenten, ausgewählt aus der Gruppe bestehend aus Halogenatomen, gegebenenfalls geschützten Hydroxylgruppen, Aminogruppen, Carboxylgruppen, Carbamoylgruppen, Cyanogruppen und C₁-C₆-Alkoxycarbonylgruppen, aufweisen kann und wobei die Aminogruppe und die Carbamoylgruppe je durch eine oder mehrere C₁-C₆-Alkylgruppe(n), gegebenenfalls substituiert durch einen oder mehrere Substituenten, ausgewählt aus der Gruppe bestehend aus Halogenatomen, Hydroxylgruppen, Aminogruppen, Carboxylgruppen, Carbamoylgruppen und C₁-C₆-Alkoxycarbonylgruppen, mono- oder disubstituiert sein kann); oder
R¹ und R² gemeinsam eine C₁-C₆-Alkylidengruppe bedeuten (die C₁-C₆-Alkylidengruppe kann 1 bis 4 Substituenten enthalten, ausgewählt aus der Gruppe bestehend aus Aminogruppen, Mono-C₁-C₆-alkylaminogruppen, Di-C₁-C₆-alkylaminogruppen, Hydroxylgruppen, Carboxylgruppen und Sulfonylgruppen); oder
R¹ und R² gemeinsam mit dem Stickstoffatom, an das sie gebunden sind, eine 5- oder 6gliedrige, Stickstoff-enthaltende heterocyclische Gruppe bilden, die weiter 1 bis 3 Heteroatome, ausgewählt aus der Gruppe bestehend aus Stickstoffatomen, Sauerstoffatomen und Schwefelatomen, enthalten kann (wobei die heterocyclische Gruppe an dem Ring eine oder mehrere C₁-C₆-Alkylgruppe(n), gegebenenfalls substituiert durch eine oder mehrere Gruppe(n), ausgewählt aus der Gruppe bestehend aus Aminogruppen, Hydroxylgruppen, Carboxylgruppen und Sulfogruppen, enthalten kann);
G eine Pentosegruppe oder Hexosegruppe bedeutet; und
X¹ und X² je unabhängig ein Wasserstoffatom, ein Halogenatom, eine Aminogruppe, eine Mono-C₁-C₆-alkylaminogruppe, eine Di-C₁-C₆-alkylaminogruppe, eine Hydroxylgruppe, eine C₁-C₆-Alkoxygruppe, eine Aralkoxygruppe, eine Carboxylgruppe, eine C₁-C₆-Alkoxycarbonylgruppe oder eine C₁-C₆-Alkylgruppe bedeuten.

2. Verbindung nach Anspruch 1, die eine Verbindung ist, welche durch die Formel: 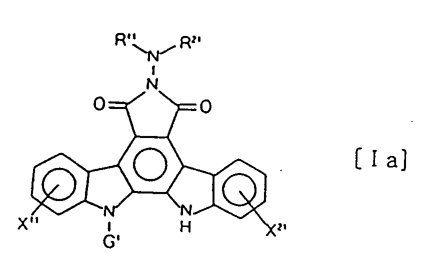 dargestellt wird, worin
R¹¹ und R²¹ je unabhängig ein Wasserstoffatom, eine C₁-C₆-Alkylgruppe, eine C₁-C₆-Alkenylgruppe, eine aromatische Kohlenwasserstoffgruppe mit 6 bis 12 Kohlenstoffatomen, eine Aralkylgruppe, eine Pyrrolylgruppe, eine Oxazolylgruppe, eine Isoxazolylgruppe, eine Thiazolylgruppe, eine Imidazolylgruppe, eine Pyridylgruppe, eine Pyrimidinylgruppe, eine Oxazolinylgruppe, eine Oxazolidinylgruppe, eine Imidazolinylgruppe, eine Imidazolidinylgruppe, eine Pyrrolidinylgruppe, eine Piperazinylgruppe, eine Thiazinylgruppe, eine Thiazolidinylgruppe (wobei die C₁-C₆-Alkylgruppe, die C₁-C₆-Alkenylgruppe, die aromatische Kohlenwasserstoffgruppe mit 6 bis 12 Kohlenstoffatomen, die Aralkylgruppe und die heterocyclische Gruppe 1 bis 5 Substituenten, ausgewählt aus der Gruppe bestehend aus Carboxylgruppen, Carbamoylgruppen, Cyanogruppen und Hydroxylgruppen, aufweisen kann) oder eine Gruppe der Formel -Y-R³¹ bedeuten, worin Y eine Carbonylgruppe, eine Thiocarbonylgruppe oder eine Sulfonylgruppe bedeutet und R³¹ ein Wasserstoffatom, eine C₁-C₆-Alkylgruppe, eine aromatische Kohlenwasserstoffgruppe mit 6 bis 12 Kohlenstoffatomen (wobei die C₁-C₆-Alkylgruppe und die aromatische Kohlenwasserstoffgruppe mit 6 bis 12 Kohlenstoffatomen 1 bis 4 Substituenten, ausgewählt aus der Gruppe bestehend aus Halogenatomen, gegebenenfalls geschützten Hydroxylgruppen, Aminogruppen und Carboxylgruppen, enthalten kann), eine Aminogruppe, Hydrazinogruppe, Arylaminogruppe, C₁-C₆-Alkoxygruppe, Carbamoylgruppe, Pyrrolylgruppe, Oxazolylgruppe, Isoxazolylgruppe, Thiazolylgruppe, Imidazolylgruppe, Pyridylgruppe, Pyrimidinylgruppe, Oxazolinylgruppe, Oxazolidinylgruppe, Imidazolinylgruppe, Imidazolidinylgruppe, Pyrrolidinylgruppe, Piperazinylgruppe, Thiazinylgruppe oder Thiazolidinylgruppe bedeutet; oder
R¹¹ und R²¹ gemeinsam eine C₁-C₆-Alkylidengruppe, die gegebenenfalls eine oder mehrere Carboxylgruppe(n) enthält, bilden; oder
R¹¹ und R²¹ zusammen mit dem Stickstoffatom, an das sie gebunden sind, eine Pyrrolidinylgruppe, Imidazolidinylgruppe, Imidazolinylgruppe, Piperidinogruppe oder Piperazinylgruppe bilden (diese heterocyclischen Gruppen können an dem Ring eine oder mehrere C₁-C₆-Alkylgruppe(n), gegebenenfalls substituiert durch eine oder mehrere Hydroxylgruppe(n), aufweisen);
G¹ eine Gruppe der Formel: 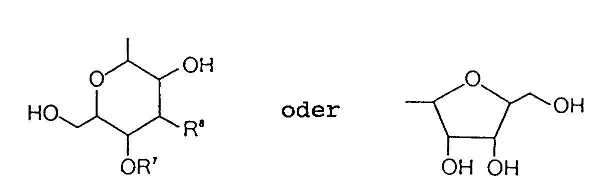 bedeutet, worin R⁷ ein Wasserstoffatom oder eine C₁-C₆-Alkylgruppe bedeutet und R⁸ eine Hydroxylgruppe oder eine Aminogruppe bedeutet; und
X¹¹ und X²¹ an den Indolopyrrolocarbazol-Ring in der 1- oder 2-Stellung bzw. in der 10- oder 11-Stellung gebunden sind und je unabhängig ein Halogenatom, eine Hydroxylgruppe, eine C₁-C₆-Alkoxygruppe oder eine Aralkoxygruppe bedeuten.

3. Verbindung nach Anspruch 1, die eine Verbindung ist, welche durch die Formel: 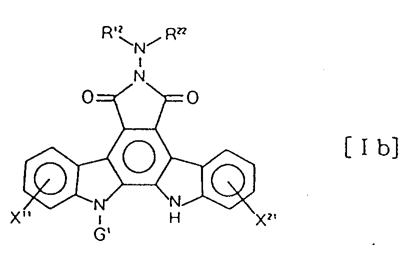 dargestellt wird, worin
R¹² ein Wasserstoffatom oder eine C₁-C₆-Alkylgruppe bedeutet;
R²² ein Wasserstoffatom, eine C₁-C₆-Alkylgruppe (die C₁-C₆-Alkylgruppe kann 1 bis 5 Substituenten, ausgewählt aus der Gruppe bestehend aus Carboxylgruppen, Carbamoylgruppen, Hydroxylgruppen und Cyanogruppen, enthalten), eine aromatische Kohlenwasserstoffgruppe mit 6 bis 12 Kohlenstoffatomen, eine Aralkylgruppe (die aromatische Kohlenwasserstoffgruppe mit 6 bis 12 Kohlenstoffatomen und die Aralkylgruppe können 1 bis 4 Substituenten, ausgewählt aus der Gruppe bestehend aus Hydroxylgruppen und Carboxylgruppen, enthalten), eine Pyridylgruppe, eine Imidazolylgruppe, eine Imidazolinylgruppe, eine Thiazolylgruppe, eine Pyrrolidinylgruppe, eine Piperazinylgruppe oder eine Gruppe der Formel -Y-R³² bedeuten, worin Y eine Carbonylgruppe, eine Thiocarbonylgruppe oder eine Sulfonylgruppe bedeutet und, wenn Y eine Carbonylgruppe oder Thiocarbonylgruppe bedeutet, R³² ein Wasserstoffatom, eine C₁-C₆-Alkylgruppe, eine aromatische Kohlenwasserstoffgruppe mit 6 bis 12 Kohlenstoffatomen (wobei die C₁-C₆-Alkylgruppe und die aromatische Kohlenwasserstoffgruppe mit 6 bis 12 Kohlenstoffatomen 1 bis 4 Substituenten, ausgewählt aus der Gruppe bestehend aus Halogenatomen, gegebenenfalls geschützten Hydroxylgruppen, Aminogruppen und Carboxylgruppen, aufweisen kann), eine Aminogruppe, eine Hydrazinogruppe, eine Arylaminogruppe, eine C₁-C₆-Alkoxygruppe, eine Carbamoylgruppe, eine Pyridylgruppe, eine Pyrimidinylgruppe, eine Imidazolinylgruppe oder eine Pyrrolidinylgruppe bedeutet und, wenn Y eine Sulfonylgruppe bedeutet, R³² eine C₁-C₆-Alkylgruppe oder eine aromatische Kohlenwasserstoffgruppe mit 6 bis 12 Kohlenstoffatomen bedeutet; oder
R¹² und R²² gemeinsam eine C₁-C₆-Alkylidengruppe mit einer oder mehreren Carboxylgruppe(n) bedeuten; oder
R¹² und R²² zusammen mit dem Stickstoffatom, an das sie gebunden sind, eine Pyrrolidinylgruppe, eine Piperidinogruppe oder eine Piperazinylgruppe bilden (diese heterocyclischen Gruppen können an dem Ring eine oder mehrere C₁-C₆-Alkylgruppe(n), gegebenenfalls mit einer oder mehreren Hydroxylgruppe(n), aufweisen); und
G¹, X¹¹ und X²¹ die gleichen Bedeutungen, wie in Anspruch 2 angegeben, besitzen.

4. Verbindung der allgemeinen Formel [I] nach Anspruch 1 oder ein pharmazeutisch annehmbares Salz davon in Form einer pharmazeutisch annehmbaren Substanz.

5. Verfahren zur Herstellung einer Verbindung der allgemeinen Formel [I] nach Anspruch 1 oder eines pharmazeutisch annehmbaren Salzes davon, dadurch **gekennzeichnet, daß** eine Verbindung der folgenden Formel oder ein Derivat davon, bei dem die funktionellen Gruppen geschützt sind: 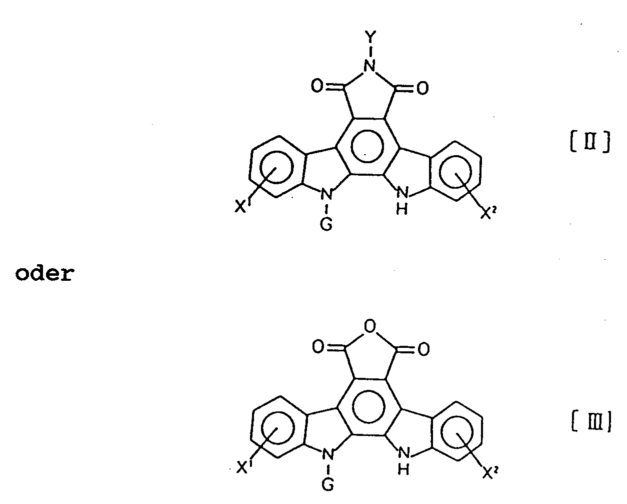 worin
Y ein Wasserstoffatom oder eine substituierte oder unsubstituierte C₁-C₆-Alkylgruppe bedeutet und X¹, X² und G die gleichen Bedeutungen, wie in Anspruch 1 angegeben, besitzen, mit einer Verbindung, die durch die folgende allgemeine Formel dargestellt wird, oder einem Derivat davon, worin R¹³ und R²³ eine funktionelle Gruppe enthalten, wobei die funktionellen Gruppen je geschützt sind, umgesetzt wird: 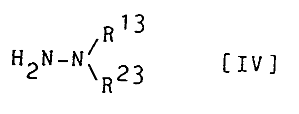 worin
R¹³ und R²³ je unabhängig ein Wasserstoffatom, eine C₁-C₆-Alkylgruppe, eine C₁-C₆-Alkenylgruppe, eine C₁-C₆-Alkinylgruppe, eine aromatische Kohlenwasserstoffgruppe mit 6 bis 12 Kohlenstoffatomen, eine Aralkylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthalten kann (wobei die C₁-C₆-Alkylgruppe, die C₁-C₆-Alkenylgruppe, die C₁-C₆-Alkinylgruppe, die aromatische Kohlenwasserstoffgruppe mit 6 bis 12 Kohlenstoffatomen, die Aralkylgruppe und die 5- oder 6gliedrige heterocyclische Gruppe, welche 1 bis 4 Heteroatome, ausgewählt aus der Gruppe bestehend aus Stickstoffatomen, Sauerstoffatomen und Schwefelatomen, 1 bis 5 Substituenten, ausgewählt aus der Gruppe bestehend aus Carboxylgruppen, Carbamoylgruppen, Sulfogruppen, Aminogruppen, Cyanogruppen, Mono-C₁-C₆-alkylaminogruppen, Di-C₁-C₆-alkylaminogruppen, Hydroxylgruppen und Halogenatomen, enthalten kann), oder eine Gruppe der Formel -Y-R³ bedeuten, worin Y eine Carbonylgruppe, eine Thiocarbonylgruppe oder eine Sulfonylgruppe bedeutet und R³ ein Wasserstoffatom, eine C₁-C₆-Alkylgruppe, eine Cycloalkylgruppe, eine Cycloalkylalkylgruppe, eine aromatische Kohlenwasserstoffgruppe mit 6 bis 12 Kohlenstoffatomen, eine Aralkylgruppe, eine C₁-C₆-Alkoxygruppe, eine Hydrazinogruppe, eine Aminogruppe, eine Arylaminogruppe, eine Carbamoylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, bedeutet (wobei die C₁-C₆-Alkylgruppe, die Cycloalkylgruppe, die Cycloalkylalkylgruppe, die aromatische Kohlenwasserstoffgruppe mit 6 bis 12 Kohlenstoffatomen, die Aralkylgruppe und die 5- oder 6gliedrige heterocyclische Gruppe je 1 bis 4 Substituenten, ausgewählt aus der Gruppe bestehend aus Halogenatomen, gegebenenfalls geschützten Hydroxylgruppen, Aminogruppen, Carboxylgruppen, Carbamoylgruppen, Cyanogruppen und C₁-C₆-Alkoxycarbonylgruppen, aufweisen kann und wobei die Aminogruppe und die Carbamoylgruppe je durch eine oder mehrere C₁-C₆-Alkylgruppe(n), gegebenenfalls substituiert durch eine oder mehrere Gruppe(n), ausgewählt aus der Gruppe bestehend aus Halogenatomen, Hydroxylgruppen, Aminogruppen, Carboxylgruppen, Carbamoylgruppen und C₁-C₆-Alkoxycarbonylgruppen, mono- oder disubstituiert sein kann); oder
R¹³ und R²³ gemeinsam mit dem Stickstoffatom, an das sie gebunden sind, eine 5- oder 6gliedrige, Stickstoff-enthaltende heterocyclische Gruppe bilden, die weiter 1 bis 3 Heteroatome, ausgewählt aus der Gruppe bestehend aus Stickstoffatomen, Sauerstoffatomen und Schwefelatomen, enthalten kann (wobei die heterocyclische Gruppe an dem Ring eine oder mehrere C₁-C₆-Alkylgruppe(n), gegebenenfalls substituiert durch eine oder mehrere Gruppe(n), ausgewählt aus der Gruppe bestehend aus Aminogruppen, Hydroxylgruppen, Carboxylgruppen und Sulfogruppen, aufweisen kann);
sofern erforderlich, die Schutzgruppe(n), die in dem Produkt vorhanden ist/sind, unter Bildung einer Verbindung, welche durch die allgemeine Formel: 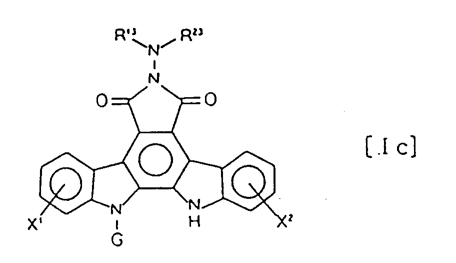 dargestellt wird, worin R¹³, R²³, X¹, X² und G die oben gegebenen Bedeutungen besitzen, entfernt; oder
entweder die Aminogruppe 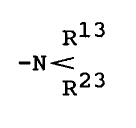 der Verbindung der obigen Formel [Ic] oder des Derivats davon, worin die funktionellen Gruppen geschützt sind, wenn R¹³ und R²³ ein Wasserstoffatom bedeuten, formyliert, alkyliert, alkenyliert, alkinyliert, aralkyliert, carbamoyliert, thiocarbamoyliert, alkanoyliert oder sulfonyliert; oder
die obige Verbindung oder ein Derivat davon mit einer Verbindung der folgenden Formel oder einem Derivat davon, worin die funktionellen Gruppen geschützt sind, kondensiert:
OHC-R⁶ [V]
worin R⁶ ein Wasserstoffatom oder eine Carboxylgruppe oder eine C₁-C₆-Alkylgruppe, die gegebenenfalls 1 bis 4 Substituenten, ausgewählt aus der Gruppe bestehend aus Aminogruppen, Mono-C₁-C₆-alkylaminogruppen, Di-C₁-C₆-alkylaminogruppen, Hydroxylgruppen, Carboxylgruppen und Sulfogruppen, aufweisen kann, bedeutet;
und, sofern erforderlich, die in dem Produkt vorhandenen Schutzgruppen entfernt; oder die Doppelbindungen der Verbindung der obigen Formel [Ic], wenn R¹³ und/oder R²³ eine Doppelbindung enthalten, oder der Verbindung, die durch Kondensation der Verbindung der Formel [Ic] mit einer Verbindung der Formel [V] oder dem Derivat davon erhalten worden ist, wobei die funktionellen Gruppen geschützt sind, reduziert und, sofern erforderlich, eine oder mehrere Schutzgruppe (n), die in dem Produkt vorhanden ist/sind, entfernt; und, sofern erforderlich, die entstehende Verbindung der Formel [I] in ein pharmazeutisch annehmbares Salz überführt.

6. Verbindung, dargestellt durch die folgende allgemeine Formel, und ein Derivat davon, worin die funktionellen Gruppen geschützt sind: 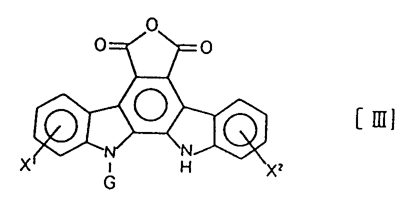 worin X¹, X² und G die gleichen Bedeutungen, wie in Anspruch 1 angegeben, besitzen.

7. Verfahren zur Herstellung einer Verbindung der allgemeinen Formel [III], wie in Anspruch 6 angegeben, dadurch **gekennzeichnet,** daß eine Verbindung, die durch die folgende allgemeine Formel dargestellt wird, oder ein Derivat davon, bei dem die funktionellen Gruppen geschützt sind: 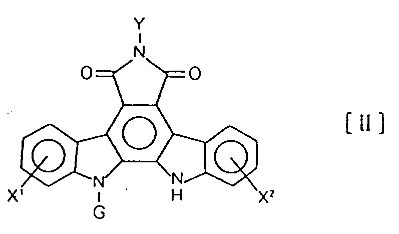 worin Y ein Wasserstoffatom oder eine substituierte oder unsubstituierte C₁-C₆-Alkylgruppe bedeutet und X¹, X² und G die gleichen Bedeutungen, wie in Anspruch 1 angegeben, besitzen, mit einer Base behandelt wird.

8. Arzneimittel, enthaltend eine Verbindung der allgemeinen Formel [I] nach Anspruch 1 oder ein pharmazeutisch annehmbares Salz davon.

9. Antitumormittel, enthaltend eine Verbindung der allgemeinen Formel [I] nach Anspruch 1 oder ein pharmazeutisch annehmbares Salz davon.

10. Pharnazeutisches Präparat, enthaltend eine wirksame Menge einer Verbindung der allgemeinen Formel [I] nach Anspruch 1 oder ein pharmazeutisch annehmbares Salz davon und einen pharmazeutisch annehmbaren Träger oder ein Verdünnungsmittel.

11. Verbindung der allgemeinen Formel [I] nach Anspruch 1 oder ein pharmazeutisch annehmbares Salz davon für die Verwendung bei einem Verfahren zur Behandlung von Krebs.

12. Verwendung einer Verbindung der allgemeinen Formel [I] nach Anspruch 1 oder eines pharmazeutisch annehmbaren Salzes davon bei der Kontrolle oder Prophylaxe einer Krankheit.

13. Verwendung einer Verbindung der alllgemeinen Formel [I] nach Anspruch 1 oder eines pharmazeutisch annehmbaren Salzes davon für die Herstellung eines aktiven Antitumor-Arzneimittels.

14. Verbindung der folgenden Formel:

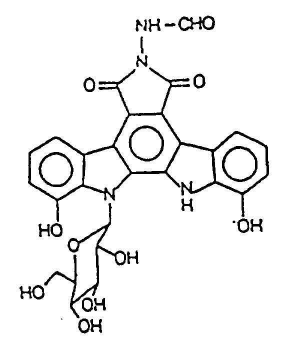

15. Verbindung der folgenden Formel:

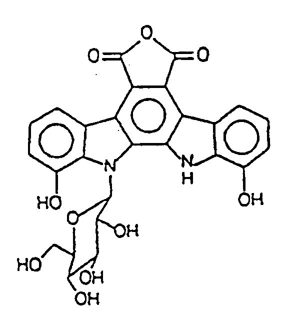

16. Verfahren nach Anspruch 5, dadurch **gekennzeichnet,** daß eine Verbindung der Formel: 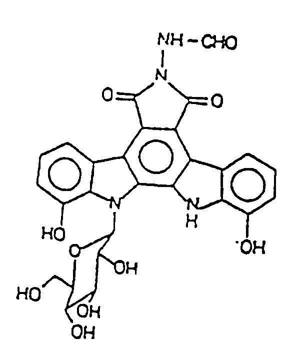 hergestellt wird.

17. Verfahren nach Anspruch 7, dadurch **gekennzeichnet,** daß eine Verbindung der Formel: 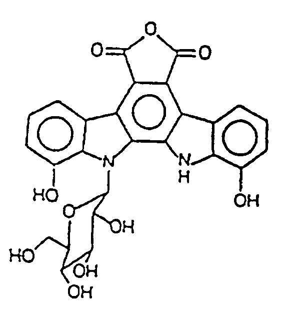 hergestellt wird.

## Patentansprüche (Patentansprüche für folgende(n) Vertragsstaat(en): ES)

1. Verfahren zur Herstellung einer Verbindung der folgenden allgemeinen Formel und eines pharmazeutisch annehmbaren Salzes davon: 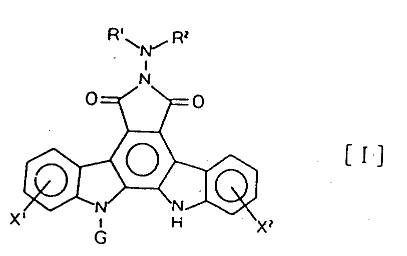 worin
R¹ und R² je unabhängig ein Wasserstoffatom, eine C₁-C₆-Alkylgruppe, eine C₁-C₆-Alkenylgruppe, eine C₁-C₆-Alkinylgruppe, eine aromatische Kohlenwasserstoffgruppe mit 6 bis 12 Kohlenstoffatomen, eine Aralkylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält (wobei die C₁-C₆-Alkylgruppe, die C₁-C₆-Alkenylgruppe, die C₁-C₆-Alkinylgruppe, die aromatische Kohlenwasserstoffgruppe mit 6 bis 12 Kohlenstoffatomen, die Aralkylgruppe und die heterocyclische Gruppe je 1 bis 5 Substituenten, ausgewählt aus der Gruppe bestehend aus Carboxylgruppen, Carbamoylgruppen, Sulfogruppen, Aminogruppen, Cyanogruppen, Mono-C₁-C₆-alkylaminogruppen, Di-C₁-C₆-alkylaminogruppen, Hydroxylgruppen und Halogenatomen, enthalten kann), oder eine Gruppe der Formel -Y-R³ bedeuten, worin Y eine Carbonylgruppe, eine Thiocarbonylgruppe oder eine Sulfonylgruppe bedeutet und R³ ein Wasserstoffatom, eine C₁-C₆-Alkylgruppe, eine Cycloalkylgruppe, eine Cycloalkylalkylgruppe, eine aromatische Kohlenwasserstoffgruppe mit 6 bis 12 Kohlenstoffatomen, eine Aralkylgruppe, eine C₁-C₆-Alkoxygruppe, eine Hydrazinogruppe, eine Aminogruppe, eine Arylaminogruppe, eine Carbamoylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, bedeutet (wobei die C₁-C₆-Alkylgruppe, die Cycloalkylgruppe, die Cycloalkylalkylgruppe, die aromatische Kohlenwasserstoffgruppe mit 6 bis 12 Kohlenstoffatomen, die Aralkylgruppe und die 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, je 1 bis 4 Substituenten, ausgewählt aus der Gruppe bestehend aus Halogenatomen, gegebenenfalls geschützten Hydroxylgruppen, Aminogruppen, Carboxylgruppen, Carbamoylgruppen, Cyanogruppen und C₁-C₆-Alkoxycarbonylgruppen, aufweisen kann und wobei die Aminogruppe und die Carbamoylgruppe je durch eine oder mehrere C₁-C₆-Alkylgruppe(n), gegebenenfalls substituiert durch einen oder mehrere Substituenten, ausgewählt aus der Gruppe bestehend aus Halogenatomen, Hydroxylgruppen, Aminogruppen, Carboxylgruppen, Carbamoylgruppen und C₁-C₆-Alkoxycarbonylgruppen, mono- oder disubstituiert sein kann); oder
R¹ und R² gemeinsam eine C₁-C₆-Alkylidengruppe bedeuten (die C₁-C₆-Alkylidengruppe kann 1 bis 4 Substituenten enthalten, ausgewählt aus der Gruppe bestehend aus Aminogruppen, Mono-C₁-C₆-alkylaminogruppen, Di-C₁-C₆-alkylaminogruppen, Hydroxylgruppen, Carboxylgruppen und Sulfonylgruppen); oder
R¹ und R² gemeinsam mit dem Stickstoffatom, an das sie gebunden sind, eine 5- oder 6gliedrige, Stickstoff-enthaltende heterocyclische Gruppe bilden, die weiter 1 bis 3 Heteroatome, ausgewählt aus der Gruppe bestehend aus Stickstoffatomen, Sauerstoffatomen und Schwefelatomen, enthalten kann (wobei die heterocyclische Gruppe an dem Ring eine oder mehrere C₁-C₆-Alkylgruppe(n), gegebenenfalls substituiert durch eine oder mehrere Gruppe(n), ausgewählt aus der Gruppe bestehend aus Aminogruppen, Hydroxylgruppen, Carboxylgruppen und Sulfogruppen, enthalten kann);
G eine Pentosegruppe oder Hexosegruppe bedeutet; und
X¹ und X² je unabhängig ein Wasserstoffatom, ein Halogenatom, eine Aminogruppe, eine Mono-C₁-C₆-alkylaminogruppe, eine Di-C₁-C₆-alkylaminogruppe, eine Hydroxylgruppe, eine C₁-C₆-Alkoxygruppe, eine Aralkoxygruppe, eine Carboxylgruppe, eine C₁-C₆-Alkoxycarbonylgruppe oder eine C₁-C₆-Alkylgruppe bedeuten,
dadurch **gekennzeichnet,** daß eine Verbindung der folgenden Formel oder ein Derivat davon, bei dem die funktionellen Gruppen geschützt sind: 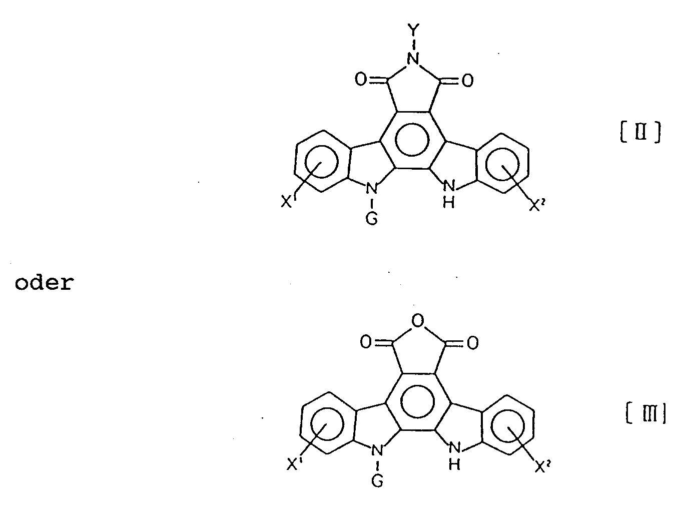 worin
Y ein Wasserstoffatom oder eine substituierte oder unsubstituierte C₁-C₆-Alkylgruppe bedeutet und X¹, X² und G die gleichen Bedeutungen, wie in Anspruch 1 angegeben, besitzen, mit einer Verbindung, die durch die folgende allgemeine Formel dargestellt wird, oder einem Derivat davon, worin R¹³ und R²³ eine funktionelle Gruppe enthalten, wobei die funktionellen Gruppen je geschützt sind, umgesetzt wird: 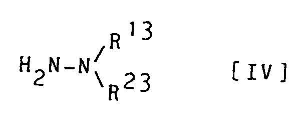 worin
R¹³ und R²³ je unabhängig ein Wasserstoffatom, eine C₁-C₆-Alkylgruppe, eine C₁-C₆-Alkenylgruppe, eine C₁-C₆-Alkinylgruppe, eine aromatische Kohlenwasserstoffgruppe mit 6 bis 12 Kohlenstoffatomen, eine Aralkylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthalten kann (wobei die C₁-C₆-Alkylgruppe, die C₁-C₆-Alkenylgruppe, die C₁-C₆-Alkinylgruppe, die aromatische Kohlenwasserstoffgruppe mit 6 bis 12 Kohlenstoffatomen, die Aralkylgruppe und die 5- oder 6gliedrige heterocyclische Gruppe, welche 1 bis 4 Heteroatome, ausgewählt aus der Gruppe bestehend aus Stickstoffatomen, Sauerstoffatomen und Schwefelatomen, 1 bis 5 Substituenten, ausgewählt aus der Gruppe bestehend aus Carboxylgruppen, Carbamoylgruppen, Sulfogruppen, Aminogruppen, Cyanogruppen, Mono-C₁-C₆-alkylaminogruppen, Di-C₁-C₆-alkylaminogruppen, Hydroxylgruppen und Halogenatomen, enthalten kann), oder eine Gruppe der Formel -Y-R³ bedeuten, worin Y eine Carbonylgruppe, eine Thiocarbonylgruppe oder eine Sulfonylgruppe bedeutet und R³ ein Wasserstoffatom, eine C₁-C₆-Alkylgruppe, eine Cycloalkylgruppe, eine Cycloalkylalkylgruppe, eine aromatische Kohlenwasserstoffgruppe mit 6 bis 12 Kohlenstoffatomen, eine Aralkylgruppe, eine C₁-C₆-Alkoxygruppe, eine Hydrazinogruppe, eine Aminogruppe, eine Arylaminogruppe, eine Carbamoylgruppe oder eine 5- oder 6gliedrige heterocyclische Gruppe, die 1 bis 4 Heteroatome, ausgewählt aus der Gruppe bestehend aus Stickstoffatomen, Sauerstoffatomen und Schwefelatomen, enthält, bedeutet (wobei die C₁-C₆-Alkylgruppe, die Cycloalkylgruppe, die Cycloalkylalkylgruppe, die aromatische Kohlenwasserstoffgruppe mit 6 bis 12 Kohlenstoffatomen, die Aralkylgruppe heterocyclische Gruppe je 1 bis 4 Substituenten, ausgewählt aus der Gruppe bestehend aus Halogenatomen, gegebenenfalls geschützten Hydroxylgruppen, Aminogruppen, Carboxylgruppen, Carbamoylgruppen, Cyanogruppen und C₁-C₆-Alkoxycarbonylgruppen, aufweisen kann und wobei die Aminogruppe und die Carbamoylgruppe je durch eine oder mehrere C₁-C₆-Alkylgruppe(n), gegebenenfalls substituiert durch eine oder mehrere Gruppe(n), ausgewählt aus der Gruppe bestehend aus Halogenatomen, Hydroxylgruppen, Aminogruppen, Carboxylgruppen, Carbamoylgruppen und C₁-C₆-Alkoxycarbonylgruppen, mono- oder disubstituiert sein kann); oder
R¹³ und R²³ gemeinsam mit dem Stickstoffatom, an das sie gebunden sind, eine 5- oder 6gliedrige, Stickstoff-enthaltende heterocyclische Gruppe bilden, die weiter 1 bis 3 Heteroatome, ausgewählt aus der Gruppe bestehend aus Stickstoffatomen, Sauerstoffatomen und Schwefelatomen, enthalten kann (wobei die heterocyclische Gruppe an dem Ring eine oder mehrere C₁-C₆-Alkylgruppe(n), gegebenenfalls substituiert durch eine oder mehrere Gruppe(n), ausgewählt aus der Gruppe bestehend aus Aminogruppen, Hydroxylgruppen, Carboxylgruppen und Sulfogruppen, aufweisen kann);
sofern erforderlich, die Schutzgruppe(n), die in dem Produkt vorhanden ist/sind, unter Bildung einer Verbindung, welche durch die allgemeine Formel: 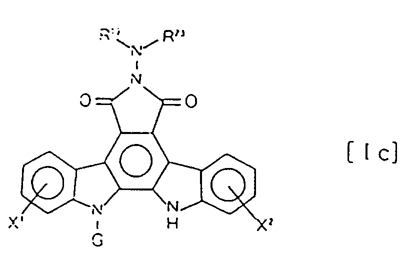 dargestellt wird, worin R¹³, R²³, X¹, X² und G die oben gegebenen Bedeutungen besitzen, entfernt; oder
entweder die Aminogruppe 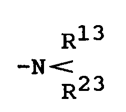 der Verbindung der obigen Formel [Ic] oder des Derivats davon, worin die funktionellen Gruppen geschützt sind, wenn R¹³ und R²³ ein Wasserstoffatom bedeuten, formyliert, alkyliert, alkenyliert, alkinyliert, aralkyliert, carbamoyliert, thiocarbamoyliert, alkanoyliert oder sulfonyliert; oder
die obige Verbindung oder ein Derivat davon mit einer Verbindung der folgenden Formel oder einem Derivat davon, worin die funktionellen Gruppen geschützt sind, kondensiert:
OHC-R⁶ [V]
worin R⁶ ein Wasserstoffatom oder eine Carboxylgruppe oder eine C₁-C₆-Alkylgruppe, die gegebenenfalls 1 bis 4 Substituenten, ausgewählt aus der Gruppe bestehend aus Aminogruppen, Mono-C₁-C₆-alkylaminogruppen, Di-C₁-C₆-alkylaminogruppen, Hydroxylgruppen, Carboxylgruppen und Sulfogruppen, aufweisen kann, bedeutet;
und, sofern erforderlich, die in dem Produkt vorhandenen Schutzgruppen entfernt; oder die Doppelbindungen der Verbindung der obigen Formel [Ic], wenn R¹³ und/oder R²³ eine Doppelbindung enthalten, oder der Verbindung, die durch Kondensation der Verbindung der Formel [Ic] mit einer Verbindung der Formel [V] oder dem Derivat davon erhalten worden ist, wobei die funktionellen Gruppen geschützt sind, reduziert und, sofern erforderlich, eine oder mehrere Schutzgruppe(n), die in dem Produkt vorhanden ist/sind, entfernt; und, sofern erforderlich, die entstehende Verbindung der Formel [I] in ein pharmazeutisch annehmbares Salz überführt.

2. Verfahren nach Anspruch 1, dadurch **gekennzeichnet,** daß eine Verbindung, die durch die Formel: 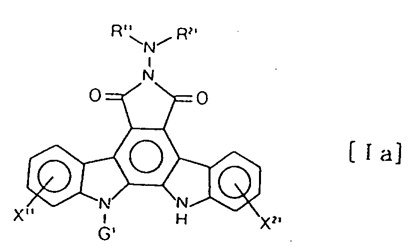 dargestellt wird, worin
R¹¹ und R²¹ je unabhängig ein Wasserstoffatom, eine C₁-C₆-Alkylgruppe, eine C₁-C₆-Alkenylgruppe, eine aromatische Kohlenwasserstoffgruppe mit 6 bis 12 Kohlenstoffatomen, eine Aralkylgruppe, eine Pyrrolylgruppe, eine Oxazolylgruppe, eine Isoxazolylgruppe, eine Thiazolylgruppe, eine Imidazolylgruppe, eine Pyridylgruppe, eine Pyrimidinylgruppe, eine Oxazolinylgruppe, eine Oxazolidinylgruppe, eine Imidazolinylgruppe, eine Imidazolidinylgruppe, eine Pyrrolidinylgruppe, eine Piperazinylgruppe, eine Thiazinylgruppe, eine Thiazolidinylgruppe (wobei die C₁-C₆-Alkylgruppe, die C₁-C₆-Alkenylgruppe, die aromatische Kohlenwasserstoffgruppe mit 6 bis 12 Kohlenstoffatomen, die Aralkylgruppe und die heterocyclische Gruppe 1 bis 5 Substituenten, ausgewählt aus der Gruppe bestehend aus Carboxylgruppen, Carbamoylgruppen, Cyanogruppen und Hydroxylgruppen, aufweisen kann) oder eine Gruppe der Formel -Y-R³¹ bedeuten, worin Y eine Carbonylgruppe, eine Thiocarbonylgruppe oder eine Sulfonylgruppe bedeutet und R³¹ ein Wasserstoffatom, eine C₁-C₆-Alkylgruppe, eine aromatische Kohlenwasserstoffgruppe mit 6 bis 12 Kohlenstoffatomen (wobei die C₁-C₆-Alkylgruppe und die aromatische Kohlenwasserstoffgruppe mit 6 bis 12 Kohlenstoffatomen 1 bis 4 Substituenten, ausgewählt aus der Gruppe bestehend aus Halogenatomen, gegebenenfalls geschützten Hydroxylgruppen, Aminogruppen und Carboxylgruppen, enthalten kann), eine Aminogruppe, Hydrazinogruppe, Arylaminogruppe, C₁-C₆-Alkoxygruppe, Carbamoylgruppe, Pyrrolylgruppe, Oxazolylgruppe, Isoxazolylgruppe, Thiazolylgruppe, Imidazolylgruppe, Pyridylgruppe, Pyrimidinylgruppe, Oxazolinylgruppe, Oxazolidinylgruppe, Imidazolinylgruppe, Imidazolidinylgruppe, Pyrrolidinylgruppe, Piperazinylgruppe, Thiazinylgruppe oder Thiazolidinylgruppe bedeutet; oder
R¹¹ und R²¹ gemeinsam eine C₁-C₆-Alkylidengruppe, die gegebenenfalls eine oder mehrere Carboxylgruppe(n) enthält, bilden; oder
R¹¹ und R²¹ zusammen mit dem Stickstoffatom, an das sie
gebunden sind, eine Pyrrolidinylgruppe, Imidazolidinylgruppe, Imidazolinylgruppe, Piperidinogruppe oder Piperazinylgruppe bilden (diese heterocyclischen Gruppen können an dem Ring eine oder mehrere C₁-C₆-Alkylgruppe(n), gegebenenfalls substituiert durch eine oder mehrere Hydroxylgruppe(n), aufweisen);
G¹ eine Gruppe der Formel: 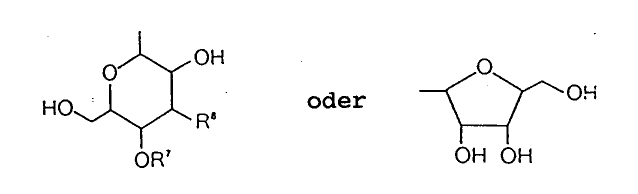 bedeutet, worin R⁷ ein Wasserstoffatom oder eine C₁-C₆-Alkylgruppe bedeutet und R⁸ eine Hydroxylgruppe oder eine Aminogruppe bedeutet; und
X¹¹ und X²¹ an den Indolopyrrolocarbazol-Ring in der 1- oder 2-Stellung bzw. in der 10- oder 11-Stellung gebunden sind und je unabhängig ein Halogenatom, eine Hydroxylgruppe, eine C₁-C₆-Alkoxygruppe oder eine Aralkoxygruppe bedeuten, hergestellt wird.

3. Verfahren nach Anspruch 1, dadurch **gekennzeichnet,** daß eine Verbindung, die durch die Formel: 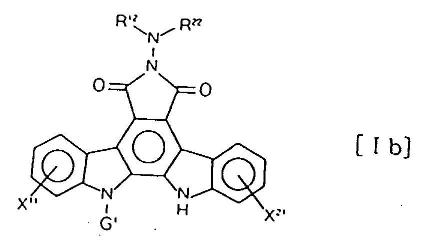 dargestellt wird, worin
R¹² ein Wasserstoffatom oder eine C₁-C₆-Alkylgruppe bedeutet;
R²² ein Wasserstoffatom, eine C₁-C₆-Alkylgruppe (die C₁-C₆-Alkylgruppe kann 1 bis 5 Substituenten, ausgewählt aus der Gruppe bestehend aus Carboxylgruppen, Carbamoylgruppen, Hydroxylgruppen und Cyanogruppen, enthalten), eine aromatische Kohlenwasserstoffgruppe mit 6 bis 12 Kohlenstoffatomen, eine Aralkylgruppe (die aromatische Kohlenwasserstoffgruppe mit 6 bis 12 Kohlenstoffatomen und die Aralkylgruppe können 1 bis 4 Substituenten, ausgewählt aus der Gruppe bestehend aus Hydroxylgruppen und Carboxylgruppen, enthalten), eine Pyridylgruppe, eine Imidazolylgruppe, eine Imidazolinylgruppe, eine Thiazolylgruppe, eine Pyrrolidinylgruppe, eine Piperazinylgruppe oder eine Gruppe der Formel -Y-R³² bedeuten, worin Y eine Carbonylgruppe, eine Thiocarbonylgruppe oder eine Sulfonylgruppe bedeutet und, wenn Y eine Carbonylgruppe oder Thiocarbonylgruppe bedeutet, R³² ein Wasserstoffatom, eine C₁-C₆-Alkylgruppe, eine aromatische Kohlenwasserstoffgruppe mit 6 bis 12 Kohlenstoffatomen (wobei die C₁-C₆-Alkylgruppe und die aromatische Kohlenwasserstoffgruppe mit 6 bis 12 Kohlenstoffatomen 1 bis 4 Substituenten, ausgewählt aus der Gruppe bestehend aus Halogenatomen, gegebenenfalls geschützten Hydroxylgruppen, Aminogruppen und Carboxylgruppen, aufweisen kann), eine Aminogruppe, eine Hydrazinogruppe, eine Arylaminogruppe, eine C₁-C₆-Alkoxygruppe, eine Carbamoylgruppe, eine Pyridylgruppe, eine Pyrimidinylgruppe, eine Imidazolinylgruppe oder eine Pyrrolidinylgruppe bedeutet und, wenn Y eine Sulfonylgruppe bedeutet, R³² eine C₁-C₆-Alkylgruppe oder eine aromatische Kohlenwasserstoffgruppe mit 6 bis 12 Kohlenstoffatomen bedeutet; oder
R¹² und R²² gemeinsam eine C₁-C₆-Alkylidengruppe mit einer oder mehreren Carboxylgruppe(n) bedeuten; oder
R¹² und R²² zusammen mit dem Stickstoffatom, an das sie gebunden sind, eine Pyrrolidinylgruppe, eine Piperidinogruppe oder eine Piperazinylgruppe bilden (diese heterocyclischen Gruppen können an dem Ring eine oder mehrere C₁-C₆-Alkylgruppe(n), gegebenenfalls mit einer oder mehreren Hydroxylgruppe(n), aufweisen); und
G¹, X¹¹ und X²¹ die gleichen Bedeutungen, wie in Anspruch 2 angegeben, besitzen, hergestellt wird.

4. Verfahren zur Herstellung einer Verbindung, dargestellt durch die folgende allgemeine Formel, oder eines Derivats davon, worin die funktionellen Gruppen geschützt sind: 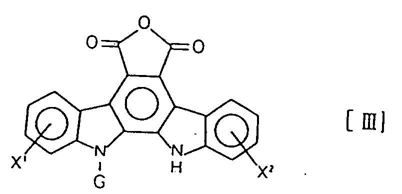 worin X¹, X² und G die gleichen Bedeutungen, wie in Anspruch 1 angegeben, besitzen, dadurch **gekennzeichnet,** daß eine Verbindung der folgenden allgemeinen Formel oder ein Derivat davon, in dem die funktionellen Gruppen geschützt sind: 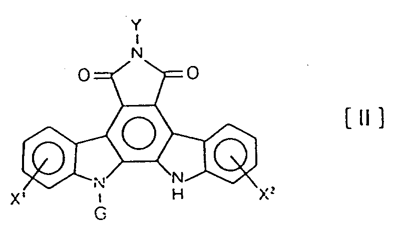 worin Y ein Wasserstoffatom oder eine substituierte oder unsubstituierte C₁-C₆-Alkylgruppe bedeutet und X¹, X² und G die gleichen Bedeutungen, wie in Anspruch 1 angegeben, besitzen, mit einer Base behandelt wird.

## Revendications (Revendications pour l'(les) Etat(s) contractant(s) suivant(s): AT, BE, CH, LI, DE, DK, FR, GB, GR, IE, IT, LU, MC, NL, PT, SE)

1. Composé représenté par la formule générale suivante et un sel pharmaceutiquement acceptable de celui-ci 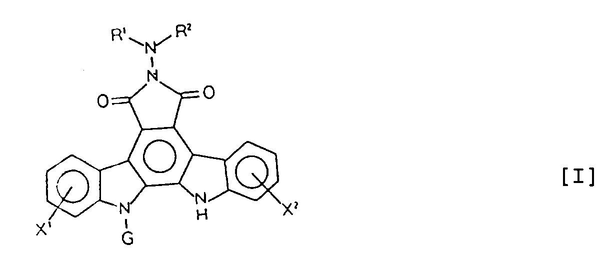 dans laquelle
R¹ et R² chacun, indépendamment l'un de l'autre, représentent un atome d'hydrogène, un groupe alkyle en C₁-C₆, un groupe alcényle en C₁-C₆, un groupe alcynyle en C₁-C₆, un groupe hydrocarboné aromatique ayant de 6 à 12 atomes de carbone, un groupe aralkyle ou un groupe hétérocyclique à 5 ou 6 chaînons contenant de 1 à 4 hétéroatomes choisis dans le groupe comportant des atomes d'azote, des atomes d'oxygène et des atomes de soufre (le groupe alkyle en C₁-C₆, le groupe alcényle en C₁-C₆, le groupe alcynyle en C₁-C₆, le groupe aromatique hydrocarboné ayant de 6 à 12 atomes de carbone, le groupe aralkyle et le groupe hétérocyclique pouvant porter chacun de 1 à 5 substituants pris parmi les groupes carboxyle, les groupes carbamoyle, les groupes sulfo, les groupes amino, les groupes cyano, les groupes mono(alkyl en C₁-C₆)amino, les groupes di(alkyl en C₁-C₆)amino, les groupes hydroxyle et les atomes d'halogènes), ou un groupe de formule -Y-R³ dans laquelle Y représente un groupe carbonyle, un groupe thiocarbonyle ou un groupe sulfonyle et R³ représente un atome d'hydrogène, un groupe alkyle en C₁-C₆, un groupe cycloalkyle, un groupe cycloalkylalkyle, un groupe hydrocarboné aromatique ayant de 6 à 12 atomes de carbone, un groupe aralkyle, un groupe alcoxy en C₁-C₆, un groupe hydrazino, un groupe amino, un groupe arylamino, un groupe carbamoyle ou un groupe hétérocyclique à 5 ou 6 chaînons contenant de 1 à 4 hétéroatomes pris dans le groupe comportant des atomes d'azote, des atomes d'oxygène et des atomes de soufre (le groupe alkyle en C₁-C₆, le groupe cycloalkyle, le groupe cycloalkylalkyle, le groupe hydrocarboné aromatique ayant de 6 à 12 atomes de carbone, le groupe aralkyle et le groupe hétérocyclique à 5 ou 6 chaînons contenant de 1 à 4 hétéroatomes pris dans le groupe comportant des atomes d'azote, des atomes d'oxygène et des atomes de soufre, chacun pouvant avoir de 1 à 4 substituants pris dans le groupe comprenant des atomes d'halogènes, des groupes alcoxycarbonyle en C₁-C₆, des groupes cyano, des groupes carbamoyle, des groupes carboxyle, des groupes amino et des groupes hydroxyle éventuellement protégés, et le groupe amino et le groupe carbamoyle pouvant être chacun mono- ou disubstitués par un (des) groupe(s) alkyle en C₁-C₆ éventuellement substitué(s) par des substituants pris dans le groupe comportant des atomes d'halogènes, des groupes hydroxyle, des groupes amino, des groupes carboxyle, des groupes carbamoyle et des groupes alcoxycarbonyle en C₁-C₆) ; ou
R¹ et R² se combinent pour représenter un groupe alkylidène en C₁-C₆ (le groupe alkylidène en C₁-C₆ peut avoir de 1 à 4 substituants pris dans le groupe comportant des groupes amino, des groupes mono(alkyl en C₁-C₆)-amino, des groupes di(alkyl en C₁-C₆)-amino, des groupes hydroxyle, des groupes carboxyle et des groupes sulfonyle) ; ou
R¹ et R² se combinent ensemble avec l'atome d'azote auquel ils sont liés pour former un groupe hétérocyclique azoté à 5 ou 6 chaînons qui peut contenir de plus de 1 à 3 hétéroatomes pris dans le groupe comportant des atomes d'azote, des atomes d'oxygène et des atomes de soufre (le groupe hétérocyclique peut porter sur le cycle un (des) groupe(s) alkyle en C₁-C₆ éventuellement substitué par un (des) groupe(s) pris dans le groupe comportant des groupes amino, des groupes hydroxyle, des groupes carboxyle et des groupes sulfo),
G représente un groupe pentose ou un groupe hexose, et
X¹ et X² chacun, indépendamment l'un de l'autre, représente un atome d'hydrogène, un atome d'halogène, un groupe amino, un groupe mono(alkyl an C₁-C₆)-amino, un groupe di(alkyl an C₁-C₆)-amino, un groupe hydroxyle, un groupe alcoxy an C₁-C₆, un groupe aralcoxy, un groupe carboxyle, un groupe (alcoxy an C₁-C₆)carbonyle ou un groupe alkyle en C₁-C₆.

2. Composé selon la revendication 1, lequel composé est représenté par la formule 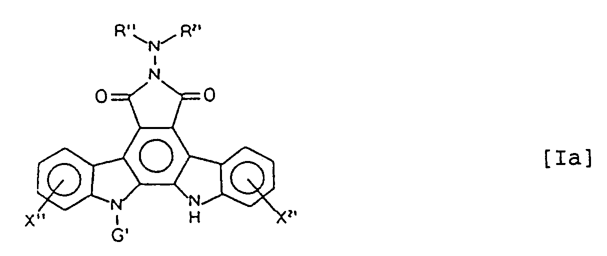 dans laquelle
R¹¹ et R²¹ chacun, indépendamment l'un de l'autre, représente un atome d'hydrogène, un groupe alkyle an C₁-C₆, un groupe alcényle an C₁-C₆, un groupe hydrocarboné aromatique ayant de 6 à 12 atomes de carbone, un groupe aralkyle, un groupe pyrrolyle, un groupe oxazolyle, un groupe isoxazolyle, un groupe thiazolyle, un groupe imidazolyle, un groupe pyridyle, un groupe pyrimidinyle, un groupe oxazolinyle, un groupe oxazolidinyle, un groue imidazolinyle, un groupe imidazolidinyle, un groupe pyrrolidinyle, un groupe pipérazinyle, un groupe thiazinyle, un groupe thiazolidinyle (le groupe alkyle en C₁-C₆, le groupe alcényle an C₁-C₆, le groupe hydrocarboné aromatique ayant de 6 à 12 atomes de carbone, le groupe aralkyle et le groupe hétérocyclique pouvant avoir de 1 à 5 substituants choisis dans le groupe comportant des groupes carboxyle, des groupes carbamoyle, des groupes cyano et des groupes hydroxyle), ou un groupe de formule -Y-R³¹, et dans laquelle Y représente un groupe carbonyle, un groupe thiocarbonyle ou un groupe sulfonyle, et R³¹ représente un atome d'hydrogène, un groupe alkyle en C₁-C₆, un groupe hydrocarboné aromatique ayant de 6 à 12 atomes de carbone (le groupe alkyle en C₁-C₆, et le groupe hydrocarboné aromatique ayant de 6 à 12 atomes de carbone pouvant avoir de 1 à 4 substituants choisis dans le groupe comportant des atomes d'halogènes, des groupes carboxyle, des groupes amino et des groupes hydroxyle éventuellement protégés), un groupe amino, un groupe hydrazino, un groupe arylamino, un groupe alcoxy en C₁-C₆, un groupe carbamoyle, un groupe pyrrolyle, un groupe oxazolyle, un groupe isoxazolyle, un groupe thiazolyle, un groupe imidazolyle, un groupe pyridyle, un groupe pyrimidinyle, un groupe oxazolinyle, un groupe oxazolidinyle, un groupe imidazolinyle, un groupe imidazolidinyle, un groupe pyrrolidinyle, un groupe pipérazinyle, un groupe thiazinyle ou un groupe thiazolidinyle ; ou
R¹¹ et R²¹ se combinent pour représenter un groupe alkylidène en C₁-C₆, ayant éventuellement un (des) groupe(s) carboxyle, ou
R¹¹ et R²¹ se combinant ensemble avec l'atome d'azote auquel ils sont liés pour former un groupe pyrrolidinyle, un groupe imidazolidinyle, un groupe imidazolinyle, un groupe pipéridino ou un groupe pipérazinyle (ces groupes hétérocycliques pouvant porter sur le cycle un (des) groupe(s) alkyle en C₁-C₆ éventuellement substitué(s) par un (des) groupe(s) hydroxyle,
G¹ représente un groupe de formule 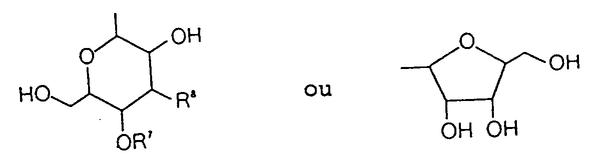 et dans ces formules R⁷ représente un atome d'hydrogène ou un groupa alkyle en C₁-C₆ et R⁸ représente un groupe hydroxyle ou un groupe amino, et X¹¹ et X²¹ se lient au cycle indolopyrrocarbazole respectivement en positions 1 ou 2 et en positions 10 ou 11, et chacun indépendamment l'un de l'autre représente un atome d'halogène, un groupe hydroxyle, un groupe alcoxy en C₁-C₆ ou un groupe aralcoxy.

3. Composé selon la revendication 1 représenté par la formule 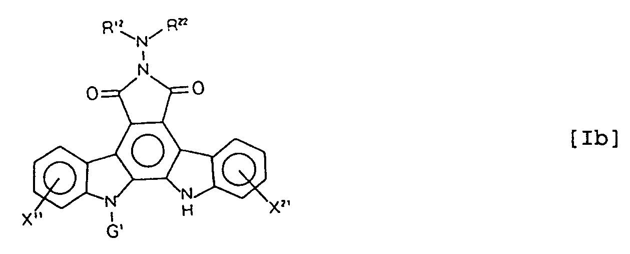 dans laquelle
R¹² représente un atome d'hydrogène ou un groupe alkyle en C₁-C₆,
R²² représente un atome d'hydrogène, un groupe alkyle en C₁-C₆ (le groupe alkyle en C₁-C₆ peut avoir de 1 à 5 substituants choisis dans un groupe comportant des groupes carboxyle, des groupes carbamoyle, des groupes hydroxyle et des groupes cyano), un groupe hydrocarboné aromatique ayant de 6 à 12 atomes de carbone, un groupe aralkyle (le groupe hydrocarboné aromatique ayant de 6 à 12 atomes de carbone et le groupe aralkyle pouvant avoir de 1 à 4 substituants choisis dans le groupe comportant des groupes hydroxyle et des groupes carboxyle), un groupe pyridyle, un groupe imidazolyle, un groupe imidazolinyle, un groupe thiazolyle, un groupe pyrrolidinyle, un groupe pipérazinyle, ou un groupe de formule -Y-R³², et dans laquelle Y représente un groupe carbonyle, un groupe thiocarbonyle ou un groupe sulfonyle, R³² représente un atome d'hydrogène, un groupe alkyle en C₁-C₆, un groupe hydrocarboné aromatique ayant de 6 à 12 atomes de carbone (le groupe alkyle en C₁-C₆ et le groupe hydrocarboné aromatique ayant de 6 à 12 atomes de carbone pouvant avoir de 1 à 4 substituants choisis dans le groupe comportant des atomes d'halogènes, des groupes carboxyle, des groupes amino et des groupes hydroxyle éventuellement protégés), un groupe amino, un groupe hydrazino, un groupe arylamino, un groupe alcoxy en C₁-C₆, un groupe carbamoyle, un groupe pyridyle, un groupe pyrimidinyle, un groupe imidazolinyle ou un groupe pyrrolidinyle, et lorsque Y est un groupe sulfonyle, R³² représente un groupe alkyle en C₁-C₆ ou un groupe hydrocarboné aromatique ayant de 6 à 12 atomes de carbone ; ou
R¹² et R²² se combinent pour représenter un groupe alkylidène en C₁-C₆ ayant un (des) groupe(s) carboxyle ; ou
R¹² et R²² se combinent ensemble avec l'atome d'azote auquel ils sont liés pour former un groupe pyrrolidinyle, un groupe pipéridino ou un groupe pipérazinyle (ces groupes hétérocycliques pouvant porter sur le cycle des groupes alkyle en C₁-C₆ ayant éventuellement un (des) groupe(s) hydroxyle), et
G¹, X¹¹ et X²¹ ont les mêmes significations que celles définies dans la revendication 2.

4. Composé de formula générale [I] selon la revendication 1 ou un de ses sels pharmaceutiquement acceptables comme substance pharmaceutiquement acceptable.

5. Procédé de préparation d'un composé de formule générale [I] selon la revendication 1 ou d'un de ses sels pharmaceutiquement acceptables qui comprend la réaction d'un composé représenté par la formule suivante ou d'un de ses dérivés dans lesquels les groupes fonctionnels sont protégés 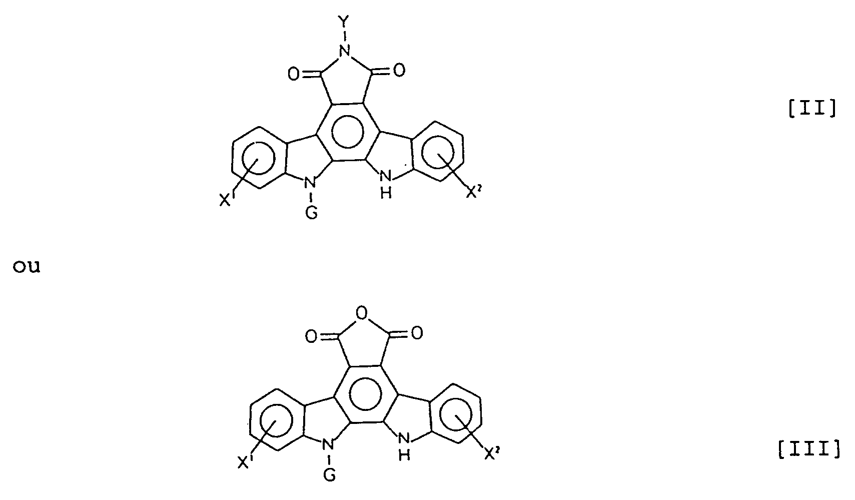 dans lesquelles
Y représente un atome d'hydrogène ou un groupe alkyle en C₁-C₆ substitué ou non substitué, et X¹, X² et G ont les mêmes significations que celles définies dans la revendication 1, avec un composé représenté par la formula générale suivante ou un de ses dérivés où, dans le cas où R¹³ et R²³ contiennent un groupe fonctionnel, chaque groupe fonctionnel est protégé 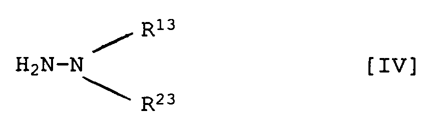 dans laquelle
R¹³ et R²³ chacun, indépendamment l'un de l'autre, représente un atome d'hydrogène, un groupe alkyle en C₁-C₆, un groupe alcényle en C₁-C₆, un groupe alcynyle en C₁-C₆, un groupe hydrocarbone aromatique ayant de 6 à 12 atomes de carbone, un groupe aralkyle ou un groupe hétérocyclique à 5 ou 6 chaînons contenant de 1 à 4 hétéroatomes choisis dans le groupe comportant des atomes d'azote, des atomes d'oxygène et des atomes de soufre (le groupe alkyle en C₁-C₆, le groupe alcényle en C₁-C₆, le groupe alcynyle en C₁-C₆, le groupe hydrocarboné aromatique ayant de 6 à 12 atomes de carbone, le groupe aralkyle et le groupe hétérocyclique à 5 ou 6 chaînons contenant de 1 à 4 hétéroatomes choisis dans le groupe comportant des atomes d'azote, des atomes d'oxygène et des atomes de soufre pouvant avoir de 1 à 5 substituants pris dans le groupe comportant des groupes carboxyle, des groupes carbamoyle, des groupes sulfo, des groupes amino, des groupes cyano, des groupes mono(alkyl en C₁-C₆)-amino, des groupes di(alkyl en C₁-C₆)-amino, des groupes hydroxyle et des atomes d'halogènes), ou un groupe de formule -Y-R³, et dans laquelle Y représente un groupe carbonyle, un groupe thiocarbonyle ou un groupe sulfonyle, et R³ représente un atome d'hydrogène, un groupa alkyle en C₁-C₆, un groupe cycloalkyle, un groupe cycloalkylalkyle, un groupe hydrocarboné aromatique ayant de 6 à 12 atomes de carbone, un groupe aralkyle, un groupe alcoxy en C₁-C₆, un groupe hydrazino, un groupe amino, un groupe arylamino, un groupe carbamoyle ou un groupe hétérocyclique à 5 ou 6 chaînons contenant de 1 à 4 hétéroatomes pris dans le groupe comprenant des atomes d'azote, des atomes d'oxygène et des atomes de soufre (le groupe alkyle en C₁-C₆, le groupe cycloalkyle, le groupe cycloalkylalkyle, le groupe hydrocarboné aromatique ayant de 6 à 12 atomes de carbone, le groupe aralkyle et le groupe hétérocyclique pouvant porter chacun de 1 à 4 substituants pris dans le groupe comportant des atomes d'halogènes, des groupes alcoxycarbonyle en C₁-C₆, des groupes cyano, des groupes carbamoyle, des groupes carboxyle, des groupes amino et des groupes hydroxyle éventuellement protégés, et le groupe amino et le groupe carbamoyle pouvant être chacun mono- ou disubstitués par un (des) groupe(s) alkyle en C₁-C₆ éventuellement substitué(s) par un (des) groupe(s) pris dans le groupe comportant des atomes d'halogènes, des groupes hydroxyle, des groupes amino, des groupes carboxyle, des groupes carbamoyle et des groupes alcoxycarbonyle en C₁-C₆) ; ou
R¹³ et R²³ se combinent ensemble avec l'atome d'azote auquel ils sont liés pour former un groupe hétérocyclique azoté à 5 ou 6 chaînons, qui peut contenir de plus de 1 à 3 hétéroatomes pris dans le groupe comportant des atomes d'azote, des atomes d'oxygène et des atomes de soufre (le groupe hétérocyclique pouvant porter sur le cycle un (des) groupe(s) alkyle en C₁-C₆ éventuellement substitué(s) par des groupes pris dans le groupe comportant des groupes amino, des groupes hydroxyle, des groupes carboxyle et des groupes sulfo) ; si nécessaire, en éliminant un (des) groupe(s) protecteur(s) présent(s) dans le produit pour préparer un composé représenté par la formule générale 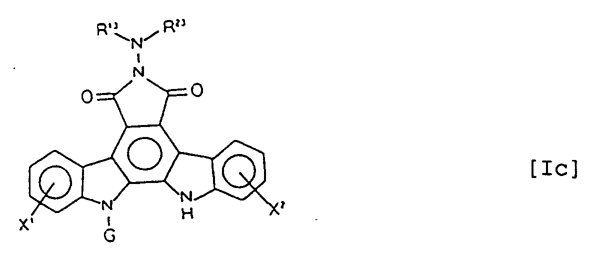 dans laquelle R¹³, R²³, X¹, X² et G ont les mêmes significations que celles définies ci-dessus ;
ou, soit par formylation, alkylation, alcénylation, alcynylation, aralkylation, carbamoylation, thiocarbamoylation, alcanoylation ou sulfonylation du groupe amino 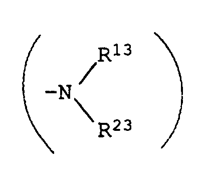 du composé de formule [Ic] ci-dessus ou de son dérivé dans lesquels les groupes fonctionnels sont protégés lorsque R¹³ et R²³ représentent un atome d'hydrogène, soit par condensation du composé ci-dessus ou dérivé, avec un composé représenté par la formule suivante ou un dérivé de celui-ci dans lesquels le groupe fonctionnel est protégé
OHC-R⁶ [V]
dans laquelle R⁶ représente un atome d'hydrogène ou un groupe carboxyle, ou un groupe alkyle en C₁-C₆ ayant éventuellement de 1 à 4 substituants choisis dans le groupe comprenant des groupes amino, des groupes mono(alkyl en C₁-C₆)-amino, des groupes di(alkyl en C₁-C₆)-amino, des groupes hydroxyle, des groupes carboxyle et des groupes sulfo ;
et si nécessaire, en éliminant des groupes protecteurs existant dans le produit ; ou en réduisant les doubles liaisons du composé de formule [Ic] ci-dessus lorsque R¹³ et/ou R²³ contiennent une double liaison, ou le composé préparé par condensation du composé de formule [Ic] avec le composé de formule [V] ou le dérivé de celui-ci, dans lesquels les groupes fonctionnels sont protégés et, si nécessaire, en éliminant le(s) groupe(s) protecteur(s) présent(s) dans le produit ; et si nécessaire, en transformant le composé résultantal de formule [I] en sel pharmaceutiquement acceptable.

6. Composé représenté par la formule générale suivante et un dérivé de celui-ci dans lesquels les groupes fonctionneles sont protégés 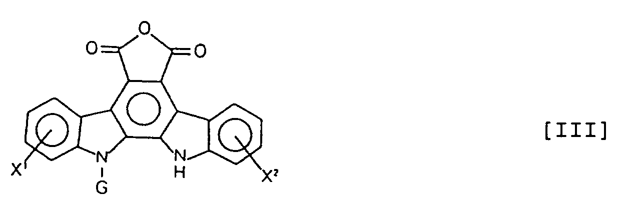 dans laquelle X¹, X² et G ont les mêmes significations que celles dans la revendication 1.

7. Procédé de préparation d'un composé de formule générale [III] selon la revendication 6 qui comprend le traitement par une base d'un composé représenté par la formule générale suivante ou d'un dérivé de celui-ci dans lesquels les groupes fonctionnels sont protégés 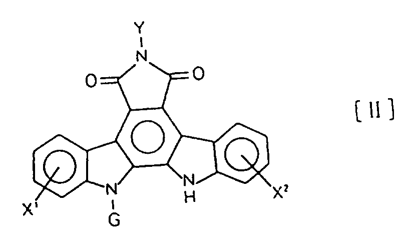 dans laquelle Y représente un atome d'hydrogène ou un groupe alkyle en C₁-C₆ substitué ou non substitué, et X¹, X² et G ont les mêmes significations que celles définies dans la revendication 1.

8. Médicament contenant un composé de formule générale [I] selon la revendication 1 ou un sel pharmaceutiquement acceptable de celui-ci.

9. Agent antitumoral contenant un composé de formule générale [I] selon la revendication 1 ou un sels pharmaceutiquement acceptable de celui-ci.

10. Préparation pharmaceutique comprenant une quantité efficace d'un composé de formule générale [I] selon la revendication 1 ou un sel pharmaceutiquement acceptable de celui-ci, et un véhicule ou diluant pharmaceutiquement acceptable.

11. Composé de formule générale [I] selon la revendication 1 ou un sel pharmaceutiquement acceptable de celui-ci pour utiliser dans un méthode de traitement du cancer.

12. Utilisation d'un composé de formule générale [I] selon la revendication 1 ou un sel pharmaceutiquement acceptable de celui-ci dans le contrôle ou la prévention de maladie.

13. Utilisation d'un composé de formule générale [I] selon la revendication 1 ou un sel pharmaceutiquement acceptable de celui-ci pour la préparation de médicamants antitumoral actif.

14. Composé représenté par la formule suivante :

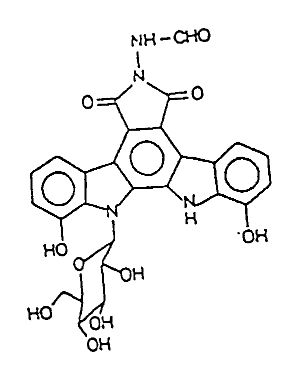

15. Composé représenté par la formule suivante :

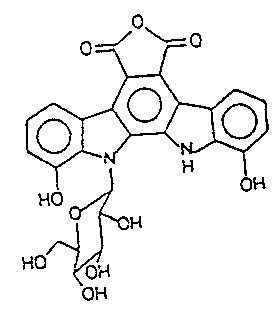

16. Procédé selon la revendication 5, dans lequel on prépare un composé représenté par la formule :

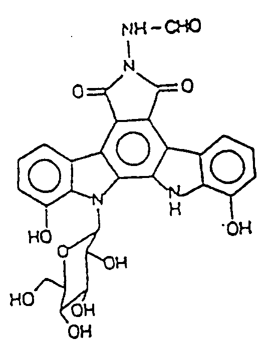

17. Procédé selon la revendication 7, dans lequel on prépare un composé représenté par la formule :

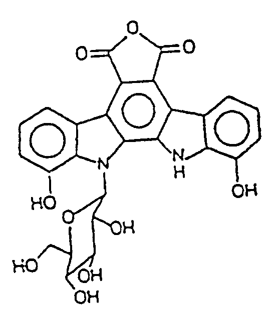

## Revendications (Revendications pour l'(les) Etat(s) contractant(s) suivant(s): ES)

1. Procédé de préparation d'un composé représenté par la formule générale suivante et d'un sel pharmaceutiquement acceptable de celui-ci 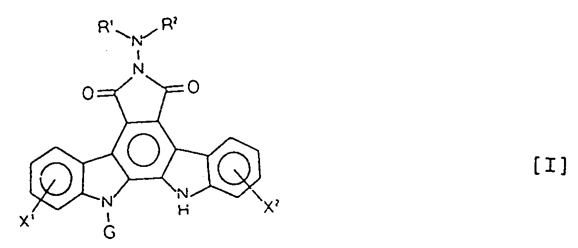 dans laquelle
R¹ et R² chacun, indépendamment l'un de l'autre, représentent un atome d'hydrogène, un groupe alkyle en C₁-C₆, un groupe alcényle en C₁-C₆, un groupe alcynyle en C₁-C₆, un groupe hydrocarboné aromatique ayant de 6 à 12 atomes de carbone, un groupe aralkyle ou un groupe hétérocyclique à 5 ou 6 chaînons contenant de 1 à 4 hétéroatomes choisis dans le groupe comportant des atomes d'azote, des atomes d'oxygène et des atomes de soufre (le groupe alkyle en C₁-C₆, le groupe alcényle en C₁-C₆, le groupe alcynyle en C₁-C₆, le groupe aromatique hydrocarboné ayant de 6 à 12 atomes de carbone, le groupe aralkyle et le groupe hétérocyclique pouvant porter chacun de 1 à 5 substituants pris parmi les groupes carboxyle, les groupes carbamoyle, les groupes sulfo, les groupes amino, les groupes cyano, les groupes mono(alkyl an C₁-C₆)amino, les groupes di(alkyl en C₁-C₆)amino, les groupes hydroxyle et les atomes d'halogènes), ou un groupe de formule -Y-R³ dans laquelle Y représente un groupe carbonyle, un groupe thiocarbonyle ou un groupe sulfonyle et R³ représente un atome d'hydrogène, un groupe alkyle en C₁-C₆, un groupe cycloalkyle, un groupe cycloalkylalkyle, un groupe hydrocarboné aromatique ayant de 6 à 12 atomes de carbone, un groupe aralkyle, un groupe alcoxy en C₁-C₆, un groupe hydrazino, un groupe amino, un groupe arylamino, un groupe carbamoyle ou un groupe hétérocyclique à 5 ou 6 chaînons contenant de 1 à 4 hétéroatomes pris dans le groupe comportant des atomes d'azote, des atomes d'oxygène et des atomes de soufre (le groupe alkyle en C₁-C₆, le groupe cycloalkyle, le groupe cycloalkylalkyle, le groupe hydrocarboné aromatique ayant de 6 à 12 atomes de carbone, le groupe aralkyle et le groupe hétérocyclique à 5 ou 6 chaînons contenant de 1 à 4 hétéroatomes pris dans le groupe comportant des atomes d'azote, des atomes d'oxygène et des atomes de soufre, chacun pouvant avoir de 1 à 4 substituants pris dans le groupe comprenant des atomes d'halogènes, des groupes alcoxycarbonyle en C₁-C₆, des groupes cyano, des groupes carbamoyle, des groupes carboxyle, des groupes amino et des groupes hydroxyle éventuellement protégés, et le groupe amino et le groupe carbamoyle pouvant être chacun mono- ou disubstitués par un (des) groupe(s) alkyle en C₁-C₆ éventuellement substitué(s) par des substituants pris dans le groupe comportant des atomes d'halogènes, des groupes hydroxyle, des groupes amino, des groupes carboxyle, des groupes carbamoyle et des groupes alcoxycarbonyle en C₁-C₆) ; ou
R¹ et R² se combinent pour représenter un groupe alkylidène en C₁-C₆ (le groupe alkylidène en C₁-C₆ pouvant avoir de 1 à 4 substituants pris dans le groupe comportant des groupes amino, des groupes mono(alkyl an C₁-C₆)-amino, des groupes di(alkyl en C₁-C₆)-amino, des groupes hydroxyle, des groupes carboxyle et des groupes sulfonyle) ; ou
R¹ et R² se combinent ensemble avec l'atome d'azote auquel ils sont liés pour former un groupe hétérocyclique azoté à 5 ou 6 chaînons qui peut contenir de plus de 1 à 3 hétéroatomes pris dans le groupe comportant des atomes d'azote, des atomes d'oxygène et des atomes de soufre (le groupe hétérocyclique pouvant porter sur le cycle un (des) groupe(s) alkyle en C₁-C₆ éventuellement substitué par un (des) groupe(s) pris dans le groupe comportant des groupes amino, des groupes hydroxyle, des groupes carboxyle et des groupes sulfo),
G représente un groupe pentose ou un groupe hexose, et
X¹ et X² chacun, indépendamment l'un de l'autre, représente un atome d'hydrogène, un atome d'halogène, un groupe amino, un groupe mono(alkyl en C₁-C₆)-amino, un groupe di(alkyl an C₁-C₆)-amino, un groupe hydroxyle, un groupe alcoxy en C₁-C₆, un groupe aralcoxy, un groupe carboxyle, un groupe (alcoxy en C₁-C₆)carbonyle ou un groupe alkyle en C₁-C₆, qui comprend la réaction d'un composé représenté par la formule suivante ou d'un dérivé de celui-ci dans lesquels les groupes fonctionnels sont protégés 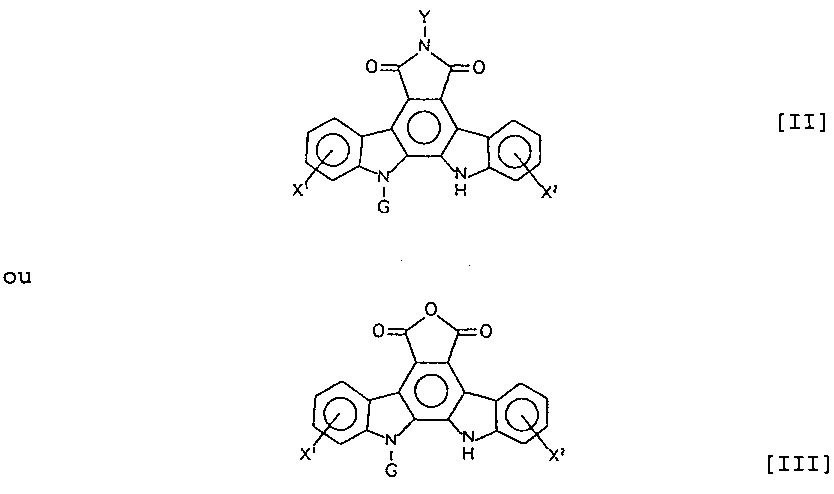 dans laquelle
Y représente un atome d'hydrogène ou un groupe alkyle an C₁-C₆ substitué ou non substitué, et X¹, X² et G ont les mêmes significations que celles définies dans la revendication 1, avec un composé représenté par la formule générale suivante ou un de ses dérivés où, dans le cas où R¹³ et R²³ contiennent un groupe fonctionnel, chaque groupe fonctionnel est protégé 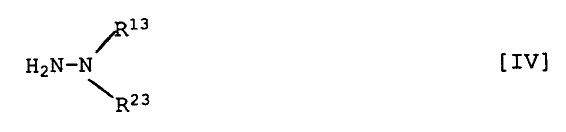 dans laquelle
R¹³ et R²³ chacun, indépendamment l'un de l'autre, représente un atome d'hydrogène, un groupe alkyle an C₁-C₆, un groupe alcényle en C₁-C₆, un groupe alcynyle an C₁-C₆, un groupe hydrocarbone aromatique ayant de 6 à 12 atomes de carbone, un groupe aralkyle ou un groupe hétérocyclique à 5 ou 6 chaînons contenant de 1 à 4 hétéroatomes choisis dans le groupe comportant des atomes d'azote, des atomes d'oxygène et des atomes de soufre (le groupe alkyle en C₁-C₆, le groupe alcényle en C₁-C₆, le groupe alcynyle en C₁-C₆, le groupe hydrocarboné aromatique ayant de 6 à 12 atomes de carbone, le groupe aralkyle et le groupe hétérocyclique à 5 ou 6 chaînons contenant de 1 à 4 hétéroatomes choisis dans le groupe comportant des atomes d'azote, des atomes d'oxygène et des atomes de soufre pouvant avoir de 1 à 5 substituants pris dans le groupe comportant des groupes carboxyle, des groupes carbamoyle, des groupes sulfo, des groupes amino, des groupes cyano, des groupes mono(alkyl en C₁-C₆)-amino, des groupes di(alkyl en C₁-C₆)-amino, des groupes hydroxyle et des atomes d'halogènes), ou un groupe de formule -Y-R³, et dans laquelle Y représente un groupe carbonyle, un groupe thiocarbonyle ou un groupe sulfonyle, et R³ représente un atome d'hydrogène, un groupe alkyle en C₁-C₆, un groupe cycloalkyle, un groupe cycloalkylalkyle, un groupe hydrocarboné aromatique ayant de 6 à 12 atomes de carbone, un groupe aralkyle, un groupe alcoxy en C₁-C₆, un groupe hydrazino, un groupe amino, un groupe arylamino, un groupe carbamoyle ou un groupe hétérocyclique à 5 ou 6 chaînons contenant de 1 à 4 hétéroatomes pris dans le groupe comprenant des atomes d'azote, des atomes d'oxygène et des atomes de soufre (le groupe alkyle en C₁-C₆, le groupe cycloalkyle, le groupe cycloalkylalkyle, le groupe hydrocarboné aromatique ayant de 6 à 12 atomes de carbone, le groupe aralkyle et le groupe hétérocyclique pouvant porter chacun de 1 à 4 substituants pris dans le groupe comportant des atomes d'halogènes, des groupes alcoxycarbonyle en C₁-C₆, des groupes cyano, des groupes carbamoyle, des groupes carboxyle, des groupes amino et des groupes hydroxyle éventuellement protégés, et le groupe amino et le groupe carbamoyle pouvant être chacun mono- ou disubstitués par un (des) groupe(s) alkyle en C₁-C₆ éventuellement substitué(s) par un (des) groupe(s) pris dans le groupe comportant des atomes d'halogènes, des groupes hydroxyle, des groupes amino, des groupes carboxyle, des groupes carbamoyle et des groupes alcoxycarbonyle en C₁-C₆) ; ou
R¹³ et R²³ se combinent ensemble avec l'atome d'azote auquel ils sont liés pour former un groupe hétérocyclique azoté à 5 ou 6 chaînons, qui peut contenir de plus de 1 à 3 hétéroatomes pris dans le groupe comportant des atomes d'azotes, des atomes d'oxygène et des atomes de soufre (le groupe hétérocyclique pouvant porter sur le cycle un (des) groupe(s) alkyle éventuellement substitué(s) par des groupes pris dans la groupe comportant des groupes amino, des groupes hydroxyle, des groupes carboxyle et des groupes sulfo) ; si nécessaire, en éliminant des groupes protecteurs existant dans le produit pour préparer un composé représenté par la formule générale 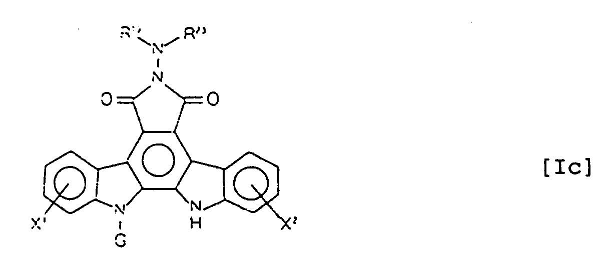 dans laquelle R¹³, R²³, X¹, X² et G ont les mêmes significations que celles définies ci-dessus ; ou soit par formylation, alkylation, alcénylation, alcynylation, aralkylation, carbamoylation, thiocarbamoylation, alcanoylation ou sulfonylation du groupe amino 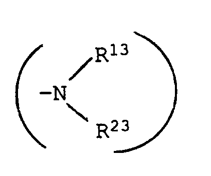 du composé de formule [Ic] ci-dessus ou de son dérivé dans lesquels les groupes fonctionnels sont protégés lorsque R¹³ et R²³ représentent un atome d'hydrogène, soit par condensation du composé ci-dessus ou dérivé, avec un composé représenté par la formule suivante ou un dérivé de celui-ci dans lesquels un groupe fonctionnel est protégé
OHC-R⁶ [V]
dans laquelle R⁶ représente un atome d'hydrogène ou un groupe carboxyle, ou un groupe alkyle en C₁-C₆ ayant éventuellement de 1 à 4 substituants choisis dans le groupe comprenant des groupes amino, des groupes mono(alkyl en C₁-C₆)-amino, des groupes di(alkyl en C₁-C₆)-amino, des groupes hydroxyle, des groupes carboxyle et des groupes sulfo ;
et si nécessaire, en éliminanr des groupes protecteurs existant dans le produit ; ou an réduisant les doubles liaisons du composé de formule [Ic] ci-dessus lorsque R¹³ et/ou R²³ contiennent une double liaison, ou le composé préparé par condensation du composé de formule [Ic] avec le composé de formule [V] ou le dérivé de celui-ci, où les groupes fonctionnels sont protégés et, si nécessaire, en éliminant le(s) groupe(s) protecteur(s) présent(s) dans le produit ; et si nécessaire, en transformant le composé résultant de formule [I] en sel pharmaceutiquement acceptable.

2. Procédé selon la revendication 1, dans lequel on prépare un composé représenté par la formule 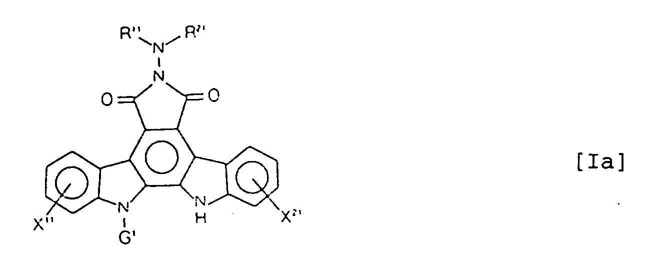 dans laquelle
R¹¹ et R²¹ chacun, indépendamment l'un de l'autre, représente un atome d'hydrogène, un groupe alkyle en C₁-C₆, un groupe alcényle en C₁-C₆, un groupe hydrocarboné aromatique ayant de 6 à 12 atomes de carbone, un groupe aralkyle, un groupe pyrrolyle, un groupe oxazolyle, un groupe isoxazolyle, un groupe thiazolyle, un groupe imidazolyle, un groupe pyridyle, un groupe pyrimidinyle, un groupe oxazolinyle, un groupe oxazolidinyle, un groue imidazolinyle, un groupe imidazolidinyle, un groupe pyrrolidinyle, un groupe pipérazinyle, un groupe thiazinyle, un groupe thiazolidinyle (le groupe alkyle en C₁-C₆, le groupe alcényle en C₁-C₆, le groupe hydrocarboné aromatique ayant de 6 à 12 atomes de carbone, le groupe aralkyle et le groupe hétérocyclique pouvant avoir de 1 à 5 substituants choisis dans le groupe comportant des groupes carboxyle, des groupes carbamoyle, des groupes cyano et des groupes hydroxyle), ou un groupe de formule -Y-R³¹, et dans laquelle Y représente un groupe carbonyle, un groupe thiocarbonyle ou un groupe sulfonyle, et R³¹ représente un atome d'hydrogène, un groupe alkyle en C₁-C₆, un groupe hydrocarboné aromatique ayant de 6 à 12 atomes de carbone (le groupe alkyle en C₁-C₆ et le groupe hydrocarboné aromatique ayant de 6 à 12 atomes de carbone pouvant avoir de 1 à 4 substituants choisis dans le groupe comportant des atomes d'halogènes, des groupes carboxyle, des groupes amino et des groupes hydroxyle éventuellement protégés), un groupe amino, un groupe hydrazino, un groupe arylamino, un groupe alcoxy en C₁-C₆, un groupe carbamoyle, un groupe pyrrolyle, un groupe oxazolyle, un groupe isoxazolyle, un groupe thiazolyle, un groupe imidazolyle, un groupe pyridyle, un groupe pyrimidinyle, un groupe oxazolinyle, un groupe oxazolidinyle, un groupe imidazolinyle, un groupe imidazolidinyle, un groupe pyrrolidinyle, un groupe pipérazinyle, un groupe thiazinyle ou un groupe thiazolidinyle ; ou
R¹¹ et R²¹ se combinent pour représenter un groupe alkylidène en C₁-C₆, ayant éventuellement un (des) groupe(s) carboxyle, ou
R¹¹ et R²¹ se combinant ensemble avec l'atome d'azote auquel ils sont liés pour former un groupe pyrrolidinyle, un groupe imidazolidinyle, un groupe imidazolinyle, un groupe pipéridino ou un groupe pipérazinyle (ces groupes hétérocycliques pouvant porter sur le cycle un (des) groupe(s) alkyle en C₁-C₆ éventuellement substitués par un (des) groupe(s) hydroxyle,
G¹ représente un groupe de formule 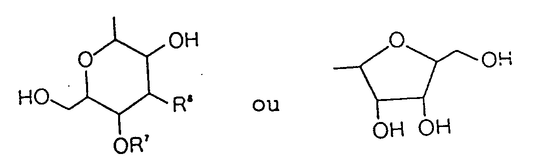 et dans ces formules R⁷ représente un atome d'hydrogène ou un groupe alkyle en C₁-C₆ et R⁸ représente un groupe hydroxyle ou un groupe amino, et
X¹¹ et X²¹ se lient au cycle indolopyrrocarbazole respectivement en positions 1 ou 2 et en positions 10 ou 11, et chacun, indépendamment l'un de l'autre, représente un atome d'halogène, un groupe hydroxyle, un groupe alcoxy en C₁-C₆ ou un groupe aralcoxy.

3. Procédé selon la revendication 1, dans lequel on prépare un composé représenté par la formule 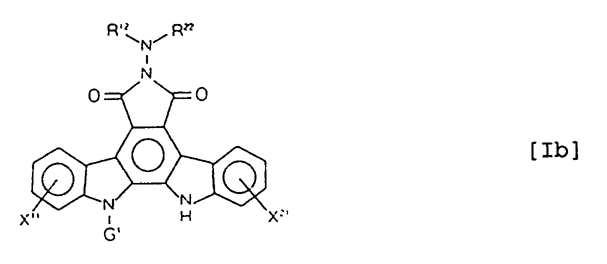 dans laquelle
R¹² représente un atome d'hydrogène ou un groupe alkyle en C₁-C₆,
R²² représente un atome d'hydrogène, un groupe alkyle en C₁-C₆ (le groupe alkyle en C₁-C₆ pouvant avoir de 1 à 5 substituants choisis dans un groupe comportant des groupes carboxyle, des groupes carbamoyle, des groupes hydroxyle et des groupes cyano), un groupe hydrocarboné aromatique ayant de 6 à 12 atomes de carbone, un groupe aralkyle (le groupe hydrocarboné aromatique ayant de 6 à 12 atomes de carbone et le groupe aralkyle pouvant avoir de 1 à 4 substituants choisis dans le groupe comportant des groupes hydroxyle et des groupes carboxyle), un groupe pyridyle, un groupe imidazolyle, un groupe imidazolinyle, un groupe thiazolyle, un groupe pyrrolidinyle, un groupe pipérazinyle, ou un groupe de formule -Y-R³², et dans laquelle Y représente un groupe carbonyle, un groupe thiocarbonyle ou un groupe sulfonyle, R³² représente un atome d'hydrogène, un groupe alkyle en C₁-C₆, un groupe hydrocarboné aromatique ayant de 6 à 12 atomes de carbone (le groupe alkyle en C₁-C₆ et le groupe hydrocarboné aromatique ayant de 6 à 12 atomes de carbone pouvant avoir de 1 à 4 substituants choisis dans le groupe comportant des atomes d'halogènes, des groupes carboxyle, des groupes amino et des groupes hydroxyle éventuellement protégés), un groupe amino, un groupe hydrazino, un groupe arylamino, un groupe alcoxy en C₁-C₆, un groupe carbamoyle, un groupe pyridyle, un groupe pyrimidinyle, un groupe imidazolinyle ou un groupe pyrrolidinyle, et lorsque Y est un groupe sulfonyle, R³² représente un groupe alkyle en C₁-C₆ ou un groupe hydrocarboné aromatique ayant de 6 à 12 atomes de carbone ; ou
R¹² et R²² se combinent pour représenter un groupe alkylidène en C₁-C₆ ayant un (des) groupe(s) carboxyle ; ou
R¹² et R²² se combinent ensemble avec l'atome d'azote auquel ils sont liés pour former un groupe pyrrolidinyle, un groupe pipéridino ou un groupe pipérazinyle (ces groupes hétérocycliques pouvant porter sur le cycle des groupes alkyle en C₁-C₆ ayant éventuellement un (des) groupe(s) hydroxyle), et
G¹, X¹¹ et X²¹ ont les mêmes significations que celles définies dans la revendication 2.

4. Procédé pour la préparation d'un composé représenté par la formule générale suivante et d'un dérivé de celui-ci dans lesquels les groupes fonctionnels sont protégés 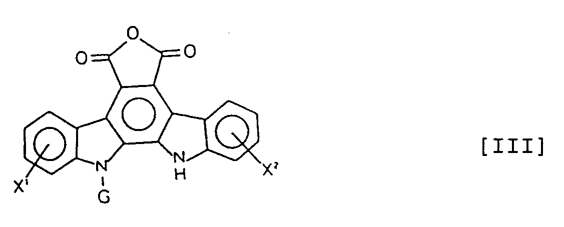 dans laquelle
X¹, X² et G ont les mêmes sigifications que dans la revendication 1, qui comprend le traitement par une base d'un composé représenté par la formule générale suivante ou d'un dérivé de celui-ci dans lesquels les groupes fonctionnels sont protégés 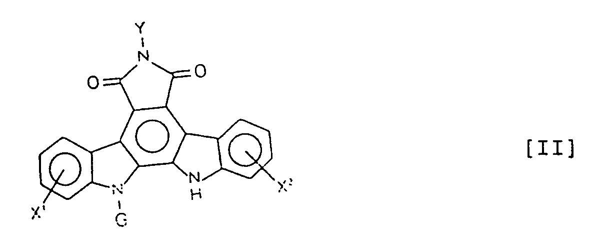 dans laquelle Y représente un atome d'hydrogène ou un groupe alkyle en C₁-C₆ substitué ou non substitué et X¹, X² et G ont les mêmes significations comme celles définies dans la revendication 1.
